(19) Europäisches Patentamt
European Patent Office
Office européen des brevets

(11) EP 4 529 924 B1

(12) EUROPEAN PATENT SPECIFICATION

(45) Date of publication and mention of the grant of the patent:
**18.02.2026 Bulletin 2026/08**

(21) Application number: **23200597.5**

(22) Date of filing: **28.09.2023**

(51) International Patent Classification (IPC):
***A61K 9/70*** *(2006.01)* ***A61K 47/14*** *(2017.01)*
***A61K 31/439*** *(2006.01)* ***A61P 25/34*** *(2006.01)*

(52) Cooperative Patent Classification (CPC):
**A61K 9/7038; A61K 31/439; A61K 47/14; A61P 25/34**

# (54) TRANSDERMAL THERAPEUTIC SYSTEM COMPRISING CYTISINE

TRANSDERMALES THERAPEUTISCHES SYSTEM MIT CYTISIN

SYSTÈME THÉRAPEUTIQUE TRANSDERMIQUE COMPRENANT DE LA CYTISINE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**02.04.2025 Bulletin 2025/14**

(73) Proprietor: **LTS Lohmann Therapie-Systeme AG
56626 Andernach (DE)**

(72) Inventors:
- **HAMMES, Florian
56626 Andernach (DE)**
- **TOMELERI, Anja
56567 Neuwied (DE)**
- **KLEUDGEN, Tobias
56729 Ettringen (DE)**

(74) Representative: **Maiwald GmbH
Elisenhof
Elisenstraße 3
80335 München (DE)**

(56) References cited:
**EP-A1- 2 559 427 US-A1- 2016 199 315**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 529 924 B1

## Description

### TECHNICAL FIELD OF THE INVENTION

The present invention relates to a transdermal therapeutic system (TTS) for the transdermal administration of cytisine to the systemic circulation, and processes of manufacture, method of treatments and uses thereof.

### BACKGROUND OF THE INVENTION

The active agent cytisine (7,11-diazatricyclo[7.3.1.0$^{2,7}$]trideca-2,4-dien-6-one) is an alkaloid naturally derived from the Faboideae sub-family of the Fabaceae family, especially from the seeds of *Laburnum anagyroides* or *Cytisus laburnum* (golden rain acacia). It is a nicotine analogue which acts as neuronal nicotinic acetylcholine receptor (nAChR) agonist.

Cytisine's interactions with various nAChR subtypes located in different areas of the central and peripheral nervous systems have been shown to be neuroprotective, to influence the autonomic and cardiovascular systems, to regulate mood, food intake and motor activity, and to have a wide range of biological effects on nicotine and alcohol addiction. In particular, cytisine is a partial agonist of $\alpha4\beta2$ and a near full agonist of $\alpha6\beta2$ nAChRs and at low doses can activate the nAChRs in the mesolimbic reward pathways, inhibit the sensory stimulation of nicotine and decrease withdrawal symptoms.

Thus, cytisine was introduced in several Eastern European countries as a smoking cessation medication in the late 1960s.

Currently, cytisine is commercially available in the form of tablets and capsules, which are administered in a dosage strength of 1.5 mg per tablet / capsule, e.g., under the brand names *Asmoken*® (Aflofarm), *Tabex*® (Sopharma), and *Cravv*® (*Zpharm*) for a treatment period of 25 days. During this time, starting with one tablet every two hours and a maximum of six tablets a day, the drug is dosed down to only one to two tablets a day. The dosage regimen corresponds to a maximum daily dose of 9 mg for the first 3 days, 7.5 mg from 4$^{th}$ to 12$^{th}$ day, 6 mg from 13$^{th}$ to 16$^{th}$ day, 4.5 mg from 1$^{7th}$ to 20$^{th}$ day and 1.5 to 3 mg from 21$^{st}$ to 25$^{th}$ day.

However, the above dosage forms require the patient aiming at smoking cessation to observe the complex dose regimen stipulated, including five variations of maximum daily dose and administration interval within 25 days. This may lead to reduced patient compliance and improper administration such as dose reduction, dose skipping, irregular drug intake or a complete abstinence from the intended cytisine intake. Further, oral administration of cytisine tablets or capsules up to six times per day usually goes along with low administration comfort.

There is thus a need for other dosage forms which are more convenient for the patient and preferably may also reduce the occurrence of adverse effects, most of them being gastrointestinal with the current dosage forms.

For example, the disadvantages of oral administration and in particular the number of cytisine administrations per day could be reduced by using a transdermal therapeutic system that releases a certain amount of cytisine over a certain time period of, e.g., 1 day. Transdermal delivery of cytisine has been investigated, but it appears that passive transdermal delivery of an appropriate amount of cytisine within 24 hours is challenging, e.g., in terms of the skin permeation profile and active ingredient utilization. Up to date, no commercial cytisine TTS is available.

It is therefore desirable to provide a transdermal therapeutic system for the transdermal administration of cytisine, which is suitable to replace cytisine oral dosage forms and to be used for aiding to smoking cessation.

### OBJECTS AND SUMMARY OF THE INVENTION

It is an object of the present invention to provide a TTS overcoming the above-mentioned disadvantages of current cytisine administration.

Thus, it is an object of the present invention to provide a TTS for the transdermal administration of cytisine providing a permeation rate which is sufficient for achieving a therapeutically effective dose. In particular, the object is to provide a TTS for the transdermal administration of cytisine, providing therapeutically effective amounts of cytisine for at least 12 hours, preferably for about 24 hours or 1 day. Longer periods of application allowing dosing intervals such as at 24 hours, 48 hours, or even a twice or once-weekly exchange may also be desirable depending on the situation.

It is a further object of the present invention to provide a TTS for the transdermal administration of cytisine providing a mean release rate of cytisine per day which corresponds to the maximum daily dose of current cytisine administration at a useful and reasonable patch size. In particular, the object is to provide a TTS for the transdermal administration of cytisine having an area of release of not more than 50 $cm^2$, preferably not more than 40 $cm^2$, releasing about 9 mg of cytisine per day.

It is a further object of the present invention to provide a TTS for the transdermal administration of cytisine with a high active ingredient utilization. In particular, the object is to provide a TTS for the transdermal administration of cytisine,

releasing at least 25 %, at least 50 % or at least 70 % of the active ingredient contained therein within about 24 hours after application.

It is a further object of the present invention to provide a TTS for the transdermal administration of cytisine, which is suitable for dosing intervals of about 24 hours or 1 day. In particular, the object is to provide a TTS for the transdermal administration of cytisine, reaching maximum skin permeation rates within about 24 hours after application.

It is also an object of the present invention to provide a TTS for the transdermal administration of cytisine which complies with the needs of a convenient application in view of size and thickness and/or which is easy and cost-efficient to manufacture.

It is another object of the present invention to provide a TTS for the transdermal administration of cytisine which can be used in a method of treatment.

These objects and others are accomplished by the present invention, which according to one aspect relates to a transdermal therapeutic system for the transdermal administration of cytisine comprising a self-adhesive layer structure containing a therapeutically effective amount of cytisine, said self-adhesive layer structure comprising:

A) a backing layer;
B) a cytisine-containing layer comprising

1. cytisine,
2. a polymer selected from the group consisting of silicone-based polymers and polyisobutylenes, and
3. a permeation enhancer comprising an organic compound having a linear backbone including 6 to 14 carbon atoms and provided with 1 hydroxy group.

It has been surprisingly found that the TTS according to the present invention, which comprises in the cytisine-containing layer a polymer selected from the group consisting of silicone-based polymers and polyisobutylenes as well as a permeation enhancer comprising an organic compound having a linear backbone including 6 to 14 carbon atoms and provided with 1 hydroxy group, which is preferably a mixture consisting of propylene glycol monocaprylate and propylene glycol dicaprylate or is dodecan-1-ol, is advantageous in terms of permeation properties and active ingredient utilization. In particular, it has been found that the above components allow for a 24 hours release of cytisine, thus enabling the resulting TTS according to the invention to be particularly suitable for dosing intervals of 24 hours or 1 day.

According to one specific aspect, the present invention relates to a transdermal therapeutic system for the transdermal administration of cytisine comprising a self-adhesive layer structure containing a therapeutically effective amount of cytisine, said self-adhesive layer structure comprising:

A) a backing layer;
B) a cytisine-containing layer comprising

1. cytisine in an amount of from 4 to 12 %, in particular about 5 % or about 10 %,
2. at least one amine-compatible polysiloxane in an amount of from 68 to 82 %, such as about 70 %, about 75 % or about 80 %,
3. a copolymer based on dimethylaminoethyl methacrylate, butyl methacrylate and methyl methacrylate or a polyvinylpyrrolidone in an amount of from 2 to 22 %, preferably from 2 to 12 % or from 18 to 22 %, in particular a copolymer based on dimethylaminoethyl methacrylate, butyl methacrylate and methyl methacrylate in an amount of about 10 %, or a polyvinylpyrrolidone in an amount of about 5 % or about 20 %, and
4. a permeation enhancer comprising a mixture consisting of propylene glycol monocaprylate and propylene glycol dicaprylate or dodecan-1-ol in an amount of from 5 to 11 %, such as about 5 % or about 10 %,

based on the total weight of the cytisine-containing layer.

According to another specific aspect, the present invention relates to a transdermal therapeutic system for the transdermal administration of cytisine comprising a self-adhesive layer structure containing a therapeutically effective amount of cytisine, said self-adhesive layer structure comprising:

A) a backing layer;
B) a cytisine-containing layer comprising

1. cytisine in an amount of from 4 to 12 %, in particular about 5 % or about 10 %,
2. at least one polyisobutylene in an amount of from 60 to 75 %, preferably from 66 to 70 %, such as about 68 %,
3. a polyvinylpyrrolidone in an amount of from 18 to 22 %, such as about 20 %,
and

4. a permeation enhancer comprising a mixture consisting of propylene glycol monocaprylate and propylene glycol dicaprylate or dodecan-1-ol in an amount of from 5 to 11 %, such as about 7.5 %,

based on the total weight of the cytisine-containing layer.

According to certain embodiments of the invention, the transdermal therapeutic system according to the invention is for use in a method of treating a human patient, preferably in a method of treating nicotine addiction, and more preferably in a method of reducing nicotine craving, in particular in a method of aiding to smoking cessation.

According to certain embodiments of the invention, the present invention relates to the use of a transdermal therapeutic system according to the invention for the manufacture of a medicament for treating a human patient, preferably in a method of treating nicotine addiction, and more preferably in a method of reducing nicotine cravings, in particular in a method of smoking cessation.

According to certain embodiments of the invention, the invention relates to a method of treating a human patient, preferably in a method of treating nicotine addiction, and more preferably in a method of reducing nicotine cravings, in particular in a method of o smoking cessation, including applying a transdermal therapeutic system according to the invention to the skin of the patient.

According to another aspect, the present invention relates to a process for manufacturing a cytisine-containing layer for use in a transdermal therapeutic system comprising the steps of:

1) combining at least the components

1. cytisine,
2. a polymer selected from the group consisting of silicone-based polymers and polyisobutylenes, and
3. a permeation enhancer selected from oxygen-containing compounds having 6 to 14 carbon atoms and exactly one hydroxy group

to obtain a coating composition;
2) coating the coating composition onto the backing layer or a release liner or any intermediate liner; and
3) drying the coated coating composition to form the cytisine-containing layer.

## DEFINITIONS

Within the meaning of this invention, the term "transdermal therapeutic system" (TTS) refers to a system by which the active agent (cytisine) is administered to the systemic circulation *via* transdermal delivery and refers to the entire individual dosing unit that is applied to the skin of a patient, and which comprises a therapeutically effective amount of cytisine in a self-adhesive layer structure and optionally an additional adhesive overlay on top of the cytisine-containing self-adhesive layer structure. The self-adhesive layer structure may be located on a release liner (a detachable protective layer). Thus, the TTS may further comprise a release liner. Within the meaning of this invention, the term "TTS" in particular refers to a system providing passive transdermal delivery excluding active transport as in methods including iontophoresis or microporation.

Within the meaning of this invention, the term "self-adhesive layer structure containing a therapeutically effective amount of cytisine" or "cytisine-containing self-adhesive layer structure" refers to the cytisine-containing structure providing the area of release for cytisine during administration. The adhesive overlay adds to the overall size of the TTS but does not add to the area of release. The cytisine-containing self-adhesive layer structure comprises a backing layer and at least one cytisine-containing layer.

Within the meaning of this invention, the term "therapeutically effective amount" refers to a quantity of active agent in the TTS sufficient to provide, if administered by the TTS to a patient, cytisine blood levels of a similar range (e.g. of about 10 % to about 1000 % as measured as an AUC) when compared to blood levels obtained in steady state administration of six, five, four or three times daily 1.5 mg, or twice daily 1.5 mg, or once daily 1.5 mg over a predefined extended period of time (e.g. at least 20 hours, such as about 24 hours or 1 day). A TTS usually contains more active in the system than is in fact provided to the skin and the systemic circulation. This excess amount of active agent is usually necessary to provide enough driving force for the passive transportation from the TTS to the systemic circulation.

Within the meaning of this invention, the terms "active", "active agent", and the like, as well as the term "cytisine" refer to cytisine in any pharmaceutically acceptable chemical and morphological form and physical state. These forms include without limitation cytisine in its free base form, protonated or partially protonated cytisine, cytisine salts and in particular acid addition salts formed by addition of an inorganic or organic acid such as cytisine hydrochloride or cytisine maleate, hydrates, complexes and so on, as well as cytisine in the form of particles which may be micronized and any mixtures of the aforementioned forms.

The cytisine, where contained in a medium such as a solvent, may be dissolved or dispersed or in part dissolved

and in part dispersed.

When cytisine is mentioned to be used in a particular form in the manufacture of the TTS, this does not exclude interactions between this form of cytisine and other ingredients of the cytisine-containing self-adhesive layer structure, e.g. salt formation or complexation, in the final TTS. This means that, even if cytisine is included in its free base form, it may be present in the final TTS in protonated or partially protonated form or in the form of an acid addition salt, or, if it is included in the form of a salt, parts of it may be present as free base in the final TTS. Unless otherwise indicated, in particular the amount of cytisine in the self-adhesive layer structure relates to the amount of cytisine included in the TTS during manufacture of the TTS and is calculated based on cytisine in the form of the free base. The cytisine starting material included in the TTS during manufacture of the TTS may be in the form of particles. Cytisine may e.g. be present in the self-adhesive layer structure in the form of particles and/or dissolved.

In this context, the term "particles" refers to a solid, particulate material comprising individual particles, the dimensions of which are negligible compared to the material. In particular, the particles are solid, including plastic/deformable solids. The term "dispersing" refers to a step or a combination of steps wherein a starting material (e.g. cytisine) is not totally dissolved. Dispersing in the sense of the invention comprises the dissolution of a part of the starting material (e.g. cytisine particles), depending on the solubility of the starting material (e.g. the solubility of cytisine in the coating composition).

There are two main types of TTS using passive active agent delivery, i.e. matrix-type TTS and reservoir-type TTS. In matrix-type TTS the active agent is included in a matrix, while in a reservoir-type TTS the active agent is included in a liquid or semi-liquid reservoir. The release of the active agent in a matrix-type TTS is mainly controlled by the matrix including the active agent itself. In contrast thereto, a reservoir-type TTS needs a rate-controlling membrane controlling the release of the active agent. Matrix-type TTS are advantageous in that, compared to reservoir type TTS, usually no rate determining membranes are necessary and no dose dumping can occur due to membrane rupture. In summary, matrix-type transdermal therapeutic systems (TTS) are less complex in manufacture and easy and convenient to use by patients.

Within the meaning of this invention, "matrix-type TTS" refers to a system or structure wherein the active is homogeneously dissolved and/or dispersed within a polymeric carrier, i.e. the matrix, which forms with the active agent and optionally remaining ingredients a matrix layer. In such a system, the matrix layer controls the release of the active agent from the TTS. A matrix-type TTS may also include a rate-controlling membrane.

TTS with a rate-controlling membrane and a liquid or semi-liquid active agent containing reservoir, wherein the release of the active agent from the TTS is controlled by the rate-controlling membrane, are referred to by the term "reservoir-type TTS". Reservoir-type TTS are not to be understood as being of matrix-type within the meaning of the invention. In particular, within the meaning of this invention, microreservoir-systems (biphasic systems having an inner active-containing phase in an outer matrix-phase), considered in the art to be a mixture between a matrix-type TTS and a reservoir-type TTS, are considered to be of matrix-type within the meaning of the invention. Matrix-type TTS may in particular be in the form of a "drug-in-adhesive"-type TTS referring to a system wherein the active is homogeneously dissolved and/or dispersed within a pressure-sensitive adhesive matrix.

The term "matrix layer" refers to any layer containing the active homogeneously dissolved and/or dispersed within a polymeric carrier. Typically, a matrix layer is present in a matrix-type TTS as the active agent-containing layer. A reservoir-type TTS may comprise, in addition to a reservoir layer and a rate-controlling membrane, an additional adhesive layer which serves as a skin contact layer. In such a reservoir-type TTS, the additional adhesive layer often is manufactured as an active agent-free layer. However, due to the concentration gradient, the active agent will migrate from the reservoir to the additional adhesive layer over time, until an equilibrium is reached. Therefore, in such a reservoir-type TTS, after some time of equilibration, the additional adhesive layer contains the active agent and is to be regarded as a matrix layer in the sense of the present invention.

Within the meaning of the invention, the term "cytisine-containing layer" refers to a layer containing cytisine and providing the area of release. If the cytisine-containing layer is a cytisine-containing matrix layer, said layer is present in a matrix-type TTS. As used herein, the cytisine-containing layer is preferably a cytisine-containing matrix layer, and it is referred to the final solidified layer, e.g., obtained after coating and drying a solvent-containing coating composition as described herein. Alternatively, a cytisine-containing matrix layer is obtained after melt-coating and cooling. The cytisine-containing matrix layer may also be manufactured by laminating two or more such solidified layers (e.g. dried or cooled layers) of the same composition to provide the desired area weight. The cytisine-containing layer may be self-adhesive (in the form of a pressure sensitive adhesive matrix) or the TTS may comprise an additional skin contact layer of a pressure sensitive adhesive for providing sufficient tack. In particular, the cytisine-containing layer is a pressure sensitive adhesive matrix layer. As used herein, the term "skin contact layer" refers to a layer that may be included in the TTS to be in direct contact with the skin of the patient during administration. When the TTS comprises a skin contact layer, the other layers do not contact the skin and do not necessarily have self-adhesive properties. As outlined above, the skin contact layer may over time absorb parts of the active agent and then may be regarded as a cytisine-containing layer. The area of release is provided by the area of the cytisine-containing layer. A skin contact layer may be used to enhance adherence. The sizes of an additional skin contact layer and the cytisine-containing layer are usually coextensive and correspond to the area of

release.

Within the meaning of the invention, the term "backing layer" refers to a layer which supports the cytisine-containing layer. At least one backing layer in the TTS and usually the backing layer of the cytisine-containing layer is substantially impermeable to cytisine, as well as optionally any additive, contained in the layer during the period of storage and administration and thus prevents active loss or cross-contamination in accordance with regulatory requirements. Preferably, the backing layer is also occlusive, meaning substantially impermeable to water and water-vapor. Suitable materials for a backing layer include polyethylene terephthalate (PET), polyethylene (PE), ethylene vinyl acetate-copolymer (EVA), polyesters, polyurethanes, and mixtures thereof. Suitable backing layers may be siliconized or fluoropolymer coated in order to improve the adhesion of the cytisine-containing layer to the backing layer.

Furthermore, an adhesive overlay may be present. In this context, the term "adhesive overlay" is understood to mean a self-adhesive layer structure that is free of cytisine and larger in area than the self-adhesive layer structure and provides additional area adhering to the skin, but no area of release of cytisine. It enhances thereby the overall adhesive properties of the TTS. The area of said adhesive overlay adds to the overall size of the TTS but does not add to the area of release. The adhesive overlay may comprise a self-adhesive polymer or a self-adhesive polymer mixture selected from the group of acrylic polymers, polyisobutylenes, styrene-isoprene-styrene copolymers, polysiloxanes, and mixtures thereof, which may be identical to or different from any polymer or polymer mixture included in the cytisine-containing self-adhesive layer structure. The adhesive overlay comprises a backing layer that may provide occlusive or non-occlusive properties and an adhesive layer. Preferably, the backing layer of the adhesive overlay provides non-occlusive properties.

Within the meaning of this invention, the term "area weight" refers to the dry weight of a specific layer, e.g. of the cytisine-containing layer, provided in $g/m^2$. The area weight values are subject to a tolerance of $\pm$ 10 %, preferably $\pm$ 7.5 %, due to manufacturing variability.

If not indicated otherwise "%" refers to wt.% (% by weight).

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2000, preferably above 5000 and more preferably above 10,000 Dalton. Correspondingly, compounds with a molecular weight below 2000, preferably below 5000 or more preferably below 10,000 Dalton are usually referred to as oligomers.

Within the meaning of this invention, the term "functional groups" refers to hydroxy- and carboxylic acid groups.

Within the meaning of the invention, the term "silicon-based polymer" refers to a non-hybrid polymer (i.e. a polymer, which does not include a hybrid species) comprising polysiloxanes. Polysiloxanes can be made from solvent-free two-component systems or a solution in organic solvents. They exist in two fundamentally different variants: polysiloxanes which have free silanol groups and amine resistant polysiloxanes which are distinguished in that the free silanol groups are derivatized by trimethylsilyl groups. The methyl groups can be completely or partially replaced by other alkyl radicals or alternatively phenyl radicals. Polysiloxanes as used herein are synthesized from linear bifunctional and branched polyfunctional oligomers, the ratio of which determines the physical properties thereof. More polyfunctional oligomers result in a more cross-linked adhesive with a higher cohesion and a reduced tack, less polyfunctional oligomers result in a higher tack and a reduced cohesion. It is preferred for the silicon-based polymer to be a mixture of high tack and medium tack, or high tack and low tack, polysiloxanes. Preferably, the at least one silicone-based polymer is a silicone-based pressure sensitive adhesive.

Within the meaning of this invention, the term "pressure-sensitive adhesive" refers to a material that in particular adheres with finger pressure, is permanently tacky, exerts a strong holding force and should be removable from smooth surfaces without leaving a residue. It is obtainable from a solvent-containing adhesive coating composition after coating on a film and evaporating the solvents (e.g. n-heptane or ethyl acetate). In this context, the term "solvent" is understood to mean any liquid substance, which preferably is a volatile organic liquid such as methanol, ethanol, isopropanol, acetone, ethyl acetate, methylene chloride, hexane, n-heptane, toluene, and mixtures thereof. Examples of useful pressure-sensitive adhesives comprising polysiloxanes which are commercially available include the standard BIO-PSA series (7-4400,7-4500 and 7-4600 series), the amine compatible (endcapped) BIO-PSA series (7-4100, 7-4200 and 7-4300 series), the Soft Skin Adhesives series (7-9800) and the BIO-PSA Hot Melt Adhesive manufactured by Dupont. Preferred pressure-sensitive adhesives comprising polysiloxane are heptane-solvated pressure-sensitive adhesives including Liveo™BIO-PSA 7-4201 and BIO-PSA 7-4301 or ethyl acetate-solvated pressure-sensitive adhesives including Liveo™BIO-PSA 7-4202 and BIO-PSA 7-4302. Also, polyisobutylenes may act as pressure-sensitive adhesives. Suitable polyisobutylenes according to the invention are available under the tradename Oppanol® or Duro-Tak™. Combinations of high-molecular weight polyisobutylenes (Oppanol® B100, B80) and low-molecular weight polyisobutylenes (Oppanol® B10, B11, B12, B13) may also be used. A pressure-sensitive adhesive layer, when in contact with the skin, is "self-adhesive", i.e., provides adhesion to the skin so that typically no further aid for fixation on the skin is needed. A "self-adhesive" layer structure includes a pressure sensitive adhesive layer for skin contact which may be provided in the form of a pressure sensitive adhesive matrix or in the form of an additional layer, i.e. a pressure sensitive adhesive skin contact layer. An adhesive overlay may still be employed to advance adhesion.

Within the meaning of the invention, the term "permeation enhancer" refers to a substance which influences the barrier properties of the stratum corneum in the sense of increasing the active agent permeability. Suitable permeation enhancers according to the invention are organic compounds having a linear backbone including 6 to 14 carbon atoms and provided with 1 hydroxy group. In this context, the term "organic compound having a linear backbone" is understood to mean a molecule consisting of one linear (i.e., unbranched) chain of covalently bonded atoms, which are mostly carbon atoms, in particular 6 to 14 carbon atoms, but may also comprise atoms of other elements, such as hydrogen, oxygen or nitrogen. For examples, the organic compound may be selected from the group consisting of alkanes, such as hexane, heptane, octane, nonane, decane, undecane, dodecane, tridecane or tetradecane, alcohols, such as hexanol, heptanol, octanol, nonanol, decanol, undecanol, dodecanol, tridecanol or tetradecanol, ethers, such as methyl pentyl ether, ethyl butyl ether, dipropyl ether, methyl hexyl ether, ethyl pentyl ether, propyl butyl ether, methyl heptyl ether, ethyl hexyl ether, propyl pentyl ether, dibutyl ether, methyl octyl ether, ethyl heptyl ether, propyl hexyl ether, butyl pentyl ether, methyl nonyl ether, ethyl octyl ether, propyl heptyl ether, butyl hexyl ether, dipentyl ether, methyl decyl ether, ethyl nonyl ether, propyl octyl ether, butyl heptyl ether, pentyl hexyl ether, methyl undecyl ether, ethyl decyl ether, propyl nonyl ether, butyl octyl ether, pentyl heptyl ether, dihexyl ether, methyl dodecyl ether, ethyl undecyl ether, propyl decyl ether, butyl nonyl ether, pentyl octyl ether, hexyl heptyl ether, methyl tridecyl ether, ethyl dodecyl ether, propyl undecyl ether, butyl decyl ether, pentyl nonyl ether, hexyl octyl ether or diheptyl ether, diethers, polyether, or esters, such as formic acid pentyl ester, acetic acid butyl ester, propanoic acid propyl ester, butanoic acid ethyl ester, valeric acid methyl ester, formic acid hexyl ester, acetic acid pentyl ester, propanoic acid butyl ester, butanoic acid propyl ester, valeric acid ethyl ester, caproic acid methyl ester, formic acid heptyl ester, acetic acid hexyl ester, propanoic acid pentyl ester, butanoic acid butyl ester, valeric acid propyl ester, caproic acid ethyl ester, enanthic acid methyl ester, formic acid octyl ester, acetic acid heptyl ester, propanoic acid hexyl ester, butanoic acid pentyl ester, valeric acid butyl ester, caproic acid propyl ester, enanthic acid ethyl ester, caprylic acid methyl ester, formic acid nonyl ester, acetic acid octyl ester, propanoic acid heptyl ester, butanoic acid hexyl ester, valeric acid pentyl ester, caproic acid butyl ester, enanthic acid propyl ester, caprylic acid ethyl ester, pelargonic acid methyl ester, formic acid decyl ester, acetic acid nonyl ester, propanoic acid octyl ester, butanoic acid heptyl ester, valeric acid hexyl ester, caproic acid pentyl ester, enanthic acid butyl ester, caprylic acid propyl ester, pelargonic acid ethyl ester, capric acid methyl ester, formic acid undecyl ester, acetic acid decyl ester, propanoic acid nonyl ester, butanoic acid octyl ester, valeric acid heptyl ester, caproic acid hexyl ester, enanthic acid pentyl ester, caprylic acid butyl ester, pelargonic acid propyl ester, capric acid ethyl ester, hendecanoic acid methyl ester, formic acid dodecyl ester, acetic acid undecyl ester, propanoic acid decyl ester, butanoic acid nonyl ester, valeric acid octyl ester, caproic acid heptyl ester, enanthic acid hexyl ester, caprylic acid pentyl ester, pelargonic acid butyl ester, capric acid propyl ester, hendecanoic acid ethyl ester, lauric acid methyl ester, formic acid tridecyl ester, acetic acid dodecyl ester, propanoic acid undecyl ester, butanoic acid decyl ester, valeric acid nonyl ester, caproic acid octyl ester, enanthic acid heptyl ester, caprylic acid hexyl ester, pelargonic acid pentyl ester, capric acid butyl ester, hendecanoic acid propyl ester, lauric acid ethyl ester, or tridecyclic acid methyl ester. If such organic compound does not comprise a hydroxy group by definition, i.e., as functional group, such as alcohols, the organic compound is required to be additionally provided with one hydroxy group, in particular exactly one hydroxy group. In other words, the organic compound does not comprise 2 hydroxy groups or more.

Within the meaning of the invention, the term "additional polymer" refers to a polymer other than the polymer selected from the group consisting of silicone-based polymers and polyisobutylenes, which is required to be contained in the cytisine-containing layer of the TTS according to the invention. The additional polymer may be selected, e.g., from acrylic polymers or polymers based on natural or synthetic rubbers. Suitable additional polymers include polymethacrylates, such as e.g., the Eudragit® series, or polyvinylpyrrolidones, in particular povidones or crospovidones. In this context, the term "soluble polyvinylpyrrolidone" refers to polyvinylpyrrolidone, also known as povidone, which is soluble with more than 10 % in at least ethanol, preferably also in water, diethylene glycol, methanol, n-propanol, 2 propanol, n-butanol, chloroform, methylene chloride, 2-pyrrolidone, macrogol 400, 1,2 propylene glycol, 1,4 butanediol, glycerol, triethanolamine, propionic acid and acetic acid. Examples of polyvinylpyrrolidones which are commercially available include Kollidon® 12 PF, Kollidon® 17 PF, Kollidon® 25, Kollidon® 30 and Kollidon® 90 F supplied by BASF, or povidone K90F. The different grades of Kollidon® are defined in terms of the K-Value reflecting the average molecular weight of the polyvinylpyrrolidone grades. Kollidon® 12 PF is characterized by a K-Value range of 10.2 to 13.8, corresponding to a nominal K-Value of 12. Kollidon® 17 PF is characterized by a K-Value range of 15.3 to 18.4, corresponding to a nominal K-Value of 17. Kollidon® 25 is characterized by a K-Value range of 22.5 to 27.0, corresponding to a nominal K-Value of 25, Kollidon® 30 is characterized by a K-Value range of 27.0 to 32.4, corresponding to a nominal K-Value of 30. Kollidon® 90 F is characterized by a K-Value range of 81.0 to 97.2, corresponding to a nominal K-Value of 90. Preferred Kollidon® grades are Kollidon® 12 PF, Kollidon® 30 and Kollidon® 90 F. In this contex, the term "K-Value" refers to a value calculated from the relative viscosity of polyvinylpyrrolidone in water according to the European Pharmacopoeia (Ph.Eur.) and USP monographs for "Povidone". In contrast, within the meaning of the invention, term "insoluble polyvinylpyrrolidone" refers to

cross-linked polyvinylpyrrolidone, also known as crospovidone, which is. substantially water-insoluble. Examples of polyvinylpyrrolidones which are commercially available include Kollidon® CL, Kollidon® CL-M and Kollidon® CL-SF supplied by BASF. For all grades and types of polyvinylpyrrolidone, it is preferred that the amount of peroxides is within certain limits, in particular, the peroxide amount is equal to or less than 500 ppm, more preferably equal to or less than 150 ppm, and most preferably equal to or less than 100 ppm.

The TTS according to the present invention can be characterized by certain parameters as measured in an *in vitro* skin permeation test.

The *in vitro* permeation test can be performed with human or animal skin and preferably with dermatomed split-thickness human skin with a thickness of 500 μm and an intact epidermis, and with phosphate buffer pH 5.5 or 7.4 as receptor medium (32 °C with 0.1 % saline azide) with or without addition of a maximum of 40 vol-% organic solvent e.g. ethanol, acetonitrile, isopropanol, dipropylenglycol, PEG 400 so that a receptor medium may e.g. contain 60 vol-% of 0.9% sodium chloride, 30 vol-% dipropylenglycol and 10 vol-% acetonitrile.

Where not otherwise indicated, the *in vitro* permeation test is performed in a Franz diffusion cell with heat separated human epidermis, and with phosphate buffer pH 7.4 as receptor medium (32 °C with 0.1 % saline azide). The amount of cytisine permeated into the receptor medium is determined in regular intervals using a partial validated HPLC method (column: stainless steel column 150 mm x 4.6 mm internal diameter with C18 base and acid deactivated stationary phase, 3.5 μm particle size, e.g. Zorbax SB C18 (Agilent); column temperature: 25 °C; mobile phase: acetonitrile / water / TEA = 20:80:0.35 (v/v/v) with a UV photometric detector by taking a sample volume. The receptor medium is completely or in part replaced by fresh medium when taking the sample volume, and the measured amount of cytisine permeated relates to the amount permeated between the two last sampling points and not the total amount permeated so far.

Thus, within the meaning of this invention, the parameter "permeated amount" is provided in μg/cm$^2$ and relates to the amount of cytisine permeated in a sample interval at certain elapsed time. E.g., in an *in vitro* permeation test as described above, wherein the amount of cytisine permeated into the receptor medium has been e.g. measured at hours 0, 4, 8, 24, 32, 48 and 72, the "permeated amount" of cytisine can be given e.g. for the sample interval from hour 8 to hour 24 and corresponds to the measurement at hour 24. The permeated amount can also be given as a "cumulative permeated amount", corresponding to the cumulated amount of cytisine permeated at a certain point in time. E.g., in an *in vitro* permeation test as described above, wherein the amount of cytisine permeated into the receptor medium has been e.g. measured at hours 0, 4, 8, 24, 32, 48 and 72, the "cumulative permeated amount" of cytisine at hour 24 corresponds to the sum of the permeated amounts from hour 0 to hour 4, hour 4 to hour 8, and hour 8 to hour 24.

Within the meaning of this invention, the parameter "skin permeation rate", also referred to as "Δ flux rate", for a certain sample interval at certain elapsed time is provided in μg/(cm$^2$*h) and is calculated from the permeated amount in said sample interval as measured by *in vitro* permeation test as described above in μg/cm$^2$, divided by the hours of said sample interval. E.g. the skin permeation rate in an *in vitro* permeation test as described above, wherein the amount of cytisine permeated into the receptor medium has been e.g. measured at minutes 0, 4, 8, 12, 24, 32 and 48 and 72, the "skin permeation rate" at hour 24 is calculated as the permeated amount in the sample interval from hour 8 to hour 24 divided by 16 hours. A "cumulative skin permeation rate" can be calculated from the respective cumulative permeated amount by dividing the cumulative permeated amount by the elapsed time.

Within the meaning of this invention, the above parameters "permeated amount" and "skin permeation rate" (as well as "cumulative permeated amount" and "cumulative skin permeation rate") refer to mean values calculated from at least 3 *in vitro* permeation test experiments. Where not otherwise indicated, the standard deviation (SD) of these mean values refer to a corrected sample standard deviation, calculated using the formula:

$$SD = \sqrt{\frac{1}{n-1}\sum_{i=1}^{n}(x_i - \overline{x})^2}$$

wherein n is the sample size, $\{x_1, x_2, ... x_n\}$ are the observed values and $\overline{x}$ is the mean value of the observed values.

Within the meaning of this invention, the parameter "mean release rate" refers to the mean release rate in μg/h, in mg/h, in μg/24 h, in mg/24 h, in μg/day or in mg/day over the period of administration (e.g. 24 hours or 1 day) by which the active agent is released through the human skin into the systemic circulation and is based on the AUC obtained over said period of administration in a clinical study. The mean release rate is a parameter used to identify the dose or the strength of a TTS. Since, in contrast to e.g. intravenous or oral administration and (as also described above) a TTS usually contains more active in the system than is in fact provided to the skin and the systemic circulation, the amount of active contained in the TTS is not meaningful as a parameter for the dose. This is why for a TTS the dose or strength is usually characterized by the mean release rate, which describes more accurately the amount of active delivered to the subject over time.

Within the meaning of this invention, the term "extended period of time" relates to a period of at least or about 12 h,

at least or about 20 h, or at least or about 24 h, or about 1 day.

For a continuous drug treatment, the frequency of drug administration is preferably kept sufficiently high so as to maintain a therapeutically effective blood plasma concentration. In other words, the interval between two dosage form administrations, also called dosing interval, needs to be adapted accordingly. Within the meaning of the present invention, the term "dosing interval" refers to the period of time between two consecutive TTS administrations, i.e. the interval between two consecutive points in time a TTS is applied to the skin of the patient. Once applied, the TTS is usually maintained on the skin of the patient for the entire dosing interval and only removed at the end of the dosing interval, at which time a new TTS is applied to the skin. E.g., if the dosing interval is 24 hour or 1 day, the TTS is applied to and maintained on the skin of the patient for 24 hours or 1 day. After 24 hours or 1 day, the TTS is removed from the skin and a new TTS is applied. Thus, a dosing interval of 24 hours or 1 day allows a once-a-day TTS exchange mode in an around-the-clock treatment.

Within the meaning of this invention, the term "room temperature" refers to the unmodified temperature found indoors in the laboratory where the experiments are conducted and usually lies within 15 to 35 °C, preferably about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated. Preferably, the patient suffers from nicotine addiction and/or needs assistance in smoking cessation.

According to the invention, the TTS as described herein is suitable for use in a method of treatment, in which the TTS is applied to the skin of the patient for a dosing interval of from about 20 to about 30 hours and preferably of about 24 hours. Within the dosing interval, it is preferred for the TTS to release almost the total amount of cytisine contained in the cytisine-containing layer of the TTS. Thus, the release profile of cytisine provided by the TTS preferably shows a global maximum within the dosing interval.

Within the meaning of this invention, the term "coating composition" refers to a composition comprising all components of the cytisine-containing layer in a solvent, which may be coated onto the backing layer or release liner to form the cytisine-containing layer upon drying.

Within the meaning of this invention, the term "dissolve" refers to the process of obtaining a solution, which is clear and does not contain any particles, as visible to the naked eye.

Within the meaning of this invention, and unless otherwise specified, the term "about" refers to an amount that is ± 10 % of the disclosed amount. In some embodiments, the term "about" refers to an amount that is ± 5 % of the disclosed amount. In some embodiments, the term "about" refers to an amount that is ± 2 % of the disclosed amount.

## BRIEF DESCRIPTION OF THE DRAWINGS

Fig. 1a depicts the cytisine skin permeation rate of transdermal therapeutic systems prepared according to Reference Examples 1a to 1c for hours 0 to 72.

Fig. 1b depicts the utilization of cytisine of transdermal therapeutic systems prepared according to Reference Examples 1a to 1c after 24 hours.

Fig. 2a depicts the cytisine skin permeation rate of transdermal therapeutic systems prepared according to Reference Examples 2b to 2f for hours 0 to 72.

Fig. 2b depicts the utilization of cytisine of transdermal therapeutic systems prepared according to Reference Examples 2b to 2f after 24 hours.

Fig. 3a depicts the cytisine skin permeation rate of transdermal therapeutic systems prepared according to Reference Examples 3a to 3c for hours 0 to 72.

Fig. 3b depicts the utilization of cytisine of transdermal therapeutic systems prepared according to Reference Examples 3a to 3c after 24 hours.

Fig. 4a depicts the cytisine skin permeation rate of transdermal therapeutic systems prepared according to Reference Examples 4a to 4c for hours 0 to 72.

Fig. 4b depicts the utilization of cytisine of transdermal therapeutic systems prepared according to Reference Examples 4a to 4c after 24 hours.

Fig. 5a depicts the cytisine skin permeation rate of transdermal therapeutic systems prepared according to Examples 5a to 5c for hours 0 to 72.

Fig. 5b depicts the utilization of cytisine of transdermal therapeutic systems prepared according to Examples 5a to 5c after 24 hours.

Fig. 6a depicts the cytisine skin permeation rate of transdermal therapeutic systems prepared according to Examples 6a to 6d as well as Reference Examples 6e and 6f for hours 0 to 72.

Fig. 6b depicts the utilization of cytisine of transdermal therapeutic systems prepared according to Examples 6a to 6d as well as Reference Examples 6e and 6f after 24 hours.

Fig. 7a depicts the cytisine skin permeation rate of transdermal therapeutic systems prepared according to Examples 7a to 7e as well as Reference Examples 7f to 7h for hours 0 to 72.

Fig. 7b depicts the utilization of cytisine of transdermal therapeutic systems prepared according to Examples 7a to 7e as well as Reference Examples 7f to 7h after 24 hours.

Fig. 8a depicts the cytisine skin permeation rate of transdermal therapeutic systems prepared according to Examples 8a to 8g for hours 0 to 72.

Fig. 8b depicts the utilization of cytisine of transdermal therapeutic systems prepared according to Examples 8a to 8g after 24 hours.

Fig. 9a depicts the cytisine skin permeation rate of transdermal therapeutic systems prepared according to Examples 9a to 9e for hours 0 to 72.

Fig. 9b depicts the utilization of cytisine of transdermal therapeutic systems prepared according to Examples 9a to 9e after 24 hours.

Fig. 10a depicts the cytisine skin permeation rate of transdermal therapeutic systems prepared according to Examples 10a to 10e for hours 0 to 72.

Fig. 10b depicts the utilization of cytisine of transdermal therapeutic systems prepared according to Examples 10a to 10e after 24 hours.

Fig. 11a depicts the cytisine skin permeation rate of transdermal therapeutic systems prepared according to Examples 11a and 11b for hours 0 to 72.

Fig. 11b depicts the utilization of cytisine of transdermal therapeutic systems prepared according to Examples 11a and 11b after 24 hours.

## DETAILED DESCRIPTION

## TTS STRUCTURE

The present invention is related to a transdermal therapeutic system for the transdermal administration of cytisine comprising a self-adhesive layer structure containing cytisine.

In particular, the self-adhesive layer structure comprises therapeutically effective amounts of cytisine. Thus, in certain embodiments, the transdermal therapeutic system, in particular the self-adhesive layer structure, comprises cytisine in an amount of from 1 to 100 mg, preferably from 10 to 70 mg, more preferably from 15 to 50 mg. Preferably, the TTS according to the invention comprises cytisine in an amount of from 10 to 30 mg or from 25 to 70 mg, preferably from 12 to 28 mg or from 28 to 60 mg, more preferably from 15 to 22 mg or from 33 to 50 mg.

The transdermal therapeutic system according to the invention comprises a self-adhesive layer structure comprising:

A) a backing layer;
B) a cytisine-containing layer comprising

1. cytisine,
2. a polymer selected from the group consisting of silicone-based polymers and polyisobutylenes, and
3. a permeation enhancer comprising an organic compound having a linear backbone including 6 to 14 carbon atoms and provided with 1 hydroxy group.

In a preferred embodiment, the self-adhesive layer structure comprises:

A) a backing layer;
B) a cytisine-containing layer comprising

1. cytisine,
2. a polymer selected from the group consisting of silicone-based polymers and polyisobutylenes,
3. an additional polymer, and
4. a permeation enhancer comprising an organic compound having a linear backbone including 6 to 14 carbon atoms and provided with 1 hydroxy group.

According to a certain embodiment of the invention, the transdermal therapeutic system for the transdermal administration of cytisine comprises a self-adhesive layer structure containing a therapeutically effective amount of

cytisine, said self-adhesive layer structure comprising:

A) a backing layer;
B) a cytisine-containing layer comprising

1. cytisine,
2. a polymer selected from the group consisting of silicone-based polymers and polyisobutylenes, and
3. a permeation enhancer comprising an organic compound having a linear backbone including 6 to 14 carbon atoms and provided with 1 hydroxy group,

wherein the cytisine-containing layer does not comprise propylene glycol, dipropylene glycol, glycerol triacetate, glycerol monocaprylate, diethyl sebacate, propylene glycol monolaurate, isopropyl palmitate or octyl dodecanol.

The backing layer is in particular substantially impermeable to cytisine.

In specific embodiments, the self-adhesive layer structure according to the invention comprises an additional skin contact layer. The additional skin contact layer is self-adhesive and provides for adhesion between the self-adhesive layer structure and the skin of the patient during administration. In such embodiments, the self-adhesive layer structure may or may not comprise a membrane which is located between the cytisine-containing layer and the additional skin contact layer, wherein the membrane is preferably a rate controlling membrane.

In another embodiment, the self-adhesive layer structure according to the invention does not comprise an additional skin contact layer. Sufficient adhesion between the self-adhesive layer structure and the skin of the patient during administration is then provided for by other means, e.g., a cytisine-containing layer and/or an adhesive overlay.

Thus, according to certain embodiments, the TTS according to the invention may further comprise an adhesive overlay or does not comprise an adhesive overlay, and preferably does not comprise an adhesive overlay. This adhesive overlay is in particular larger than the cytisine-containing self-adhesive layer structure and is attached thereto for enhancing the adhesive properties of the overall transdermal therapeutic system. Said adhesive overlay comprises also a backing layer. The area of said adhesive overlay adds to the overall size of the TTS but does not add to the area of release. The adhesive overlay comprises a self-adhesive polymer or a self-adhesive polymer mixture selected from the group of acrylic polymers, polyisobutylenes, styrene-isoprene-styrene block copolymers, polysiloxanes, and mixtures thereof, which may be identical to or different from any polymer or polymer mixture included in the cytisine-containing self-adhesive layer structure.

The area of release of the TTS according to the invention may range from 5 to 100 $cm^2$ or from 5 to 80 $cm^2$. In certain preferred embodiments, the transdermal therapeutic system has an area of release of from 20 to 70 $cm^2$, preferably from 30 to 50 $cm^2$, more preferably from 33 to 44 $cm^2$, most preferably from 35 to 40 $cm^2$. In particular, the area of release is 40 $cm^2$ or less, such as 35 $cm^2$ or less, 30 $cm^2$ or less, or 25 $cm^2$ or less.

In certain embodiments, the transdermal therapeutic system contains at least 0.25 mg/$cm^2$, preferably at least 0.45 mg/$cm^2$, more preferably at least 0.70 mg/$cm^2$ and most preferably at least 0.95 mg/$cm^2$ cytisine per area of release, such as at least 1.0 mg/$cm^2$, at least 1.05 mg/$cm^2$, or at least 1.1 mg/$cm^2$ cytisine per area of release. In the same or other embodiments, the transdermal therapeutic system contains less than 2.0 mg/$cm^2$, less than 1.5 mg/$cm^2$, less than 1.2 mg/$cm^2$, or less than 1.0 mg/$cm^2$ cytisine per area of release, such as less than 0.9 mg/$cm^2$, less than 0.8 mg/$cm^2$, less than 0.7 mg/$cm^2$, less than 0.6 mg/$cm^2$, less than 0.5 mg/$cm^2$, less than 0.4 mg/$cm^2$, or less than 0.3 mg/$cm^2$ cytisine per area of release.

In some preferred embodiments, the TTS according to the invention contains from 0.25 mg/$cm^2$ to 2.0 mg/$cm^2$, from 0.25 mg/$cm^2$ to 1.5 mg/$cm^2$, or from 0.25 mg/$cm^2$ to 1.2 mg/$cm^2$ cytisine per area of release, preferably from 0.45 mg/$cm^2$ to 1.2 mg/$cm^2$, from 0.70 mg/$cm^2$ to 1.2 mg/$cm^2$, or from 0.95 mg/$cm^2$ to 1.2 mg/$cm^2$, most preferably from 0.95 mg/$cm^2$ to 1.1 mg/$cm^2$ cytisine per area of release. In other preferred embodiments, the TTS according to the invention contains from 0.25 mg/$cm^2$ to 1.0 mg/$cm^2$, from 0.3 mg/$cm^2$ to 1.0 mg/$cm^2$, or from 0.45 mg/$cm^2$ to 1.0 mg/$cm^2$ cytisine per area of release, preferably from 0.45 mg/$cm^2$ to 0.6 mg/$cm^2$, or from 0.45 mg/$cm^2$ to 0.5 mg/$cm^2$ cytisine per area of release.

The self-adhesive layer structure according to the invention is normally located on a detachable protective layer (release liner) from which it is removed immediately before application to the surface of the patient's skin. Thus, the TTS according to the invention may further comprise a release liner. A TTS protected this way is usually stored in a seam-sealed pouch. The packaging may be child resistant and/or senior friendly.

## CYTISINE-CONTAINING LAYER

As outlined in more detail above, the TTS according to the invention comprises a self-adhesive layer structure comprising *inter alia* a cytisine-containing layer, which comprises

1. cytisine, preferably cytisine in the form of the free base,
2. a polymer selected from the group consisting of silicone-based polymers and polyisobutylenes, and
3. a permeation enhancer comprising an organic compound having a linear backbone including 6 to 14 carbon atoms and provided with 1 hydroxy group.

In certain embodiments, the cytisine-containing layer further comprises an additional polymer. Thus, in certain preferred embodiments, the TTS according to the invention comprises a self-adhesive layer structure comprising *inter alia* a cytisine-containing layer, which preferably comprises

1. cytisine, preferably cytisine in the form of the free base,
2. a polymer selected from the group consisting of silicone-based polymers and polyisobutylenes,
3. an additional polymer, and
4. a permeation enhancer comprising an organic compound having a linear backbone including 6 to 14 carbon atoms and provided with 1 hydroxy group.

Without wishing to be bound by theory, it is believed that the advantageous properties of the TTS according to the present invention, such as the good *in vitro* skin permeation, suitability for 24 hours dosing intervals as well as high active ingredient utilization are *inter alia* achieved by the selection of the polymer in the cytisine-containing layer, which is selected from the group consisting of silicone-based polymers and polyisobutylenes and/or by the presence of a permeation enhancer comprising an organic compound having a linear backbone including 6 to 14 carbon atoms and provided with (exactly) 1 hydroxy group. In particular, it has been found that silicone-based polymers or polyisobutylenes are particularly advantageous for providing TTS suitable for 24 hours dosing intervals (i.e., for adjusting the release profile of the TTS in x direction). Further, permeations enhancers as defined herein, in particular a mixture consisting of propylene glycol monocaprylate and propylene glycol dicaprylate in a ratio of 90: 10 or dodecan-1-ol, have been found to be advantageous for providing a permeation rate which is sufficient for achieving a therapeutically effective dose (i.e., for adjusting the release profile of the TTS in y direction).

Thus, in a preferred embodiment, the cytisine-containing layer comprises a permeation enhancer comprising a mixture consisting of propylene glycol monocaprylate and propylene glycol dicaprylate or dodecan-1-ol. Preferably, the cytisine-containing layer does not comprise a permeation enhancer selected from the group consisting of propylene glycol, dipropylene glycol, glycerol triacetate, glycerol monocaprylate, diethyl sebacate, propylene glycol monolaurate, isopropyl palmitate and octyl dodecanol.

In a specific embodiment, the cytisine-containing layer comprises

1. cytisine in an amount of from 2 to 25 %, preferably 2.5 to 18 %, more preferably 4 to 12 %, and most preferably of about 5% or about 10 %,
2. a polymer selected from the group consisting of silicone-based polymers and polyisobutylenes in an amount of from 55 to 97 %, preferably from 60 to 75 % or from 68 to 82 %,
3. an additional polymer in an amount of from 0 to 30 % or from 0.1 to 30 %, preferably 1 to 25 %, more preferably 2 to 22 %, and most preferably from 2 to 12 % or from 18 to 22 %, and
4. a permeation enhancer comprising an organic compound having a linear backbone including 6 to 14 carbon atoms and provided with 1 hydroxy group in an amount of from 1 to 20 %, preferably from 2 to 15 %, more preferably from 4 to 12%, and most preferably from 5 to 11 %,

based on the cytisine-containing layer.

Preferably, the cytisine-containing layer does not comprise any of acrylic polymers or styrene-isoprene-styrene block copolymers in an amount of more than 55 %, preferably more than 30 %, and most preferably more than 22 % of the cytisine-containing layer. In particular, the cytisine-containing layer preferably does not comprise an acrylic polymer containing hydroxyl group, carboxylic acid groups and/or neutralized carboxylic acid groups.

Further, in certain embodiments, the area weight of the cytisine-containing layer ranges from 70 to 200 $g/m^2$, preferably from 80 to 160 $g/m^2$, and most preferably from 85 to 130 $g/m^2$.

**CYTISINE**

In accordance with the invention, the self-adhesive layer structure contains cytisine, in particular in a therapeutically effective amount. In certain embodiments, the amount of cytisine in the cytisine-containing layer ranges from 2 to 25 %, preferably from 2.5 to 18 %, more preferably from 4 to 12 %, and is most preferably about 5 % or about 10 % by weight based on the total weight of the cytisine-containing layer.

While in accordance with the present invention, the active agent may be present in the TTS in protonated or in free

base form, the free base form is preferred. Thus, in certain embodiments, the cytisine in the cytisine-containing layer is included in the form of the free base. In the same or other embodiments, the cytisine in the cytisine-containing layer is included as a pharmaceutically acceptable salt thereof.

In particular, at least 90 mol%, preferably at least 95 mol%, more preferably at least 98 mol% and most preferably at least 99 mol% of the cytisine in the cytisine-containing layer is present in the form of the free base.

In one embodiment, the cytisine-containing layer is obtainable by incorporating the cytisine in the form of the free base.

As outlined above, the TTS of the invention provides for a high active ingredient utilization and a permeation rate which is sufficient for a continuous administration over 24 hours. Typically, a therapeutically effective amount of cytisine is released from the TTS over a dosing interval of 24 hours.

**POLYMER**

As outlined above, the TTS according to the present invention comprises a self-adhesive layer structure comprising a cytisine-containing layer, which comprises a polymer.

This polymer may provide for sufficient cohesion of the cytisine-containing layer. According to certain embodiments, the polymer may also provide for sufficient adhesion of the cytisine-containing layer. In those embodiments, the polymer is selected from pressure sensitive adhesive polymers, in particular the polymer is a pressure-sensitive adhesive or a mixture of pressure-sensitive adhesives.

Polymers which are suitable as the polymer in accordance with the invention are silicone-based polymers and polyisobutylenes. These polymers allow for improved permeability of cytisine compared to formulations based on, e.g., acrylic polymers or styrene-isoprene-styrene block copolymers. In particular, these polymers are advantageous in terms of the active ingredient utilization and the permeation properties of the TTS. The resulting TTS according to the invention are particularly suitable for dosing intervals of 24 hours.

Thus, in some embodiments, the polymer may be selected from silicone-based polymers, and in particular is at least one silicone-based polymer or a combination of silicone-based polymers. In other embodiments, the polymer may be selected from polyisobutylenes, and in particular is at least one polyisobutylene or a combination of polyisobutylenes.

Silicone-based polymers are based on polysiloxanes. They may therefore also be referred to as polymers based on polysiloxanes. Silicone-based polymers are generally obtainable by polycondensation of silanol endblocked polydimethylsiloxane with a silicate resin. Amine-compatible silicone-based polymers can be obtained by reacting the silicone-based polymer with trimethylsilyl (e.g., hexamethyldisilazane) in order to reduce the silanol content of the polymer and thus provide enhanced stability in the presence of amines. As a result, the residual silanol functionality is at least partly, preferably mostly or fully capped with trimethylsiloxy groups.

Thus, in a preferred embodiment, the silicone-based polymer is an amine-compatible polysiloxane, and preferably is obtainable by polycondensation of a silanol endblocked polydimethylsiloxane with a silicate resin followed by at least partial trimethylsilylation of the residual silanol functionality.

In certain embodiments, the silicone-based polymer is pressure sensitive adhesive or a mixture of pressure sensitive adhesives, i.e., a pressure sensitive adhesive based on polysiloxanes, or a mixture of pressure sensitive adhesives based on polysiloxanes.

Pressure sensitive adhesives based on polysiloxanes provide for suitable tack and for quick bonding to various skin types, including wet skin, suitable adhesive and cohesive qualities, long lasting adhesion to the skin, a high degree of flexibility, a permeability to moisture, and compatibility to many actives and film-substrates. Such pressure sensitive adhesives are based on a resin-in-polymer concept wherein, by condensation reaction of silanol endblocked polydimethylsiloxane with a silica resin (also referred to as silicate resin), a pressure sensitive adhesive based on polysiloxane is prepared. For amine stability, the residual silanol functionality is additionally capped with trimethylsiloxy groups. The silanol endblocked polydimethylsiloxane content contributes to the viscous component of the visco-elastic behavior, and impacts the wetting and the spreadability properties of the adhesive. The resin acts as a tackifying and reinforcing agent, and participates in the elastic component. The correct balance between silanol endblocked polydimethylsiloxane and resin provides for the correct adhesive properties.

As indicated before, the tackiness of the silicone-based polymer may be modified by the resin-to-polymer ratio, i.e. the ratio of the silanol endblocked polydimethylsiloxane to the silicate resin, which is preferably in the range of from 50:50 to 70:30, preferably from 55:45 to 65:35. The tackiness will be increased with increasing amounts of the polydimethylsiloxane relative to the resin. High tack silicone-based polymers preferably have a resin-to-polymer ratio of 55:45, medium tack silicone-based polymers preferably have a resin-to-polymer ratio of 60:40, and low tack silicone-based polymers preferably have a resin-to-polymer ratio of 65:35.

According to certain embodiments, the pressure sensitive adhesive is obtainable by polycondensation of a silanol endblocked polydimethylsiloxane with a silicate resin, preferably with a resin-to-polymer ratio of from 50:50 to 70:30 and particularly preferably of 55:45, 60:40 or 65:35. Thus, in a preferred embodiment, the silicone-based polymer is a

mixture of pressure sensitive adhesives obtainable by polycondensation of a silanol endblocked polydimethylsiloxane with a silicate resin with a resin-to-polymer ratio of 55:45 or of 60:40.

Further, according to certain embodiments, the silicone-based polymer is a mixture of pressure sensitive adhesives with

a solution viscosity at 25 °C and about 60 % solids content in heptane of 450 mPa s and/or a complex viscosity at 0.01 rad/s at 30 °C of $1\times10^8$ Poise, and

a solution viscosity at 25 °C and about 60 % solids content in heptane of 500 mPa s and/or a complex viscosity at 0.01 rad/s at 30 °C of $5\times10^6$ Poise.

The pressure sensitive adhesives based on polysiloxanes are supplied and used in solvents like n-heptane, ethyl acetate or other volatile silicone fluids. The solids content of pressure sensitive adhesives based on polysiloxanes in solvents is usually between 60 and 85 %, preferably between 70 and 80 % or between 60 and 75 %. The skilled person is aware that the solids content may be modified by adding a suitable amount of solvent.

High tack silicone-based polymers preferably have a complex viscosity at 0.01 rad/s and 30 °C of about 5 x $10^6$ Poise, medium tack silicone-based polymers preferably have a complex viscosity at 0.01 rad/s and 30 °C of about 5 x $10^7$ Poise, and low tack silicone-based polymers preferably have a complex viscosity at 0.01 rad/s and 30 °C of about 5 x $10^8$ Poise. High tack amine-compatible silicone-based polymers preferably have a complex viscosity at 0.01 rad/s and 30 °C of about 5 x $10^6$ Poise, medium tack amine-compatible silicone-based polymers preferably have a complex viscosity at 0.01 rad/s and 30 °C of about 5 x $10^8$ Poise, and low tack amine-compatible silicone-based polymers preferably have a complex viscosity at 0.01 rad/s and 30 °C of about 5 x $10^9$ Poise. Preferred pressure sensitive adhesives based on polysiloxanes in accordance with the invention are characterized by a solution viscosity at 25 °C and 60 % solids content in n-heptane of more than about 150 mPa s, or from about 200 mPa s to about 700 mPa s, preferably as measured using a Brookfield RVT viscometer equipped with a spindle number 5 at 50 rpm. Theses may also be characterized by a complex viscosity at 0.01 rad/s at 30 °C of less than about 1 x $10^9$ Poise or from about 1 x $10^5$ to about 9 x $10^8$ Poise.

Suitable silicone-based polymers are commercially available under the brand names BIO-PSAs. Examples of silicone-based PSA compositions which are commercially available include the standard Liveo™ BIO-PSA series (7-4400,7-4500 and 7-4600 series) and the amine compatible (endcapped) Liveo™ BIO-PSA series (7-4100, 7-4200 and 7-4300 series) manufactured and typically supplied in n-heptane or ethyl acetate. For example, BIO-PSA 7-4201 is characterized by a solution viscosity at 25 °C and about 60 % solids content in heptane of 450 mPa s and a complex viscosity at 0.01 rad/s at 30 °C of $1\times10^8$ Poise. BIO-PSA 7-4301 has a solution viscosity at 25 °C and about 60 % solids content in heptane of 500 mPa s and a complex viscosity at 0.01 rad/s at 30 °C of $5\times10^6$ Poise.

Pressure sensitive adhesives based on polysiloxanes may be obtained according to the following scheme:

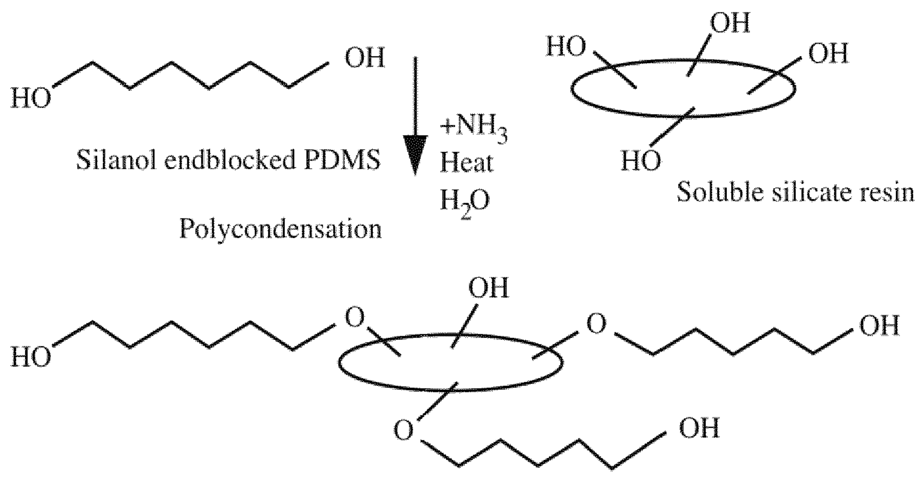


Such pressure sensitive adhesives based on polysiloxanes are available under the tradenames Liveo™ BIO-PSA 7-4401, BIO-PSA-7-4501, or BIO-PSA 7-4601, which are provided in the solvent n-heptane (indicated by the code "01"), or under the tradenames Liveo™ BIO-PSA 7-4402, BIO-PSA 7-4502, and BIO 7-4602, which are provided in the solvent ethyl acetate (indicated by the code "02"). Typical solids contents in the solvent are in the range of from 60 to 75 %. The code "44" indicates a resin-to-polymer ratio of 65:35 resulting in a low tackiness, the code "45" indicates a resin-to-polymer ratio of 60:40 resulting in medium tackiness, the code "46" indicates a resin-to-polymer ratio of 55:45 resulting in high tackiness.

Amine-compatible pressure sensitive adhesives based on polysiloxanes may be obtained according to the following scheme:

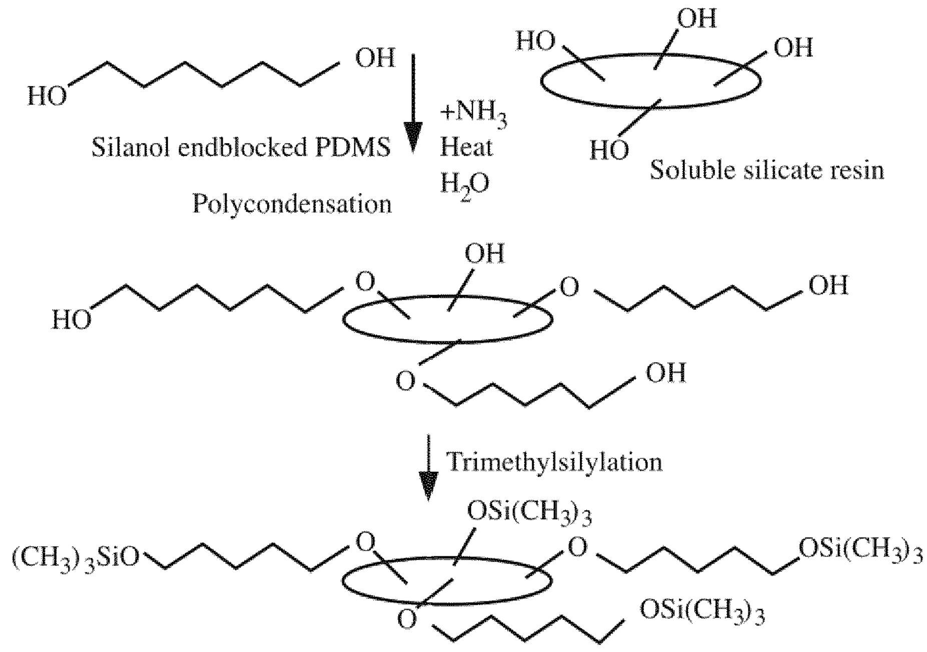


Such amine-compatible pressure sensitive adhesives based on polysiloxanes are available under the tradenames Liveo™ BIO-PSA 7-4101, BIO-PSA-7-4201, or BIO-PSA 7-4301, which are provided in the solvent n-heptane (indicated by the code "01"), or under the tradenames Liveo™ BIO-PSA 7-4102, BIO-PSA 7-4202, and BIO 7-4302, which are provided in the solvent ethyl acetate (indicated by the code "02"). Typical solids contents in the solvent are in the range of from 60 to 75 %. The code "41" indicates a resin-to-polymer ratio of 65:35 resulting in a low tackiness, the code "42" indicates a resin-to-polymer ratio of 60:40 resulting in medium tackiness, the code "43" indicates a resin-to-polymer ratio of 55:45 resulting in high tackiness.

Suitable polyisobutylenes as used herein are available under the tradename Oppanol®. Combinations of high-molecular weight polyisobutylenes (B100, B80) and low-molecular weight polyisobutylenes (B10, B11, B12, B13) may be used. Suitable ratios of low-molecular weight polyisobutylene to high-molecular weight polysisobutylene are in the range of from 100:1 to 1:100, preferably from 95:5 to 40:60, more preferably from 90:10 to 75:25. A preferred example for a polyisobutylene combination is B10/B100 in a ratio of 85/15, or B12/B100 in a ratio of 80/20. Oppanol® B100 has a viscosity average molecular weight Mv of 1,110,000, and a weight average molecular weight Mw of 1,550,000, and an average molecular weight distribution Mw/Mn of 2.9. Oppanol® B10 has a viscosity average molecular weight Mv of 40,000, and a weight average molecular weight Mw of 53,000, and an average molecular weight distribution Mw/Mn of 3.2. Oppanol® B12 has a viscosity average molecular weight Mv of 55,000, and a weight average molecular weight Mw of 70,000, and an average molecular weight distribution Mw/Mn of 3.2. A suitable polyisobutylene adhesive is also commercially available e.g. under the brand name Duro-Tak™ 87-6908.

In certain embodiments, the polymer which is selected from silicone-based polymers and polyisobutylenes is present in the cytisine-containing layer in an amount of more than 50 %, more than 55 %, more than 60 %, more than 65 %, more than 70 %, more than 75 %, or more than 80 %, and or in an amount of less than 90 %, less than 85 %, or less than 80 % based on the total weight of the cytisine-containing layer. Preferably, the amount ranges from 55 to 97 %, preferably from 60 to 75 % or from 68 to 82 % based on the total weight of the cytisine-containing layer. If the polymer, which is preferably present in the cytisine-containing layer in an amount as defined above, is selected from silicone-based polymers, it is to be understood that also combinations of silicone-based polymers are covered. If the polymer, which is preferably present in the cytisine-containing layer in an amount as defined above, is selected from polyisobutylenes, it is to be understood that also combinations of polyisobutylenes are covered.

In some embodiments, the polymer is at least one silicone-based polymer, in particular one type of silicone-based polymer or a combination of two different types of silicone-based polymers, and is present in the cytisine-containing layer in an amount of more than 65 %, preferably more than 70 %, and more preferably more than 75 %, such as from 68 to 82 % based on the total weight of the cytisine-containing layer. In one embodiment, only one type of silicone-based polymers is present in the cytisine-containing layer, which is preferably selected from either medium or high tack silicone-based polymers. In another embodiment, a mixture of two different types of silicone-based polymers is present in the cytisine-containing layer, wherein preferably the first one is selected from medium tack silicone-based polymers and the other one is selected from high tack silicone-based polymers. Preferably, the ratio of the medium tack silicone-based polymer to the high tack silicone-based polymer is in the range of from 40:60 to 60:40 or from 45:55 to 55:40, in particular the ratio of the

medium tack silicone-based polymer to the high tack silicone-based polymer is about 50:50 or 1: 1, e.g., each of the medium tack silicone-based polymer and the high tack silicone-based polymer is present in the cytisine-containing layer in an amount of about 35 %, or about 40 % based on the total weight of the cytisine-containing layer.

In other embodiments, the polymer is at least one polyisobutylene and may be a combination of two different types of polyisobutylenes, in particular a combination of low-molecular weight polyisobutylene and high-molecular weight polyisobutylene, and is preferably present in the cytisine containing layer in an amount of more than 55 %, preferably more than 60 %, and more preferably more than 65 %, such as from 60 to 75 % based on the total weight of the cytisine-containing layer. Preferably, the ratio of the low-molecular weight polyisobutylene to the high-molecular weight polyisobutylene is in the range of from 75:25 to 90: 10, in particular the ratio of the low-molecular weight polyisobutylene to the high-molecular weight polyisobutylene is about 80:20.

In view of the above, as the polymer is advantageously selected from the group consisting of silicone-based polymers and polyisobutylenes, the polymer as used in the cytisine-containing layer of the self-adhesive layer structure of the TTS according to the invention is preferably not selected from the group consisting of acrylic polymers and styrene-isoprene-styrene block copolymers. In particular, the polymer as used in the cytisine-containing layer of the self-adhesive layer structure of the TTS according to the invention is neither an acrylic polymer nor a styrene-isoprene-styrene block copolymer.

## ADDITONAL POLYMER

As outlined above, the TTS according to the present invention comprises a self-adhesive layer structure comprising a cytisine-containing layer, which may further comprise an additional polymer (i.e. a polymer in addition to the polymer described above). In particular, the cytisine-containing layer comprises an additional polymer which provides for further increasing the permeation rate and/or blood plasma concentration of cytisine released from the TTS according to the invention.

Of particular interests are polymers with an enhanced ability to absorb water, as higher water and/or moisture absorption assists in maintaining / improving the adhesive properties of the cytisine-containing layer and further in increasing cytisine release from cytisine-containing layer. Thus, in certain embodiments, the cytisine-containing layer further comprises an additional polymer selected from polymers, which provide for an improved water and/or moisture absorption of the cytisine-containing layer. Such polymers are well known in the art.

Of those, particularly suitable and preferred are polyvinylpyrrolidones. Thus, in certain embodiments, the cytisine-containing layer further comprises an additional polymer selected from polyvinylpyrrolidones, in particular an additional polymer which is at least one polyvinylpyrrolidone. The polyvinylpyrrolidone may be an insoluble / cross-linked polyvinylpyrrolidone also known as crospovidone, such as, e.g., Kollidon® CL, Kollidon® CL-M or Kollidon® CL-SF, or a soluble polyvinylpyrrolidone also known as povidone, such as, e.g., Kollidon® 12 PF, Kollidon® 17 PF, Kollidon® 25, Kollidon® 30 or Kollidon® 90 F, preferably a soluble polyvinylpyrrolidone having a K-Value within a range selected from the group of ranges consisting of

9 to 15, and preferably 10.2 to 13.8,
15 to 20, and preferably 15.3 to 18.4,
20 to 27, and preferably 22.5 to 27.0,
27 to 35, and preferably 27.0 to 32.4, and
75 to 110, and preferably 81.0 to 97.2,

or any mixtures thereof, and more preferably a polyvinylpyrrolidone having a K-Value within a range of 27.0 to 32.4 or of 81.0 to 97.2, and any mixtures thereof, and most preferably a polyvinylpyrrolidone having a K-Value within range of 27.0 to 32.4.

The at least one polyvinylpyrrolidone may be present in the cytisine-containing layer for example in an amount of from 2 to 12 % or from 18 to 22 % of the cytisine-containing layer, such as in an amount of about 5 % or about 20% of the cytisine-containing layer.

Likewise, particularly suitable and preferred are basic acrylate polymers. Thus, in certain embodiments, the cytisine-containing layer further comprises an additional polymer selected from basic polymers, in particular an additional polymer which is at least one basic polymer. Preferably, the additional polymer is selected from amine-functional acrylates, in particular the additional polymer is at least one amine-functional acrylate. More preferably, the additional polymer is a basic aminoalkyl methacrylate copolymer. Particularly preferably, the additional polymer is a copolymer based on dimethylaminoethyl methacrylate, butyl methacrylate and methyl methacrylate. For example, such copolymer is commercially available as Eudragit® E100 supplied by Evonik and has a ratio of dimethylaminoethyl methacrylate, butyl methacrylate, and methyl methacrylate of 2:1:1.

Alternatively preferred are neutral methacrylic acid copolymers, such as a copolymer based on trimethylammonioethyl methacrylate chloride, ethyl acrylate and methyl methacrylate. Examples of such copolymers which are commercially available include Eudragit® RL100 and Eudragit® RS100 supplied by Evonik. Eudragit® RL100 and Eudragit® RS100 have a ratio of trimethylammonioethyl methacrylate chloride, ethyl acrylate and methyl methacrylate of 0.2:1:2 or 0.1:1:2, respectively.

The at least one basic aminoalkyl methacrylate copolymer or neutral methacrylic acid copolymer may be present in the cytisine-containing layer for example in an amount of from 2 to 12 % of the cytisine-containing layer, such as in an amount of about 5 % or about 10% of the cytisine-containing layer.

Other or the same additional polymers may also be added to enhance cohesion and/or adhesion, or to be advantageous in reducing cold flow.

In certain preferred embodiments, the additional polymer does not comprise any of hydroxyl groups, carboxylic acid groups and/or neutralized carboxylic acid groups.

According to certain embodiments, the amount of the additional polymer in the cytisine-containing layer ranges from 0.1 to 30 %, preferably from 1 to 25 %, more preferably from 2 to 22 %, and most preferably from 2 to 12 % or from 18 to 22 % based on the total weight of the cytisine-containing layer.

## PERMEATION ENHANCER

As outlined above, the TTS according to the present invention comprises a self-adhesive layer structure comprising a cytisine-containing layer, which comprises a permeation enhancer comprising an organic compound having a linear backbone including 6 to 14 carbon atoms and provided with 1 hydroxy group.

Preferably, the permeation enhancer comprises at least one C6 to C14 alcohol, or at least one hydroxy-functionalized C6 to C14 (di)ether or ester. More preferably, the permeation enhancer comprises at least one C11 or C12 alcohol or at least one hydroxy-functionalized C11 or C12 ester.

In certain embodiments, the permeation enhancer comprises at least one ester or alcohol having 11 or 12 carbon atoms and provided with 1 hydroxy group.

The permeation enhancers as defined herein may provide for increasing the cytisine release rate from the TTS according to the invention. Without wishing to be bound by theory, it is believed that the surfactant-like structure of organic compound comprised in the permeation enhancers, i.e., the aliphatic carbon chain and the polar hydroxy group, may advantageously allow for interacting with cytisine and/or influencing the barrier properties of the skin, resulting in an increased cytisine release. It is also known that the skin permeability also depends on the size of a given permeate.

Thus, in certain embodiments, the permeation enhancer, in particular the organic compound having a linear backbone including 6 to 14 carbon atoms and provided with 1 hydroxy group, i.e., the surfactant, has a molecular weight of not less than 150 g/mol, preferably not less than 175 g/mol, and most preferably not less than 185 g/mol, and/or the permeation enhancer has a molecular weight of not more than 210 g/mol, preferably not more than 205 g/mol, and most preferably not more than 202.5 g/mol. Preferably, the permeation enhancer has a molecular weight of from 185 g/mol to 202.5 g/mol. In particular, the permeation enhancer has a molecular weight of about 186 g/mol or of about 202 g/mol.

In some embodiments, the permeation enhancer comprises at least one ester having 11 carbon atoms and provided with 1 hydroxy group. Such esters may comprise hydroxy-functionalized esters selected from the group consisting of formic acid decyl ester, acetic acid nonyl ester, propanoic acid octyl ester, butanoic acid heptyl ester, valeric acid hexyl ester, caproic acid pentyl ester, enanthic acid butyl ester, caprylic acid propyl ester, pelargonic acid ethyl ester, and capric acid methyl ester. Preferably, the permeation enhancer comprises a mixture of propylene glycol monoesters and diesters of fatty acids. Particularly preferably, the permeation enhancer comprises at least caprylic acid 2-hydroxypropyl ester, also referred to as propylene glycol monocaprylate. In certain preferred embodiments, the permeation enhancer comprises a mixture consisting of propylene glycol monocaprylate and propylene glycol dicaprylate, in particular in a ratio of propylene glycol monocaprylate to propylene glycol dicaprylate of at least 75:25, preferably at least 85:15, and most preferably at least 90:10. In certain particularly preferred embodiments, the permeation enhancer is a mixture consisting or propylene glycol monocaprylate and propylene glycol dicaprylate in a ratio of 90:10. Such mixture of propylene glycol esters of caprylic acid, mainly composed of monoesters and further comprising a small fraction of diesters with a ratio of no less than 90 % monoesters and not more than 10 % diesters, which is also referred to as propylene glycol monocaprylate type II, is commercially available as Capryol® 90.

In other embodiments, the permeation enhancer comprises at least one alcohol having 12 carbon atoms and provided with 1 hydroxy group, such as dodecan-1ol, dodecan-2-ol, dodecan-3-ol, dodecan-4-ol or dodecan-5-ol. In certain preferred embodiments, the permeation enhancer is dodecan-1-ol.

According to certain embodiments, the amount of the permeation enhancer in the cytisine-containing layer ranges from 1 to 20 %, preferably from 2 to 15 %, more preferably from 4 to 12 %, and most preferably from 5 to 11 % based on the total weight of the cytisine-containing layer.

## FURTHER ADDITIVES

The transdermal therapeutic system according to the invention, and in particular the cytisine-containing layer may further comprise at least one additive or excipient. Said additives or excipients are preferably selected from the group consisting of additional polymers or permeation enhancers (see aboe), solubilizers, fillers, substances for skincare, pH regulators, preservatives, tackifiers, softeners, plastizisers and stabilizers. Such additives may be present in the cytisine-containing layer in an amount of from 0.001 to 15 % by weight, e.g., from 1 to 10 % by weight or from 0.01 to 5 % by weight, based on the total weight of the cytisine-containing layer.

It should be noted that in pharmaceutical formulations, the formulation components are categorized according to their physicochemical and physiological properties, and in accordance with their function. This means in particular that a substance or a compound falling into one category is not excluded from falling into another category of formulation component. E.g., a certain polymer can be a crystallization inhibitor but also a tackifier. Some substances may e.g., be a typical softener but at the same time act as a permeation enhancer. The skilled person is able to determine based on his general knowledge in which category or categories of formulation component a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention. Hereinafter, where a range for an amount of a specific additive is given, such a range refers to the amount per individual additive.

In certain embodiments, the TTS, in particular the cytisine-containing layer may further comprise a stabilizer, wherein the stabilizer is preferably selected from tocopherol and ester derivatives thereof and ascorbic acid and ester derivatives thereof. Preferred stabilizers include sodium metabisulfite, ascorbyl esters of fatty acids such as ascorbyl palmitate, ascorbic acid, butylated hydroxytoluene, tocopherol, tocopheryl acetate and tocopheryl linoleate.

In certain embodiments, the TTS, in particular the cytisine-containing layer may further comprise a softener/ plasticizer. Exemplary softeners/plasticizers include linear or branched, saturated or unsaturated alcohols having 6 to 20 carbon atoms, triglycerides and polyethylene glycols.

In certain embodiments, the TTS, in particular the cytisine-containing layer may further comprise a solubilizer. The solubilizer preferably improves the solubility of cytisine in the cytisine-containing layer.

In certain embodiments, the TTS, in particular the cytisine-containing layer may further comprise a pH regulator. Suitable pH regulators include mild acids and bases including amine derivatives, inorganic alkali derivatives, and polymers with basic or acidic functionality.

In certain embodiments, the TTS, in particular the cytisine-containing layer may further comprise a preservative. Suitable preservatives include parabens, formaldehyde releasers, isothiazolinones, phenoxyethanol, and organic acids such as benzoic acid, sorbic acid, levulinic acid and anisic acid.

In certain embodiments, the TTS, in particular the cytisine-containing layer may further comprise a substance for skincare. Such substances may be used to avoid or reduce skin irritation as detectable by the dermal response score. Suitable substances for skincare include sterol compounds such as cholesterol, dexpanthenol, alpha-bisabolol, and antihistamines.

Fillers such as silica gels, titanium dioxide and zinc oxide may be used in conjunction with the silicone-based polymer or polyisobutylenes in order to influence certain physical parameters, such as cohesion and bond strength, in the desired way.

In case the cytisine-containing layer is required to have self-adhesive properties and one or more polymers is/are selected which does/do not provide sufficient self-adhesive properties, a tackifier is added. The tackifier may be selected from polyvinylpyrrolidone (which, due to its ability to absorb water, is able to maintain the adhesive properties of the matrix layer and thus can be regarded as a tackifier in a broad sense), triglycerides, polyethylene glycols, dipropylene glycol, terpenes, and derivatives thereof, ethylene vinyl acetate adhesives, dimethylpolysiloxanes and polybutenes, preferably polyvinylpyrrolidone and more preferably soluble polyvinylpyrrolidone.

## RELEASE CHARACTERISTICS

The TTS according to the invention are designed for transdermally administering cytisine to the systemic circulation for a predefined extended period of time.

In certain embodiments, the transdermal therapeutic system provides a mean release rate of 1 to 20 mg/day, 1 to 20 mg/24 h, 1000 to 20,000 μg/day, 1000 to 20,000 μg/24 h, preferably of 2 to 12 mg/day, 2 to 12 mg/24 h, 2,000 to 12,000 μg/day, 2,000 to 12,000 μg/24 h, and more preferably of 3 to 10 mg/day, 8 to 10 mg/24 h, 3,000 to 10,000 μg/day, 3,000 to 10,000 μg/24 h over at least 24 hours of administration. In particular, the transdermal therapeutic system provides a mean release rate of 5 to 15 mg/day, 5 to 15 mg/24 h, 5,000 to 15,000 μg/day, 5,000 to 15,000 μg/24 h, or of 8 to 12 mg/day, 8 to 12 mg/24 h, 8,000 to 12,000 μg/day, 8,000 to 12,000 μg/24 h over at least 24 hours of administration. In some specific

embodiments, the TTS according to the invention provides a mean release rate of about 9 mg/day, 9 mg/24 h, 9,000 µg/day, 9,000 µg/24 h, or about 7.5 mg/day, 7.5 mg/24 h, 7,500 µg/day, 7,500 µg/24 h, or about 6 mg/day, 6 mg/24 h, 6,000 µg/day, 6,000 µg/24 h, or about 4.5 mg/day, 4.5 mg/24 h, 4,500 µg/day, 4,500 µg/24 h, or about 3 mg/day, 3 mg/24 h, 3,000 µg/day, 3,000 µg/24 h, or about 1.5 mg/day, 1.5 mg/24 h, 1,500 µg/day, 1,500 µg/24 h over at least 24 hours of administration.

According to certain embodiments, the transdermal therapeutic system provides a cumulative permeated amount of cytisine as measured in a Franz diffusion cell with dermatomed human skin of at least 150 µg/cm$^2$, at least 210 µg/cm$^2$, at least 250 µg/cm$^2$, at least 270 µg/cm$^2$, at least 280 µg/cm$^2$, or at least 285 µg/cm$^2$ after a time period of 24 hours. Preferably, the transdermal therapeutic system provides a cumulative permeated amount of cytisine of at least 290 µg/cm$^2$, at least 310 µg/cm$^2$, at least 320 µg/cm$^2$, or at least 325 µg/cm$^2$ after a time period of 24 hours. More preferably, the transdermal therapeutic system provides a cumulative permeated amount of cytisine of at least 290 µg/cm$^2$, at least 310 µg/cm$^2$, at least 320 µg/cm$^2$, or at least 325 µg/cm$^2$ after a time period of 24 hours. Alternatively, the transdermal therapeutic system provides a cumulative permeated amount of cytisine of at least 360 µg/cm$^2$, at least 400 µg/cm$^2$, at least 415 µg/cm$^2$, at least 425 µg/cm$^2$, or at least 450 µg/cm$^2$ after a time period of 24 hours.

According to certain embodiments, the transdermal therapeutic system provides a cumulative permeated amount of cytisine as measured in a Franz diffusion cell with dermatomed human skin of 100 µg/cm$^2$ to 600 µg/cm$^2$, preferably 200 µg/cm$^2$ to 550 µg/cm$^2$, more preferably 300 µg/cm2 to 500 µg/cm$^2$ after a time period of 24 hours. In some embodiments, the transdermal therapeutic system provides a cumulative permeated amount of cytisine of 150 µg/cm$^2$ to 500 µg/cm$^2$, preferably 210 µg/cm$^2$ to 400 µg/cm$^2$, more preferably 260 µg/cm$^2$ to 320 µg/cm$^2$ after a time period of 24 hours. In other embodiments, the transdermal therapeutic system provides a cumulative permeated amount of cytisine of 100 µg/cm$^2$ to 400 µg/cm$^2$, preferably 160 µg/cm$^2$ to 300 µg/cm$^2$, more preferably 210 µg/cm$^2$ to 270 µg/cm$^2$ after a time period of 24 hours. In yet other embodiments, the transdermal therapeutic system provides a cumulative permeated amount of cytisine of 200 µg/cm$^2$ to 600 µg/cm$^2$, preferably 300 µg/cm$^2$ to 540 µg/cm$^2$, more preferably 400 µg/cm$^2$ to 490 µg/cm$^2$ after a time period of 24 hours. In specific embodiments, TTS according to the invention, which preferably has an area of release of about 40 cm$^2$, about 35 cm$^2$, about 30 cm$^2$, about 25 cm$^2$ or about 20 cm$^2$, provides a cumulative permeated amount of cytisine of about 225µg/cm$^2$, about 255 µg/cm$^2$, about 300 µg/cm$^2$, about 360 µg/cm$^2$, or about 450 µg/cm$^2$ after a time period of 24 hours. Preferably, the TTS according to the invention having an area of release of 40 cm$^2$ or less provides a cumulative permeated amount of cytisine of at least 3,000 µg/TTS, more preferably at least 6,000 µg/TTS, or most preferably at least 9,000 µg/TTS.

The TTS according to the invention preferably releases at least 25 % of the total amount of cytisine contained in the self-adhesive layer structure within a time period of 24 hours after application, preferably at least 30 %, at least 40 %, at least 50 %, at least 60 %, or at least 70 % of the total amount of cytisine contained in the self-adhesive layer structure within a time period of 24 hours after application. Particularly preferably, the TTS according to the invention releases at least 75 % or at least 80 %, most preferably at least 90 %, of the total amount of cytisine contained in the self-adhesive layer structure within a time period of 24 hours after application.

Preferably, the TTS according to the invention provides the maximum of the skin permeation rate of cytisine as measured in a Franz diffusion cell with dermatomed human skin within a time period of 24 hours after application. In particular, the global maximum of the skin permeation rate of cytisine appears within a time period of 24 hours after application. Thus, the TTS according to the invention are particularly suitable for 24 hours dosing intervals.

## METHOD OF TREATMENT / MEDICAL USE

In accordance with a specific aspect of the present invention, the TTS according to the invention is for use in a method of treatment, and in particular in a method of treating a human patient.

In certain embodiments, the transdermal therapeutic system is for use in a method of treatment, wherein the TTS according to the invention is preferably applied to the skin of the patient for a dosing interval of from 20 to 30 hours. Preferably, the TTS according to the invention is for use in a method of treatment, wherein the TTS is applied to the skin of the patient for a dosing interval of about 24 hours or about 1 day. Alternatively, the TTS according to the invention may be applied to the skin of the patient for a dosing interval of up to three days, up to four days, or up to 7 days. At or after the end of a dosing interval provided for by a first TTS according to the invention, a second TTS according to the invention may be applied. Such second TTS may be applied immediately after removal of the first TTS. In particular, the first, second and any further TTS according to the invention may be applied consecutively. The second, third, and any further TTS may be identical with or may differ from the previously applied TTS. For example, a subsequent TTS may have a lower amount or may provide a lower mean release rate of cytisine than a previously applied TTS.

In a preferred embodiment, the transdermal therapeutic system is for use in a method of treating nicotine addiction, in particular for use in a method of reducing nicotine cravings and/or in a method of (aiding to) smoking cessation. Preferably, the TTS according to the invention is for use in a method of permanent cessation of using or consuming nicotine-containing products.

In connection with the above, the TTS according to the invention is for use in a method of treating a human patient suffering from nicotine addiction. Preferably, the TTS according to the invention is for use in a method of treating a human patient who is willing to quit smoking.

The TTS according to the invention is preferably applied to and/or in contact with at least one body surface on the patient, which may be located at any part of the body, and is in certain embodiments selected from upper outer arm, upper chest, upper back or the side of the chest.

The method of treatment may be performed for a treatment period of at least about 10 days, at least about 18 days, at least about 24 days, or at least about 28 days or about 4 weeks or about 1 month. Also, the method of treatment may be performed for a treatment period of at least about 42 days or about 6 weeks or about 1.5 months, at least about 56 days or about 8 weeks or about 2 months, or at least about 84 days or about 12 weeks or about 3 months. Thus, the TTS according to the invention is preferably for use in a method of treatment, wherein at least one TTS is applied daily for at least about 10, at least about 18, at least about 24, at least about 28, at least about 42, at least about 56, or at least about 84 consecutive days

According to a specific aspect, the present invention is also related to a method of treatment, preferably a method of treating nicotine addiction, in particular a method of reducing nicotine cravings and/or a method of (aiding to) smoking cessation, including applying a TTS as described herein to the skin of a patient.

## PROCESS OF MANUFACTURE

The invention further relates to a process of manufacturing a cytisine-containing layer for use in a transdermal therapeutic system, preferably a cytisine-containing layer of a transdermal therapeutic system, as well as a corresponding self-adhesive layer structure and a corresponding transdermal therapeutic system.

In accordance with the invention, the process of manufacture of a cytisine-containing layer for use in a transdermal therapeutic system comprises the steps of:

1) combining at least the components

   1. cytisine,
   2. a polymer selected from the group consisting of silicone-based polymers and polyisobutylenes, and optionally an additional polymer, and
   3. a permeation enhancer selected from an organic compound having a linear backbone including 6 to 14 carbon atoms and provided with 1 hydroxy group

to obtain a coating composition;

2) coating the coating composition onto the backing layer or a release liner or any intermediate liner; and

3) drying the coated coating composition to form the cytisine-containing layer.

In this process of manufacture, preferably in step 1) the cytisine is dissolved to obtain a coating composition.

In certain embodiments, the polymer is preferably applied by a solvent-based process. Accordingly, the polymer is preferably provided in a solvent, wherein the solids content in the solvent is preferably from 40 to 75 % by weight.

In the above described process, the solvent is preferably selected from alcoholic solvents, in particular methanol, ethanol, isopropanol and mixtures thereof, and from non-alcoholic solvents, in particular ethyl acetate, hexane, heptane, petroleum ether, toluene, and mixtures thereof, and is more preferably selected from non-alcoholic solvents, and is most preferably ethyl acetate or n-heptane.

In step 3), drying is performed preferably at a temperature of from 50 to 90 °C, more preferably from 60 to 80 °C.

## EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

## REFERENCE EXAMPLES 1A-C

### Coating composition

The formulations of the cytisine-containing coating compositions of Reference Examples 1a to 1c are summarized in Table 1.1 below. The formulations are based on weight percent, as also indicated in Table 1.1.

Table 1.1

| Ingredient (Trade Name) | Ref Ex. 1a | | Ref. Ex. 1b | | Ref. Ex. 1c | |
|---|---|---|---|---|---|---|
| | Amt [g] | Solids [%] | Amt [g] | Solids [%] | Amt [g] | Solids [%] |
| Cytisine base | 0.45 | 3.0 | 0.30 | 3.0 | 0.30 | 3.0 |
| Acrylic adhesive in ethyl acetate and hexane. Solids content of 40.5 % by weight (Duro-Tak™ 87-2510[1]) | 33.49 | 91.9 | - | - | - | - |
| Acrylic adhesive in ethyl acetate. Solids content of 39 % by weight (Duro-Tak™ 87-4287[1]) | - | - | 24.02 | 91.9 | - | - |
| Acrylic adhesive in ethyl acetate. Solids content of 36.5 % by weight (Duro-Tak™ 87-9301[2]) | - | - | - | - | 25.14 | 92.0 |
| Propylene glycol | - | - | 0.51 | 5.1 | - | - |
| Propylene glycol monocaprylate type II (Capryol® 90) | - | - | - | - | 0.50 | 5.0 |
| Isopropyl palmitate | 0.76 | 5.1 | - | - | - | - |
| Ethyl acetate | - | - | 1.15 | - | 1.23 | - |
| Total | 34.70 | 100.0 | 25.98 | 100.0 | 27.17 | 100.0 |
| Area Weight [g/m$^2$] | 116.8 | | 98.9 | | 105.1 | |
| Cytisine content [mg/cm$^2$] | 0.351 | | 0.297 | | 0.318 | |
| [1]acrylic copolymer comprising hydroxyl groups, [2]acrylic copolymer without functional groups | | | | | | |

**Preparation of the coating composition**

For Reference Examples 1a to 1c, a beaker was loaded with cytisine base and propylene glycol, propylene glycol monocaprylate type II, or isopropyl palmitate, as applicable, are added. The solvent (ethyl acetate) and acrylic adhesive are added and the mixture was stirred at approx. 500 rpm until a homogeneous mixture was obtained.

**Coating of the coating composition**

The resulting cytisine-containing coating composition was coated on a polyethylene terephthalate film (siliconized, 100 $\mu$m thickness, which may function as release liner) and dried for approx. 15 min at room temperature and approx. 15 min at 60 °C. The coating thickness led to an area weight of the matrix layer of 116.8 g/m$^2$ (Reference Example 1a), 98.9 g/m$^2$ (Reference Example 1b), and 105.1 g/m$^2$ (Reference Example 1c), respectively. The dried film was laminated with a polyethylene terephthalate backing layer (23 $\mu$m thickness) to provide a cytisine-containing self-adhesive layer structure.

**Preparation of the TTS (concerning all examples)**

The individual systems (TTS) were then punched out from the cytisine-containing self-adhesive layer structure. In specific embodiments a TTS as described above can be provided with a further self-adhesive layer of larger surface area, preferably with rounded corners, comprising a pressure-sensitive adhesive matrix layer which is free of active agent. This is of advantage when the TTS, on the basis of its physical properties alone, does not adhere sufficiently to the skin and/or when the cytisine-containing matrix layer, for the purpose of avoiding waste, has pronounced corners (square or rectangular shapes). The TTS are then punched out and sealed into pouches of the primary packaging material.

**Measurement of skin permeation rate**

The permeated amount and the corresponding skin permeation rates of TTS prepared according to Reference Examples 1a to 1c were determined by *in vitro* experiments in accordance with the OECD Guideline (adopted April 13, 2004) carried out with a 10.0 ml Franz diffusion cell. Split thickness human skin from cosmetic surgeries (female abdomen,

date of birth 1989) was used. A dermatome was used to prepare skin to a thickness of 500 μm, with an intact epidermis for all TTS. Diecuts with an area of 1.164 $cm^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 1.2 and 1.3 as well as in Figure 1a.

Table 1.2

| Cumulative permeated amount with SD* [μg/$cm^2$] | | | | | | |
|---|---|---|---|---|---|---|
| **Elapsed time [h]** | **Ref. Ex. 1a (n = 3)** | | **Ref. Ex. 1b (n = 3)** | | **Ref. Ex. 1c (n = 3)** | |
| | **Rate** | **SD** | **Rate** | **SD** | **Rate** | **SD** |
| **0** | 0 | 0 | 0 | 0 | 0 | 0 |
| **4** | 0.0 | 0.00 | 0.0 | 0.00 | 0.0 | 0.00 |
| **8** | 0.3 | 0.27 | 0.1 | 0.16 | 0.5 | 0.53 |
| **24** | 4.2 | 1.36 | 1.6 | 0.55 | 5.2 | 2.90 |
| **32** | 7.7 | 1.79 | 2.8 | 0.75 | 8.5 | 3.94 |
| **48** | 16.6 | 2.66 | 5.7 | 1.25 | 15.0 | 4.94 |
| **72** | 34.7 | 4.48 | 10.9 | 1.96 | 25.4 | 5.32 |

Table 1.3

| Skin permeation rate with SD* [μg/($cm^2$ h)] | | | | | | |
|---|---|---|---|---|---|---|
| **Elapsed time [h]** | **Ref. Ex. 1a (n = 3)** | | **Ref. Ex. 1b (n = 3)** | | **Ref. Ex. 1c (n = 3)** | |
| | **Rate** | **SD** | **Rate** | **SD** | **Rate** | **SD** |
| **0** | 0 | 0 | 0 | 0 | 0 | 0 |
| **4** | 0.00 | 0.000 | 0.00 | 0.000 | 0.00 | 0.000 |
| **8** | 0.07 | 0.069 | 0.02 | 0.040 | 0.12 | 0.133 |
| **24** | 0.24 | 0.070 | 0.10 | 0.024 | 0.29 | 0.148 |
| **32** | 0.44 | 0.068 | 0.14 | 0.026 | 0.41 | 0.131 |
| **48** | 0.55 | 0.074 | 0.18 | 0.032 | 0.41 | 0.066 |
| **72** | 0.76 | 0.107 | 0.22 | 0.038 | 0.43 | 0.024 |
| *: Standard deviation was calculated based on the n-method. | | | | | | |

**Utilization of cytisine**

The utilization of cytisine at 24 h was calculated based on the cumulative permeated amount at 24 h and the initial cytisine content. The results are shown in Table 1.4 and in Figure 1b.

Table 1.4

| Utilization of cytisine after 24 h [%] | | |
|---|---|---|
| **Ex. 1a (n = 3)** | **Ref. Ex. 1b (n = 3)** | **Ref. Ex. 1c (n = 3)** |
| **1.20** | **0.54** | **1.63** |

**REFERENCE EXAMPLES 2A-F**

**Coating composition**

The formulations of the cytisine-containing coating compositions of Reference Examples 2a to 2f are summarized in Tables 2.1 or 2.2 below. The formulations are based on weight percent, as also indicated in Tables 2.1 and 2.2.

Table 2.1

| **Ingredient (Trade Name)** | **Ref Ex. 2a** | | **Ref Ex. 2b** | |
|---|---|---|---|---|
| | **Amt [g]** | **Solids [%]** | **Amt [g]** | **Solids [%]** |
| Cytisine base | 0.45 | 3.0 | 0.45 | 3.0 |
| Acrylic adhesive in ethyl acetate and hexane. Solids content of 40.5 % by weight (Duro-Tak™ 87-2510) | 33.49 | 91.9 | - | - |
| Acrylic adhesive in ethyl acetate. Solids content of 39 % by weight (Duro-Tak™ 87-4287) | - | - | 34.05 | 86.9 |
| Diethylene glycol monoethyl ether (Transcutol®) | - | - | 1.52 | 10.1 |
| Isopropyl palmitate | - | 5.1 | - | - |
| Ethyl acetate | - | - | 4.78 | - |
| Hexane | 0.76 | - | - | - |
| Total | - | 100.0 | 40.80 | 100.0 |
| Area Weight [g/m$^2$] | 116.8 | | 89.9 | |
| Cytisine content [mg/cm$^2$] | 0.351 | | 0.270 | |

Table 2.2

| **Ingredient (Trade Name)** | **Ref. Ex. 2c** | | **Ref. Ex. 2d** | | **Ref. Ex. 2e** | | **Ref. Ex. 2f** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| Cytisine base | 0.45 | 3.0 | 0.45 | 3.0 | 0.45 | 3.0 | 0.91 | 6.0 |
| Acrylic adhesive in ethyl acetate. Solids content of 39 % by weight (Duro-Tak™ 87-4287) | 34.01 | 87.0 | - | - | - | - | - | - |
| Acrylic adhesive in ethyl acetate. Solids content of 36.5 % by weight (Duro-Tak™ 87-9301) | - | - | 33.99 | 86.9 | 33.99 | 86.8 | 30.94 | 79.0 |
| Propylene glycol monocaprylate type II (Capryol® 90) | 1.50 | 10.0 | 1.51 | 10.1 | 0.78 | 5.2 | 1.49 | 10.0 |
| Methyl laurate | - | - | - | - | 0.76 | 5.0 | - | - |
| Polyvinylpyrrolidone (Povidone K90F) | - | - | - | - | - | - | 3.75 | 5.0 |
| Ethyl acetate | 4.76 | - | 4.76 | - | 4.74 | - | 3.67 | - |
| Total | 40.72 | 100.0 | 40.71 | 100.0 | 40.72 | 100.0 | 40.76 | 100.0 |
| Area Weight [g/m$^2$] | 96.2 | | 92.1 | | 94.0 | | 95.0 | |
| Cytisine content [mg/cm$^2$] | 0.291 | | 0.277 | | 0.284 | | 0.574 | |

**Preparation of the coating composition**

For Reference Examples 2a to 2f, a beaker was loaded with Cytisine base, diethylene glycol monoethyl ether, isopropyl palmitate, or propylene glycol monocaprylate type II (and methyl laurate), as applicable. The solvent (ethyl acetate was added and the mixture was stirred at up to 500 rpm. Optionally, the polyvinylpyrrolidone was added and finally the acrylic adhesive. The mixture was then stirred at approx. 250 rpm until a homogeneous mixture was obtained. Coating of the coating composition

**Coating of the coating composition**

The resulting cytisine-containing coating composition was coated on a polyethylene terephthalate film (siliconized, 100 μm thickness, which may function as release liner) and dried for approx. 15 min at room temperature and approx. 15 min at 60 °C. The coating thickness gave an area weight of the matrix layer of 116.8 g/m$^2$ (Reference Example 2a), 89.9 g/m$^2$ (Reference Example 2b), 96.2 g/m$^2$ (Reference Example 2c), 92.1 g/m$^2$ (Reference Example 2d), and 94.0 g/m$^2$ (Reference Example 2e) and 95.0 g/m$^2$ Reference Example 2f), respectively. The dried film was laminated with a polyethylene terephthalate backing layer (23 μm thickness) to provide a cytisine-containing self-adhesive layer structure.

**Preparation of the TTS (concerning all examples)**

The individual systems (TTS) were then punched out from the cytisine-containing self-adhesive layer structure. In specific embodiments a TTS as described above can be provided with a further self-adhesive layer of larger surface area, preferably with rounded corners, comprising a pressure-sensitive adhesive matrix layer which is free of active agent. This is of advantage when the TTS, on the basis of its physical properties alone, does not adhere sufficiently to the skin and/or when the cytisine-containing matrix layer, for the purpose of avoiding waste, has pronounced corners (square or rectangular shapes). The TTS are then punched out and sealed into pouches of the primary packaging material.

**Measurement of skin permeation rate**

The permeated amount and the corresponding skin permeation rates of TTS prepared according to Reference Examples 2a to 2f were determined by *in vitro* experiments in accordance with the OECD Guideline (adopted April 13, 2004) carried out with a 10.0 ml Franz diffusion cell. Split thickness human skin from cosmetic surgeries (female abdomen, date of birth 1982) was used. A dermatome was used to prepare skin to a thickness of 500 μm, with an intact epidermis for all TTS. Diecuts with an area of 1.161 cm$^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 2.3 and 2.4 as well as in Figure 2a.

Table 2.3

| Cumulative permeated amount with SD* [μg/cm$^2$] | | | | | | | | | | | | |
|---|---|---|---|---|---|---|---|---|---|---|---|---|
| **Elapsed time [h]** | **Ref. Ex. 2a (n = 3)** | | **Ref. Ex. 2b (n = 3)** | | **Ref. Ex. 2c (n = 3)** | | **Ref. Ex. 2d (n = 3)** | | **Ref. Ex. 2e (n = 3)** | | **Ref. Ex. 2f (n = 3)** | |
| | **Rate** | **SD** | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 | 0 | 0 |
| **4** | 5.1 | 7.54 | 4.1 | 5.01 | 13.9 | 11.08 | 2.4 | 1.19 | 2.0 | 1.69 | 9.8 | 10.11 |
| **8** | 14.9 | 19.36 | 9.3 | 10.90 | 29.3 | 21.45 | 8.6 | 3.57 | 8.5 | 5.91 | 25.8 | 19.69 |
| **24** | 59.4 | 55.24 | 33.1 | 30.26 | 82.3 | 40.27 | 40.2 | 10.92 | 41.3 | 15.09 | 85.9 | 38.57 |
| **32** | 80.4 | 67.21 | 44.8 | 37.35 | 102.4 | 44.97 | 55.3 | 13.50 | 57.7 | 17.65 | 113.0 | 44.41 |
| **48** | 117.6 | 79.59 | 66.3 | 47.10 | 134.2 | 49.13 | 81.7 | 15.92 | 87.7 | 21.12 | 160.9 | 51.19 |
| **72** | 160.4 | 80.42 | 96.1 | 53.28 | 165.9 | 48.60 | 116.2 | 17.39 | 126.5 | 23.86 | 222.3 | 53.84 |

Table 2.4

| Skin permeation rate with SD* [μg/(cm$^2$ h)] | | | | | | | | | | | | |
|---|---|---|---|---|---|---|---|---|---|---|---|---|
| **Elapsed time [h]** | **Ref. Ex. 2a (n = 3)** | | **Ref. Ex. 2b (n = 3)** | | **Ref. Ex. 2c (n = 3)** | | **Ref. Ex. 2d (n = 3)** | | **Ref. Ex. 2e (n = 3)** | | **Ref. Ex. 2f (n = 3)** | |
| | **Rate** | **SD** | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 | 0 | 0 |
| **4** | 1.28 | 1.885 | 1.02 | 1.252 | 3.48 | 2.771 | 0.60 | 0.298 | 0.49 | 0.423 | 2.45 | 2.526 |
| **8** | 2.46 | 2.956 | 1.31 | 1.473 | 3.85 | 2.594 | 1.55 | 0.595 | 1.63 | 1.058 | 4.01 | 2.405 |

(continued)

| Skin permeation rate with SD* [μg/(cm² h)] | | | | | | | | | | | | |
|---|---|---|---|---|---|---|---|---|---|---|---|---|
| Elapsed time [h] | Ref. Ex. 2a (n = 3) | | Ref. Ex. 2b (n = 3) | | Ref. Ex. 2c (n = 3) | | Ref. Ex. 2d (n = 3) | | Ref. Ex. 2e (n = 3) | | Ref. Ex. 2f (n = 3) | |
| | Rate | SD | Rate | SD | Rate | SD | Rate | SD | Rate | SD | Rate | SD |
| **24** | 2.78 | 2.242 | 1.49 | 1.210 | 3.31 | 1.183 | 1.97 | 0.473 | 2.05 | 0.585 | 3.76 | 1.182 |
| **32** | 2.62 | 1.497 | 1.46 | 0.886 | 2.51 | 0.611 | 1.89 | 0.352 | 2.05 | 0.365 | 3.39 | 0.730 |
| **48** | 2.33 | 0.774 | 1.34 | 0.611 | 1.99 | 0.283 | 1.65 | 0.208 | 1.87 | 0.285 | 2.99 | 0.424 |
| **72** | 1.78 | 0.094 | 1.24 | 0.261 | 1.32 | 0.043 | 1.44 | 0.103 | 1.62 | 0.179 | 2.56 | 0.116 |
| *: Standard deviation was calculated based on the n-method. | | | | | | | | | | | | |

**Utilization of cytisine**

The utilization of cytisine at 24 h was calculated based on the cumulative permeated amount at 24 h and the initial cytisine content. The results are shown in Table 2.5 and in Figure 2b.

Table 2.5

| Utilization of cytisine after 24 h [%] | | | | | |
|---|---|---|---|---|---|
| Ref. Ex. 2a (n = 3) | Ref. Ex. 2b (n = 3) | Ref. Ex. 2c (n = 3) | Ref. Ex. 2d (n = 3) | Ref. Ex. 2e (n = 3) | Ref. Ex. 2f (n = 3) |
| **16.91** | **12.25** | **28.24** | **14.53** | **14.52** | **14.97** |

**REFERENCE EXAMPLES 3A-C**

**Coating composition**

The formulations of the cytisine-containing coating compositions of Reference Examples 3a to 3c are summarized in Table 3.1 below. The formulations are based on weight percent, as also indicated in Table 3.1.

Table 3.1

| Ingredient (Trade Name) | Ref. Ex. 3a | | Ref Ex. 3b | | Ref. Ex. 3c | |
|---|---|---|---|---|---|---|
| | Amt [g] | Solids [%] | Amt [g] | Solids [%] | Amt [g] | Solids [%] |
| Cytisine base | 0.91 | 6.0 | 0.90 | 6.0 | 1.51 | 10.1 |
| Acrylic adhesive in ethyl acetate. Solids content of 36.5 % by weight (Duro-Tak™ 87-9301) | 30.94 | 79.0 | 28.98 | 74.0 | 27.40 | 69.9 |
| Propylene glycol monocaprylate type II (Capryol® 90) | 1.49 | 10.0 | 1.49 | 10.0 | 1.51 | 10.0 |
| Polyvinylpyrrolidone (Povidone K90F) | 3.75 | 5.0 | - | - | - | - |
| Basic aminoalkyl methacrylate copolymer (Eudragit® E100) | - | - | 1.50 | 10.0 | 1.50 | 10.0 |
| Ethyl acetate | 3.67 | - | 7.87 | - | 8.85 | - |
| Total | 40.76 | 100.0 | 40.74 | 100.0 | 40.77 | 100.0 |
| Area Weight [g/m²] | 95.0 | | 95.9 | | 100.9 | |
| Cytisine content [mg/cm²] | 0.574 | | 0.577 | | 1.014 | |

**Preparation of the coating composition**

For Reference Examples 3a to 3c, a beaker was loaded with cytisine base and the propylene glycol monocaprylate type II. and. The solvent (ethyl acetate) was added and the polyvinylpyrrolidone rsp. basic aminoalkyl methacrylate copolymer was added as well. The mixture was then stirred at approx. 500 rpm until a homogeneous mixture was obtained. Finally, the with the acrylic adhesive cytisin was added and the mixture was again stirred at up to 500 rpm until a homogeneous mixture was obtained.

**Coating of the coating composition**

The resulting cytisine-containing coating composition was coated on a polyethylene terephthalate film (siliconized, 100 μm thickness, which may function as release liner) and dried for approx. 15 min at room temperature and approx. 15 min at 60 °C. The coating thickness gave an area weight of the matrix layer of 95.0 g/m$^2$ (Reference Example 3a), 95.9 g/m$^2$ (Reference Example 3b), and 100.9 g/m$^2$ (Reference Example 3c), respectively. The dried film was laminated with a polyethylene terephthalate backing layer (23 μm thickness) to provide a cytisine-containing self-adhesive layer structure.

**Preparation of the TTS (concerning all examples)**

The individual systems (TTS) were then punched out from the cytisine-containing self-adhesive layer structure. In specific embodiments a TTS as described above can be provided with a further self-adhesive layer of larger surface area, preferably with rounded corners, comprising a pressure-sensitive adhesive matrix layer which is free of active agent. This is of advantage when the TTS, on the basis of its physical properties alone, does not adhere sufficiently to the skin and/or when the cytisine-containing matrix layer, for the purpose of avoiding waste, has pronounced corners (square or rectangular shapes). The TTS are then punched out and sealed into pouches of the primary packaging material.

**Measurement of skin permeation rate**

The permeated amount and the corresponding skin permeation rates of TTS prepared according to Reference Examples 3a to 3c were determined by *in vitro* experiments in accordance with the OECD Guideline (adopted April 13, 2004) carried out with a 10.0 ml Franz diffusion cell. Split thickness human skin from cosmetic surgeries (female abdomen, date of birth 1976) was used. A dermatome was used to prepare skin to a thickness of 500 μm, with an intact epidermis for all TTS. Diecuts with an area of 1.161 cm$^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 3.2 and 3.3 as well as in Figure 3a.

Table 3.2

| Cumulative permeated amount with SD* [μg/cm$^2$] | | | | | | |
|---|---|---|---|---|---|---|
| **Elapsed time [h]** | **Ref. Ex. 3a (n = 3)** | | **Ref. Ex. 3b (n = 3)** | | **Ref. Ex. 3c (n = 3)** | |
| | **Rate** | **SD** | **Rate** | **SD** | **Rate** | **SD** |
| **0** | 0 | 0 | 0 | 0 | 0 | 0 |
| **4** | 1.9 | 0.28 | 2.7 | 1.07 | 12.1 | 9.47 |
| **8** | 7.6 | 0.83 | 10.5 | 3.00 | 33.2 | 18.43 |
| **24** | 57.6 | 1.68 | 66.1 | 16.50 | 142.1 | 41.81 |
| **32** | 95.9 | 4.36 | 102.7 | 24.73 | 217.1 | 44.13 |
| **48** | 184.1 | 11.89 | 174.8 | 29.86 | 347.3 | 28.90 |
| **72** | 284.7 | 7.08 | 246.6 | 24.15 | 442.5 | 19.37 |

Table 3.3

| Skin permeation rate with SD* [µg/(cm² h)] | | | | | | |
|---|---|---|---|---|---|---|
| **Elapsed time [h]** | **Ref. Ex. 3a (n = 3)** | | **Ref. Ex. 3b (n = 3)** | | **Ref. Ex. 3c (n = 3)** | |
| | **Rate** | **SD** | **Rate** | **SD** | **Rate** | **SD** |
| **0** | 0 | 0 | 0 | 0 | 0 | 0 |
| **4** | 0.47 | 0.069 | 0.68 | 0.267 | 3.02 | 2.366 |
| **8** | 1.44 | 0.150 | 1.94 | 0.530 | 5.27 | 2.257 |
| **24** | 3.13 | 0.054 | 3.48 | 0.869 | 6.81 | 1.627 |
| **32** | 4.78 | 0.335 | 4.57 | 1.031 | 9.37 | 1.116 |
| **48** | 5.52 | 0.473 | 4.51 | 0.342 | 8.14 | 1.298 |
| **72** | 4.19 | 0.221 | 2.99 | 0.239 | 3.96 | 0.669 |
| *: Standard deviation was calculated based on the n-method. | | | | | | |

**Utilization of cytisine**

The utilization of cytisine at 24 h was calculated based on the cumulative permeated amount at 24 h and the initial cytisine content. The results are shown in Table 3.4 and in Figure 3b.

Table 3.4

| Utilization of cytisine after 24 h [%] | | |
|---|---|---|
| **Ref. Ex. 3a (n = 3)** | **Ref. Ex. 3b (n = 3)** | **Ref. Ex. 3c (n = 3)** |
| **10.04** | **11.46** | **14.01** |

**REFERENCE EXAMPLES 4A-C**

**Coating composition**

The formulations of the cytisine-containing coating compositions of Reference Examples 4a to 4c are summarized in Table 4.1 below. The formulations are based on weight percent, as also indicated in Table 4.1.

Table 4.1

| **Ingredient (Trade Name)** | **Ref. Ex. 4a** | | **Ref. Ex. 4b** | | **Ref. Ex. 4c** | |
|---|---|---|---|---|---|---|
| | **Amt [g]** | **Solids [%]** | **Amt [g]** | **Solids [%]** | **Amt [g]** | **Solids [%]** |
| Cytisine base | 0.45 | 3.0 | 0.45 | 3.0 | 0.45 | 3.0 |
| Acrylic adhesive in ethyl acetate and hexane. Solids content of 40.5 % by weight (Duro-Tak™ 87-2510) | 33.49 | 91.9 | - | - | - | - |
| Silicone adhesive in n-heptane. Solids content of 70 % by weight (Liveo™ BIO-PSA 7-4301) | - | - | 18.46 | 89.0 | - | - |
| Silicone adhesive in ethyl acetate. Solids content of 60 % by weight (Liveo™ BIO-PSA 7-4302) | - | - | - | - | 22.24 | 88.9 |
| Dipropylene glycol | - | - | 1.20 | 7.8 | 1.18 | 7.9 |
| Isopropyl palmitate | 0.76 | 5.1 | - | - | - | - |
| Hydroxypropyl cellulose | - | - | 0.03 | 0.2 | 0.03 | 0.2 |
| Ethyl acetate | - | - | - | - | 0.30 | - |
| n-heptane | - | - | 2.96 | - | - | - |

(continued)

| Ingredient (Trade Name) | Ref. Ex. 4a | | Ref. Ex. 4b | | Ref. Ex. 4c | |
|---|---|---|---|---|---|---|
| | Amt [g] | Solids [%] | Amt [g] | Solids [%] | Amt [g] | Solids [%] |
| Total | 34.70 | 100.0 | 23.07 | 100.0 | 24.21 | 100.0 |
| Area Weight [g/m$^2$] | 116.8 | | 89.5 | | 108.6 | |
| Cytisine content [mg/cm$^2$] | 0.351 | | 0.269 | | 0.328 | |

**Preparation of the coating composition**

For Reference Example 4a, a beaker was loaded with cytisine base and isopropyl palmitate is added. After adding the acrylic adhesive the mixture was stirred at up to 500 rpm until a homogeneous mixture was obtained.

For Reference Examples 4b and 4c, a beaker was loaded with cytisine base, dipropylene glycol and hydroxypropyl cellulose). The solvent (ethyl acetate or n-heptane, as applicable) was added, followed by the addition of the silicone adhesive. The mixture was stirred at approx. 700 rpm until a homogenous mixture was obtained.

**Coating of the coating composition**

The resulting cytisine-containing coating composition according to Reference Example 4a was coated on a polyethylene terephthalate film (siliconized, 100 μm thickness, which may function as release liner) and dried for approx. 15 min at room temperature and approx. 15 min at 60 °C. The resulting cytisine-containing coating composition according to Reference examples 4b and 4c was coated on a polyethylene terephthalate film (one side fluoropolymer coated, 75 μm thickness, which may function as release liner) and dried for approx. 15 min at room temperature and approx. 15 min at 60 °C. The coating thickness gave an area weight of the matrix layer of 116.8 g/m$^2$ (Example 4a), 89.5 g/m$^2$ (Reference Example 4b), and 108.6 g/m$^2$ (Reference Example 4c), respectively. The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

The permeated amount and the corresponding skin permeation rates of TTS prepared according to Reference Examples 4a to 4c were determined by *in vitro* experiments in accordance with the OECD Guideline (adopted April 13, 2004) carried out with a 10.0 ml Franz diffusion cell. Split thickness human skin from cosmetic surgeries (female abdomen, date of birth 1987) was used. A dermatome was used to prepare skin to a thickness of 500 μm, with an intact epidermis for all TTS. Diecuts with an area of 1.161 cm$^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 4.2 and 4.3 as well as in Figure 4a.

Table 4.2

| Cumulative permeated amount with SD* [μg/cm$^2$] | | | | | | |
|---|---|---|---|---|---|---|
| Elapsed time [h] | Ref. Ex. 4a (n = 3) | | Ref. Ex. 4b (n = 3) | | Ref. Ex. 4c (n = 3) | |
| | Rate | SD | Rate | SD | Rate | SD |
| **0** | 0 | 0 | 0 | 0 | 0 | 0 |
| **4** | 0.0 | 0.00 | 0.4 | 0.47 | 0.0 | 0.00 |
| **8** | 0.1 | 0.15 | 0.5 | 0.59 | 0.0 | 0.00 |
| **24** | 2.0 | 0.67 | 1.7 | 0.86 | 0.3 | 0.04 |
| **32** | 3.5 | 1.05 | 2.2 | 0.98 | 0.3 | 0.04 |

(continued)

| Cumulative permeated amount with SD* [µg/cm²] | | | | | | |
|---|---|---|---|---|---|---|
| **Elapsed time [h]** | **Ref. Ex. 4a (n = 3)** | | **Ref. Ex. 4b (n = 3)** | | **Ref. Ex. 4c (n = 3)** | |
| | **Rate** | **SD** | **Rate** | **SD** | **Rate** | **SD** |
| **48** | 7.0 | 1.90 | 3.5 | 1.15 | 0.7 | 0.12 |
| **72** | 13.1 | 3.45 | 5.5 | 1.33 | 1.4 | 0.28 |

Table 4.3

| Skin permeation rate with SD* [µg/(cm² h)] | | | | | | |
|---|---|---|---|---|---|---|
| **Elapsed time [h]** | **Ref. Ex. 4a (n = 3)** | | **Ref. Ex. 4b (n = 3)** | | **Ref. Ex. 4c (n = 3)** | |
| | **Rate** | **SD** | **Rate** | **SD** | **Rate** | **SD** |
| **0** | 0 | 0 | 0 | 0 | 0 | 0 |
| **4** | 0.00 | 0.000 | 0.10 | 0.117 | 0.00 | 0.000 |
| **8** | 0.02 | 0.037 | 0.04 | 0.034 | 0.00 | 0.000 |
| **24** | 0.12 | 0.033 | 0.07 | 0.019 | 0.02 | 0.002 |
| **32** | 0.18 | 0.047 | 0.07 | 0.016 | 0.00 | 0.000 |
| **48** | 0.22 | 0.054 | 0.08 | 0.010 | 0.03 | 0.005 |
| **72** | 0.25 | 0.064 | 0.09 | 0.009 | 0.03 | 0.007 |
| *: Standard deviation was calculated based on the n-method. | | | | | | |

**Utilization of cytisine**

The utilization of cytisine at 24 h was calculated based on the cumulative permeated amount at 24 h and the initial cytisine content. The results are shown in Table 4.4 and in Figure 4b.

Table 4.4

| Utilization of cytisine after 24 h [%] | | |
|---|---|---|
| **Ref. Ex. 4a (n = 3)** | **Ref. Ex. 4b (n = 3)** | **Ref. Ex. 4c (n = 3)** |
| **0.57** | **0.63** | **0.09** |

**EXAMPLES 5A-C**

**Coating composition**

The formulations of the cytisine-containing coating compositions of Examples 5a to 5c are summarized in Table 5.1 below. The formulations are based on weight percent, as also indicated in Table 5.1.

Table 5.1

| **Ingredient (Trade Name)** | **Ex. 5a** | | **Ex. 5b** | | **Ex. 5c** | |
|---|---|---|---|---|---|---|
| | **Amt [g]** | **Solids [%]** | **Amt [g]** | **Solids [%]** | **Amt [g]** | **Solids [%]** |
| Cytisine base | 1.50 | 10.0 | 1.50 | 10.0 | 1.50 | 10.0 |
| Silicone adhesive in ethyl acetate. Solids content of 60 % by weight (Liveo™ BIO-PSA 7-4302) | 19.98 | 80.0 | 18.76 | 75.0 | 18.74 | 74.9 |
| Diethylene glycol monoethyl ether (Transcutol®) | - | - | - | - | 1.50 | 10.0 |

(continued)

| Ingredient (Trade Name) | Ex. 5a | | Ex. 5b | | Ex. 5c | |
|---|---|---|---|---|---|---|
| | Amt [g] | Solids [%] | Amt [g] | Solids [%] | Amt [g] | Solids [%] |
| Propylene glycol monocaprylate type II (Capryol® 90) | 1.50 | 10.0 | 1.50 | 10.0 | - | - |
| Basic aminoalkyl methacrylate copolymer (Eudragit® E100) | - | - | 0.75 | 5.0 | 0.76 | 5.1 |
| Ethyl acetate | 2.02 | - | 2.35 | - | 2.51 | - |
| Total | 25.00 | 100.0 | 24.86 | 100.0 | 25.01 | 100.0 |
| Area Weight [g/m$^2$] | 103.6 | | 105.0 | | 112.8 | |
| Cytisine content [mg/cm$^2$] | 1.039 | | 1.051 | | 1.130 | |

**Preparation of the coating composition**

For Examples 5a to 5c, a beaker was loaded with cytisine base. Propylene glycol monocaprylate type II or diethylene glycol monoethyl ether, as applicable, are added. The solvent (ethyl acetate) was added, followed by the addition of the silicone adhesive. Optionally, the mixture was stirred at approx. 400 rpm, followed by the addition of the basic aminoalkyl methacrylate. The mixture was then stirred at approx. 500 rpm until a homogenous mixture was obtained.

**Coating of the coating composition**

The resulting cytisine-containing coating composition according to Examples 5a to 5c was coated on a polyethylene terephthalate film (one side fluoropolymer coated, 75 μm thickness, which may function as release liner) and dried for approx. 15 min at room temperature and approx. 15 min at 60 °C. The coating thickness gave an area weight of the matrix layer of 103.6 g/m$^2$ (Example 5a), 105.0 g/m$^2$ (Reference Example 5b), and 112.8 g/m$^2$ (Reference Example 5c), respectively. The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

The permeated amount and the corresponding skin permeation rates of TTS prepared according to Example 5a to 5c were determined by *in vitro* experiments in accordance with the OECD Guideline (adopted April 13, 2004) carried out with a 10.0 ml Franz diffusion cell. Split thickness human skin from cosmetic surgeries (female abdomen, date of birth 1988) was used. A dermatome was used to prepare skin to a thickness of 500 μm, with an intact epidermis for all TTS. Diecuts with an area of 1.161 cm$^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 5.2 and 5.3 as well as in Figure 5a.

Table 5.2

| Cumulative permeated amount with SD* [μg/cm$^2$] | | | | | | |
|---|---|---|---|---|---|---|
| Elapsed time [h] | Ex. 5a (n = 3) | | Ex. 5b (n = 3) | | Ex. 5c (n = 3) | |
| | Rate | SD | Rate | SD | Rate | SD |
| **0** | 0 | 0 | 0 | 0 | 0 | 0 |
| **4** | 57.4 | 6.69 | 23.1 | 6.20 | 21.6 | 28.75 |
| **8** | 149.7 | 8.83 | 91.9 | 10.36 | 43.3 | 55.06 |

(continued)

| Cumulative permeated amount with SD* [μg/cm²] | | | | | | |
|---|---|---|---|---|---|---|
| Elapsed time [h] | Ex. 5a (n = 3) | | Ex. 5b (n = 3) | | Ex. 5c (n = 3) | |
| | Rate | SD | Rate | SD | Rate | SD |
| **24** | 279.8 | 9.37 | 232.5 | 14.42 | 115.0 | 107.79 |
| **32** | 324.1 | 9.20 | 277.8 | 14.72 | 151.3 | 122.82 |
| **48** | 386.3 | 9.26 | 337.5 | 14.33 | 222.6 | 134.33 |
| **72** | 457.1 | 10.18 | 403.1 | 14.25 | 338.0 | 125.19 |

Table 5.3

| Skin permeation rate with SD* [μg/(cm² h)] | | | | | | |
|---|---|---|---|---|---|---|
| Elapsed time [h] | Ex. 5a (n = 3) | | Ex. 5b (n = 3) | | Ex. 5c (n = 3) | |
| | Rate | SD | Rate | SD | Rate | SD |
| **0** | 0 | 0 | 0 | 0 | 0 | 0 |
| **4** | 14.36 | 1.672 | 5.77 | 1.549 | 5.40 | 7.187 |
| **8** | 23.05 | 1.000 | 17.21 | 1.054 | 5.41 | 6.580 |
| **24** | 8.13 | 0.297 | 8.79 | 0.261 | 4.49 | 3.296 |
| **32** | 5.53 | 0.039 | 5.66 | 0.134 | 4.53 | 1.879 |
| **48** | 3.89 | 0.015 | 3.73 | 0.140 | 4.46 | 0.730 |
| **72** | 2.95 | 0.041 | 2.73 | 0.147 | 4.81 | 0.423 |
| *: Standard deviation was calculated based on the n-method. | | | | | | |

**Utilization of cytisine**

The utilization of cytisine at 24 h was calculated based on the cumulative permeated amount at 24 h and the initial cytisine content. The results are shown in Table 5.4 and in Figure 5b.

Table 5.4

| Utilization of cytisine after 24 h [%] | | |
|---|---|---|
| Ex. 5a (n = 3) | Ex. 5b (n = 3) | Ex. 5c (n = 3) |
| **26.94** | **22.13** | **10.18** |

**EXAMPLES 6A-D AND REFERENCE EXAMPLES 6E+F**

**Coating composition**

The formulations of the cytisine-containing coating compositions of Examples 6a to 6d are summarized in Table 6.1 below and the formulations of the cytisine-containing coating compositions of Reference Examples 6e and 6f are summarized in Table 6.2 below. The formulations are based on weight percent, as also indicated in Tables 6.1 and 6.2.

Table 6.1

| Ingredient (Trade Name) | Ex. 6a | | Ex. 6b | | Ex. 6c | | Ex. 6d | |
|---|---|---|---|---|---|---|---|---|
| | Amt [g] | Solids [%] | Amt [g] | Solids [%] | Amt [g] | Solids [%] | Amt [g] | Solids [%] |
| Cytisine base | 1.50 | 10.0 | 1.50 | 10.0 | 1.50 | 10.0 | 1.50 | 10.0 |

(continued)

| Ingredient (Trade Name) | Ex. 6a | | Ex. 6b | | Ex. 6c | | Ex. 6d | |
|---|---|---|---|---|---|---|---|---|
| | Amt [g] | Solids [%] | Amt [g] | Solids [%] | Amt [g] | Solids [%] | Amt [g] | Solids [%] |
| Silicone adhesive in ethyl acetate. Solids content of 60 % by weight (Liveo™ BIO-PSA 7-4302) | 18.74 | 74.9 | 19.37 | 77.5 | 20.00 | 79.9 | 17.50 | 69.9 |
| Propylene glycol monocaprylate, type II (Capryol® 90) | 1.52 | 10.1 | 1.51 | 10.0 | 1.14 | 7.6 | 1.51 | 10.0 |
| Basic aminoalkyl methacrylate copolymer (Eudragit® E100) | 0.75 | 5.0 | 0.38 | 2.5 | 0.38 | 2.5 | 1.51 | 10.1 |
| Ethyl acetate | 2.51 | - | 2.28 | - | 2.00 | - | 3.00 | - |
| Total | 23.03 | 100.0 | 24.04 | 100.0 | 25.02 | 100.0 | 25.02 | 100.0 |
| Area Weight [g/m$^2$] | 102.9 | | 101.0 | | 101.0 | | 112.0 | |
| Cytisine content [mg/cm$^2$] | 1.029 | | 1.011 | | 1.010 | | 1.120 | |

Table 6.2

| Ingredient (Trade Name) | Ref. Ex. 6e | | Ref. Ex. 6f | |
|---|---|---|---|---|
| | Amt [g] | Solids [%] | Amt [g] | Solids [%] |
| Cytisine base | 1.50 | 10.0 | 1.50 | 10.0 |
| Silicone adhesive in ethyl acetate. Solids content of 60 % by weight (Liveo™ BIO-PSA 7-4302) | 20.00 | 80.0 | 19.36 | 77.5 |
| Polysorbate (Tween® 20) | 1.13 | 7.5 | 1.12 | 7.5 |
| Basic aminoalkyl methacrylate copolymer (Eudragit® E100) | 0.38 | 2.5 | 0.75 | 5.0 |
| Ethyl acetate | 2.02 | - | 2.25 | - |
| Total | 25.03 | 100.0 | 24.98 | 100.0 |
| Area Weight [g/m$^2$] | 112.1 | | 106.6 | |
| Cytisine content [mg/cm$^2$] | 1.121 | | 1.069 | |

**Preparation of the coating composition**

For Examples 6a to 6d as well as Reference Examples 6e and 6f, a beaker was loaded with cytisine base. Propylene glycol monocaprylate type II or polysorbate, as applicable, are added. The solvent (ethyl acetate) was added, followed by the addition of the silicone adhesive. The mixture was stirred at approx. 400 rpm, followed by the addition of the basic aminoalkyl methacrylate. The mixture was then stirred at approx. 500 rpm until a homogenous mixture was obtained.

**Coating of the coating composition**

The resulting cytisine-containing coating composition was coated on a polyethylene terephthalate film (one side fluoropolymer coated, 75 μm thickness, which may function as release liner) and dried for approx. 15 min at room temperature and approx. 25 min at 60 °C. The coating thickness gave an area weight of the matrix layer of 102.9 g/m$^2$ (Example 6a), 101.0 g/m$^2$ (Examples 6b and 6c), 112.0 g/m$^2$ (Example 6d), 112.1 g/m$^2$ (Reference Example 6e), and 106.6 g/m$^2$ (Reference Example 6f), respectively. The dried film was laminated with a polyethylene terephthalate backing layer (23 μm thickness) to provide a cytisine-containing self-adhesive layer structure.

**Preparation of the TTS**

See Reference Example 1.

## Measurement of skin permeation rate

The permeated amount and the corresponding skin permeation rates of TTS prepared according to Examples 6a to 6d as well as Reference Examples 6e and 6f were determined by *in vitro* experiments in accordance with the OECD Guideline (adopted April 13, 2004) carried out with a 10.0 ml Franz diffusion cell. Split thickness human skin from cosmetic surgeries was used. A dermatome was used to prepare skin to a thickness of 500 μm, with an intact epidermis for all TTS. Diecuts with an area of 1.161 $cm^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 6.3 and 6.4 as well as in Figure 6a.

Table 6.3

| Cumulative permeated amount with SD* [μg/$cm^2$] | | | | | | | | | | | | |
|---|---|---|---|---|---|---|---|---|---|---|---|---|
| **Elapsed time [h]** | **Ex. 6a (n = 3)** | | **Ex. 6b (n = 3)** | | **Ex. 6c (n = 3)** | | **Ex. 6d (n = 3)** | | **Ref. Ex. 6e (n = 3)** | | **Ref. Ex. 6f (n = 3)** | |
| | **Rate** | **SD** | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 | 0 | 0 |
| **4** | 85.3 | 28.67 | 73.9 | 12.29 | 56.2 | 7.16 | 63.2 | 17.90 | 2.6 | 1.25 | 2.9 | 1.95 |
| **8** | 167.2 | 35.25 | 160.4 | 15.44 | 123.4 | 3.12 | 151.6 | 28.92 | 15.9 | 3.81 | 14.4 | 6.17 |
| **24** | 291.1 | 39.73 | 280.6 | 12.28 | 237.7 | 12.75 | 312.5 | 33.70 | 91.1 | 13.65 | 79.6 | 17.50 |
| **32** | 327.5 | 40.36 | 316.1 | 10.65 | 274.9 | 14.35 | 359.2 | 31.94 | 129.1 | 18.95 | 114.7 | 22.68 |
| **48** | 376.6 | 41.03 | 367.0 | 8.98 | 326.6 | 15.56 | 415.7 | 27.94 | 195.4 | 26.32 | 181.3 | 32.45 |
| **72** | 435.1 | 43.12 | 428.3 | 8.07 | 388.5 | 15.43 | 472.5 | 24.84 | 281.0 | 30.55 | 277.5 | 46.17 |

Table 6.4

| Skin permeation rate with SD* [μg/($cm^2$ h)] | | | | | | | | | | | | |
|---|---|---|---|---|---|---|---|---|---|---|---|---|
| **Elapsed time [h]** | **Ex. 6a (n = 3)** | | **Ex. 6b (n = 3)** | | **Ex. 6c (n = 3)** | | **Ex. 6d (n = 3)** | | **Ref. Ex. 6e (n = 3)** | | **Ref. Ex. 6f (n = 3)** | |
| | **Rate** | **SD** | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 | 0 | 0 |
| **4** | 21.33 | 7.168 | 18.46 | 3.073 | 14.04 | 1.790 | 15.79 | 4.474 | 0.65 | 0.313 | 0.74 | 0.487 |
| **8** | 20.48 | 2.991 | 21.65 | 0.789 | 16.82 | 2.122 | 22.12 | 2.804 | 3.32 | 0.640 | 2.88 | 1.061 |
| **24** | 7.74 | 0.801 | 7.51 | 0.424 | 7.14 | 0.796 | 10.05 | 0.656 | 4.70 | 0.634 | 4.07 | 0.712 |
| **32** | 4.55 | 0.116 | 4.44 | 0.424 | 4.65 | 0.248 | 5.83 | 0.455 | 4.75 | 0.665 | 4.39 | 0.690 |
| **48** | 3.07 | 0.127 | 3.18 | 0.261 | 3.23 | 0.163 | 3.53 | 0.385 | 4.14 | 0.461 | 4.16 | 0.666 |
| **72** | 2.44 | 0.169 | 2.55 | 0.208 | 2.58 | 0.107 | 2.37 | 0.270 | 3.57 | 0.179 | 4.01 | 0.603 |
| *: Standard deviation was calculated based on the n-method. | | | | | | | | | | | | |

## Utilization of cytisine

The utilization of cytisine at 24 h was calculated based on the cumulative permeated amount at 24 h and the initial cytisine content. The results are shown in Table 6.5 and in Figure 6b.

Table 6.5

| Utilization of cytisine after 24 h [%] | | | | | |
|---|---|---|---|---|---|
| **Ex. 6a (n = 3)** | **Ex. 6b (n = 3)** | **Ex. 6c (n = 3)** | **Ex. 6d (n = 3)** | **Ref. Ex. 6e (n = 3)** | **Ref. Ex. 6f (n = 3)** |
| **28.30** | **27.77** | **23.53** | **27.90** | **8.12** | **7.45** |

**EXAMPLES 7A-E AND REFERENCE EXAMPLES 7F-H**

**Coating composition**

The formulations of the cytisine-containing coating compositions of Examples 7a to 7e are summarized in Table 7.1 below and the formulations of the cytisine-containing coating compositions of Reference Examples 7f to 7h are summarized in Table 7.2 below. The formulations are based on weight percent, as also indicated in Tables 7.1 and 7.2.

Table 7.1

| Ingredient (Trade Name) | Ex. 7a | | Ex. 7b | | Ex. 7c | | Ex. 7d | | Ex. 7e | |
|---|---|---|---|---|---|---|---|---|---|---|
| | Amt [g] | Solids [%] | Amt [g] | Solids [%] | Amt [g] | Solids [%] | Amt [g] | Solids [%] | Amt [g] | Solids [%] |
| Cytisine base | 1.50 | 10.0 | 1.50 | 10.0 | 1.50 | 10.0 | 1.50 | 10.0 | 1.50 | 10.0 |
| Isobutylene adhesive in n-heptane. Solids content of 38 % by weight (Duro-Tak™ 87-6908) | - | - | - | - | - | - | - | - | 26.80 | 70.0 |
| Silicone adhesive in ethyl acetate. Solids content of 60 % by weight (Liveo™ BIO-PSA 7-4202) | - | - | 8.80 | 35.1 | - | - | - | - | - | - |
| Silicone adhesive in n-heptane. Solids content of 70 % by weight (Liveo™ BIO-PSA 7-4301) | - | - | - | - | 14.52 | 69.9 | - | - | - | - |
| Silicone adhesive in ethyl acetate. Solids content of 60 % by weight (Liveo™ BIO-PSA 7-4302) | 17.49 | 69.9 | 8.75 | 34.9 | - | - | 17.50 | 69.9 | - | - |
| Propylene glycol monocaprylate, type II (Capryol® 90) | 1.50 | 10.0 | 1.50 | 10.0 | 1.51 | 10.1 | - | - | 1.50 | 10.0 |
| Dodecan-1-ol | - | - | - | - | - | - | 1.51 | 10.1 | - | - |

(continued)

| Ingredient (Trade Name) | Ex. 7a | | Ex. 7b | | Ex. 7c | | Ex. 7d | | Ex. 7e | |
|---|---|---|---|---|---|---|---|---|---|---|
| | Amt [g] | Solids [%] | Amt [g] | Solids [%] | Amt [g] | Solids [%] | Amt [g] | Solids [%] | Amt [g] | Solids [%] |
| Basic aminoalkyl methacrylate copolymer (Eudragit® E100) | 1.51 | 10.1 | 1.50 | 10.0 | 3.77 | 10.0 | 1.50 | 10.0 | 3.76 | 10.0 |
| Ethyl acetate | 3.03 | - | 3.00 | - | - | - | 3.00 | - | - | - |
| n-heptane | - | - | - | - | 3.76 | - | - | - | 2.18 | - |
| Total | 25.03 | 100.0 | 25.05 | 100.0 | 25.06 | 100.0 | 25.01 | 100.0 | 35.74 | 100.0 |
| Area Weight [g/m²] | 105.8 | | 104.3 | | 87.6 | | 97.8 | | 96.6 | |
| Cytisine content [mg/cm²] | 1.059 | | 1.042 | | 0.877 | | 0.977 | | 0.967 | |

Table 7.2

| Ingredient (Trade Name) | Ref. Ex. 7f | | Ref. Ex. 7g | | Ref. Ex. 7h | |
|---|---|---|---|---|---|---|
| | Amt [g] | Solids [%] | Amt [g] | Solids [%] | Amt [g] | Solids [%] |
| Cytisine base | 1.50 | 10.0 | 1.51 | 10.0 | 1.51 | 10.0 |
| Acrylic adhesive in ethyl acetate and hexane. Solids content of 40.5 % by weight (Duro-Tak™ 87-2510) | 30.65 | 84.9 | - | - | - | - |
| Acrylic adhesive in ethyl acetate. Solids content of 39.10 % by weight (Duro-Tak™ 87-4098[1]) | - | - | 26.36 | 69.9 | - | - |
| Silicone adhesive in ethyl acetate. Solids content of 60 % by weight (Liveo™ BIO-PSA 7-4302) | - | - | - | - | 17.51 | 69.9 |
| Propylene glycol monocaprylate, type II (Capryol® 90) | - | - | 1.50 | 10.0 | - | - |
| Isopropyl palmitate | 0.77 | 5.1 | - | - | - | - |
| Oleyl oleate | - | - | - | - | 1.52 | 10.1 |
| Basic aminoalkyl methacrylate copolymer (Eudragit® E100) | - | - | 1.51 | 10.1 | 1.51 | 10.0 |
| Ethyl acetate | 7.88 | - | 9.87 | - | 3.01 | - |
| Total | 40.80 | 100.0 | 40.75 | 100.0 | 25.06 | 100.0 |
| Area Weight [g/m²] | 101.9 | | 103.9 | | 96.7 | |
| Cytisine content [mg/cm²] | 1.019 | | 1.044 | | 0.969 | |
| [1]acrylic copolymer without functional groups | | | | | | |

**Preparation of the coating composition**

For Examples 7a to 7d as well as Reference Example 7h, a beaker was loaded with cytisine base. Propylene glycol monocaprylate type II, Dodecan-1-ol or oleyl oleate, as applicable are added. The solvent (ethyl acetate rsp. n-heptane) was added, followed by the addition of the silicone adhesive. The mixture was stirred at approx. 400 rpm, followed by the addition of the basic aminoalkyl methacrylate. The mixture was then stirred at approx. 500 rpm until a homogenous mixture was obtained.

For Example 7e, a beaker was loaded with cytisine base and propylene glycol monocaprylate type II. The solvent

(n-heptane) was added, followed by the addition of the isobutylene pressure sensitive adhesive. The mixture was stirred at approx. 800 rpm, followed by the addition of the basic aminoalkyl methacrylate. The mixture was then stirred at approx. 500 rpm until a homogenous mixture was obtained.

For Reference Examples 7f and 7g, a beaker was loaded with cytisine base, propylene glycol monocaprylate type II or isopropyl palmitate, as applicable, and with the acrylic adhesive. The solvent (ethyl acetate) was added and the mixture was stirred at up to 500 rpm. Optionally, the basic aminoalkyl methacrylate was added and the mixture was then stirred at approx. 500 rpm until a homogeneous mixture was obtained.

**Coating of the coating composition**

The resulting cytisine-containing coating composition according to Examples 7a to 7d as well as Reference Example 7h was coated on a polyethylene terephthalate film (one side fluoropolymer coated, 75 μm thickness, which may function as release liner) and dried for approx. 15 min at room temperature and approx. 15 min at 60 °C. The resulting cytisine-containing coating composition according to Example 7e was coated on a polyethylene terephthalate film (one side fluoropolymer coated, 75 μm thickness, which may function as release liner) and dried for approx. 15 min at room temperature and approx. 15 min at 60 °C. The resulting cytisine-containing coating composition according to Reference Examples 7f and 7g was coated on a polyethylene terephthalate film (siliconized, 100 μm thickness, which may function as release liner) and dried for approx. 15 min at room temperature and approx. 15 min at 60 °C. The coating thickness gave an area weight of the matrix layer of 105.8 g/$m^2$ (Example 7a), 104.3 g/$m^2$ (Example 7b), 87.6 g/$m^2$ (Example 7c), 97.8 g/$m^2$ (Example 7d), 96.6 g/$m^2$ (Example 7e), 101.9 g/$m^2$ (Reference Example 7f), 103.9 g/$m^2$ (Reference Example 7g), and 96.7 g/$m^2$ (Reference Example 7h), respectively. The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

The permeated amount and the corresponding skin permeation rates of TTS prepared according to Examples 7a to 7e as well as Reference Examples 7f to 7h were determined by *in vitro* experiments in accordance with the OECD Guideline (adopted April 13, 2004) carried out with a 10.0 ml Franz diffusion cell. Split thickness human skin from cosmetic surgeries (male abdomen, date of birth 1991) was used. A dermatome was used to prepare skin to a thickness of 500 μm, with an intact epidermis for all TTS. Diecuts with an area of 1.161 $cm^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 7.3 to 7.6 as well as in Figure 7a.

Table 7.3

| | Cumulative permeated amount with SD* [μg/$cm^2$] | | | | | | | | | |
|---|---|---|---|---|---|---|---|---|---|---|
| Elapsed time [h] | Ex. 7a (n = 3) | | Ex. 7b (n = 3) | | Ex. 7c (n = 3) | | Ex. 7d (n = 3) | | Ex. 7e (n = 3) | |
| | Rate | SD | Rate | SD | Rate | SD | Rate | SD | Rate | SD |
| **0** | 0 | 0 | 0 | 0 | 0 | 0 | 0 | 0 | 0 | 0 |
| **4** | 60.9 | 13.34 | 56.9 | 26.79 | 67.2 | 7.63 | 74.2 | 17.60 | 101.0 | 48.01 |
| **8** | 164.9 | 21.01 | 184.3 | 63.92 | 177.9 | 13.40 | 193.7 | 22.18 | 243.4 | 82.99 |
| **24** | 360.6 | 28.26 | 415.0 | 79.44 | 414.8 | 19.59 | 461.0 | 8.39 | 525.8 | 79.53 |
| **32** | 414.2 | 33.32 | 478.0 | 74.98 | 487.7 | 17.19 | 537.6 | 7.22 | 601.5 | 68.51 |
| **48** | 475.4 | 42.30 | 552.3 | 62.16 | 577.1 | 9.17 | 633.2 | 7.86 | 681.8 | 56.06 |
| **72** | 533.0 | 49.60 | 619.1 | 47.43 | 655.2 | 11.08 | 721.8 | 8.71 | 739.9 | 57.37 |

Table 7.4

| Cumulative permeated amount with SD* [μg/cm²] | | | | | | |
|---|---|---|---|---|---|---|
| **Elapsed time [h]** | **Ref. Ex. 7f (n = 3)** | | **Ref. Ex. 7g (n = 3)** | | **Ref. Ex. 7h (n = 3)** | |
| | **Rate** | **SD** | **Rate** | **SD** | **Rate** | **SD** |
| **0** | 0 | 0 | 0 | 0 | 0 | 0 |
| **4** | 2.8 | 1.60 | 8.7 | 5.20 | 2.5 | 0.79 |
| **8** | 13.5 | 6.05 | 31.9 | 12.76 | 9.2 | 4.10 |
| **24** | 135.1 | 29.06 | 177.0 | 37.43 | 59.6 | 23.78 |
| **32** | 225.1 | 34.64 | 246.7 | 42.91 | 93.2 | 30.77 |
| **48** | 404.9 | 30.88 | 344.4 | 39.59 | 159.6 | 39.27 |
| **72** | 606.8 | 25.65 | 433.3 | 28.77 | 243.9 | 41.48 |

Table 7.5

| Skin permeation rate with SD* [μg/(cm² h)] | | | | | | | | | | |
|---|---|---|---|---|---|---|---|---|---|---|
| **Elapsed time [h]** | **Ex. 7a (n = 3)** | | **Ex. 7b (n = 3)** | | **Ex. 7c (n = 3)** | | **Ex. 7d (n = 3)** | | **Ex. 7e (n = 3)** | |
| | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 |
| **4** | 15.22 | 3.336 | 14.23 | 6.698 | 16.81 | 1.907 | 18.56 | 4.399 | 25.25 | 12.002 |
| **8** | 26.01 | 1.947 | 31.85 | 9.814 | 27.66 | 2.041 | 29.86 | 1.216 | 35.59 | 8.746 |
| **24** | 12.23 | 0.602 | 14.42 | 1.150 | 14.81 | 0.784 | 16.71 | 1.001 | 17.65 | 0.883 |
| **32** | 6.71 | 0.000 | 7.87 | 0.566 | 9.12 | 0.306 | 9.58 | 0.360 | 9.47 | 2.163 |
| **48** | 3.83 | 0.574 | 4.65 | 0.860 | 5.59 | 0.600 | 5.98 | 0.122 | 5.02 | 1.806 |
| **72** | 2.40 | 0.308 | 2.78 | 0.688 | 3.25 | 0.576 | 3.69 | 0.082 | 2.42 | 1.160 |

Table 7.6

| Skin permeation rate with SD* [μg/(cm² h)] | | | | | | |
|---|---|---|---|---|---|---|
| **Elapsed time [h]** | **Ref. Ex. 7f (n = 3)** | | **Ref. Ex. 7g (n = 3)** | | **Ref. Ex. 7h (n = 3)** | |
| | **Rate** | **SD** | **Rate** | **SD** | **Rate** | **SD** |
| **0** | 0 | 0 | 0 | 0 | 0 | 0 |
| **4** | 0.70 | 0.399 | 2.17 | 1.301 | 0.62 | 0.197 |
| **8** | 2.68 | 1.231 | 5.79 | 1.951 | 1.68 | 0.830 |
| **24** | 7.60 | 1.487 | 9.07 | 1.872 | 3.15 | 1.230 |
| **32** | 11.26 | 0.699 | 8.71 | 1.025 | 4.20 | 0.952 |
| **48** | 11.24 | 0.329 | 6.11 | 0.396 | 4.15 | 0.663 |
| **72** | 8.41 | 0.445 | 3.71 | 0.480 | 3.51 | 0.161 |
| *: Standard deviation was calculated based on the n-method. | | | | | | |

**Utilization of cytisine**

The utilization of cytisine at 24 h was calculated based on the cumulative permeated amount at 24 h and the initial cytisine content. The results are shown in Table 7.7 and in Figure 7b.

Table 7.7

| Utilization of cytisine after 24 h [%] | | | | | | | |
|---|---|---|---|---|---|---|---|
| Ex. 7a (n = 3) | Ex. 7b (n = 3) | Ex. 7c (n = 3) | Ex. 7d (n = 3) | Ex. 7e (n = 3) | Ref. Ex. 7f (n = 3) | Ref. Ex. 7g (n = 3) | Ref. Ex. 7e (n = 3) |
| **34.05** | **39.82** | **47.31** | **47.16** | **54.38** | **13.26** | **16.95** | **6.15** |

**EXAMPLES 8A-G**

**Coating composition**

The formulations of the cytisine-containing coating compositions of Examples 8a to 8g are summarized in Table 8.1 or 8.2 below. The formulations are based on weight percent, as also indicated in Tables 8.1 and 8.2.

Table 8.1

| Ingredient (Trade Name) | Ex. 8a | | Ex. 8b | | Ex. 8c | |
|---|---|---|---|---|---|---|
| | Amt [g] | Solids [%] | Amt [g] | Solids [%] | Amt [g] | Solids [%] |
| Cytisine base | 1.50 | 10.0 | 1.12 | 7.5 | 1.50 | 10.0 |
| Isobutylene adhesive in n-heptane. Solids content of 38 % by weight (Duro-Tak™ 87-6908) | - | - | - | - | 26.80 | 70.0 |
| Silicone adhesive in ethyl acetate. Solids content of 60 % by weight (Liveo™ BIO-PSA 7-4302) | 17.49 | 69.9 | 18.12 | 72.5 | - | - |
| Propylene glycol monocaprylate type II (Capryol® 90) | 1.50 | 10.0 | 1.50 | 10.0 | - | - |
| Dodecan-1-ol | - | - | - | - | 1.50 | 10.0 |
| Basic aminoalkyl methacrylate copolymer (Eudragit® E100) | 1.51 | 10.1 | 1.51 | 10.0 | - | - |
| Crospovidone | - | - | - | - | 1.50 | 10.0 |
| Ethyl acetate | 3.03 | - | 2.75 | - | - | - |
| n-heptane | - | - | - | - | 4.43 | - |
| Total | 25.03 | 100.0 | 25.00 | 100.0 | 35.73 | 100.0 |
| Area Weight [g/m$^2$] | 105.8 | | 106.0 | | 94.7 | |
| Cytisine content [mg/cm$^2$] | 1.059 | | 0.795 | | 0.947 | |

Table 8.2

| Ingredient (Trade Name) | Ex. 8d | | Ex. 8e | | Ex. 8f | | Ex. 8g | |
|---|---|---|---|---|---|---|---|---|
| | Amt [g] | Solids [%] | Amt [g] | Solids [%] | Amt [g] | Solids [%] | Amt [g] | Solids [%] |
| Cytisine base | 1.50 | 10.0 | 1.50 | 10.0 | 1.50 | 10.0 | 1.50 | 10.0 |
| Silicone adhesive in n-heptane. Solids content of 70 % by weight (Liveo™ BIO-PSA 7-4201) | 8.22 | 40.0 | - | - | 8.21 | 40.0 | - | - |
| Silicone adhesive in ethyl acetate. Solids content of 60 % by weight (Liveo™ BIO-PSA 7-4202) | - | - | 10.00 | 40.0 | - | - | 10.00 | 40.0 |

(continued)

| Ingredient (Trade Name) | Ex. 8d | | Ex. 8e | | Ex. 8f | | Ex. 8g | |
|---|---|---|---|---|---|---|---|---|
| | Amt [g] | Solids [%] | Amt [g] | Solids [%] | Amt [g] | Solids [%] | Amt [g] | Solids [%] |
| Silicone adhesive in n-heptane. Solids content of 70 % by weight (Liveo™ BIO-PSA 7-4301) | 8.30 | 40.0 | - | - | 8.29 | 40.0 | - | - |
| Silicone adhesive in ethyl acetate. Solids content of 60 % by weight (Liveo™ BIO-PSA 7-4302) | - | - | 10.01 | 40.0 | - | - | 10.01 | 40.0 |
| Propylene glycol monocaprylate type II (Capryol® 90) | 0.76 | 5.0 | 0.75 | 5.0 | - | - | - | - |
| Dodecan-1-ol | - | - | - | - | 0.76 | 5.0 | 0.75 | 5.0 |
| Polyvinylpyrrolidone (Povidone K90F) | 3.74 | 5.0 | 3.74 | 5.0 | 3.75 | 5.0 | 3.75 | 5.0 |
| n-heptane | 2.50 | - | - | - | 2.50 | - | - | - |
| Total | 25.02 | 100.0 | 26.00 | 100.0 | 25.00 | 100.0 | 26.01 | 100.0 |
| Area Weight [g/m$^2$] | 95.1 | | 103.8 | | 94.5 | | 96.5 | |
| Cytisine content [mg/cm$^2$] | 0.949 | | 1.040 | | 0.945 | | 0.964 | |

**Preparation of the coating composition**

For Examples 8a, 8b and 8d to 8g, a beaker was loaded with cytisine base. Propylene glycol monocaprylate type II or dodecan-1-ol, as applicable was added. The solvent (ethyl acetate rsp. n-heptane, as applicable) was added, followed by the addition of the silicone adhesive. The mixture was stirred at approx. 300 rpm, followed by the addition of the polyvinylpyrrolidone. The mixture was then stirred at approx. 500 rpm until a homogenous mixture was obtained.

For Example 8c, a beaker was loaded with cytisine base and dodecan-1-ol. The solvent (n-heptane) was added, followed by the addition of the isobutylene pressure sensitive adhesive. The mixture was stirred at approx. 800 rpm, followed by the addition of the crospovidone. The mixture was then stirred at approx. 500 rpm until a homogenous mixture was obtained.

**Coating of the coating composition**

The resulting cytisine-containing coating composition according to Examples 8a, 8b and 8d to 8g was coated on a polyethylene terephthalate film (one side fluoropolymer coated, 75 μm thickness, which may function as release liner) and dried for approx. 15 min at room temperature and approx. 15 min at 60 °C. The resulting cytisine-containing coating composition according to Example 8c was coated on a polyethylene terephthalate film (one side fluoropolymer coated, 75 μm thickness, which may function as release liner) and dried for approx. 15 min at room temperature and approx. 15 min at 60 °C. The coating thickness gave an area weight of the matrix layer of 104.2 g/m$^2$ (Example 8a), 106.0 g/m$^2$ (Example 8b), 94.7 g/m$^2$ (Example 8c), 95.1 g/m$^2$ (Example 8d), 103.8 g/m$^2$ (Example 8e), 94.5 g/m$^2$ (Example 7f), and 96.5 g/m$^2$ (Example 8g), respectively. The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

The permeated amount and the corresponding skin permeation rates of TTS prepared according to Examples 8a to 8g were determined by *in vitro* experiments in accordance with the OECD Guideline (adopted April 13, 2004) carried out with a 10.0 ml Franz diffusion cell. Split thickness human skin from cosmetic surgeries (female abdomen, date of birth 1961) was used. A dermatome was used to prepare skin to a thickness of 500 μm, with an intact epidermis for all TTS.

Diecuts with an area of 1.161 $cm^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 8.3 to 8.6 as well as in Figure 8a.

Table 8.3

| Cumulative permeated amount with SD* [μg/$cm^2$] | | | | | | |
|---|---|---|---|---|---|---|
| Elapsed time [h] | Ex. 8a (n = 3) | | Ex. 8b (n = 3) | | Ex. 8c (n = 3) | |
| | Rate | SD | Rate | SD | Rate | SD |
| **0** | 0 | 0 | 0 | 0 | 0 | 0 |
| **4** | 26.3 | 7.22 | 48.2 | 18.50 | 163.3 | 43.24 |
| **8** | 76.5 | 14.12 | 100.5 | 24.12 | 347.0 | 39.83 |
| **24** | 212.2 | 28.64 | 204.6 | 21.35 | 639.4 | 28.99 |
| **32** | 264.9 | 31.07 | 237.1 | 18.59 | 707.1 | 32.15 |
| **48** | 338.8 | 29.75 | 278.5 | 14.77 | 778.6 | 30.98 |
| **72** | 414.8 | 27.11 | 324.6 | 12.32 | 834.3 | 25.95 |

Table 8.4

| Cumulative permeated amount with SD* [μg/$cm^2$] | | | | | | | | |
|---|---|---|---|---|---|---|---|---|
| Elapsed time [h] | Ex. 8d (n = 3) | | Ex. 8e (n = 3) | | Ex. 8f (n = 3) | | Ex. 8g(n = 3) | |
| | Rate | SD | Rate | SD | Rate | SD | Rate | SD |
| **0** | 0 | 0 | 0 | 0 | 0 | 0 | 0 | 0 |
| **4** | 74.0 | 20.60 | 50.3 | 11.22 | 86.7 | 11.87 | 125.1 | 4.96 |
| **8** | 153.2 | 26.03 | 102.5 | 15.31 | 212.2 | 21.21 | 236.5 | 5.60 |
| **24** | 348.3 | 31.50 | 231.4 | 14.34 | 424.6 | 34.05 | 413.1 | 7.66 |
| **32** | 412.1 | 30.15 | 281.1 | 11.96 | 489.0 | 37.22 | 466.1 | 8.80 |
| **48** | 498.9 | 28.93 | 353.0 | 8.38 | 575.9 | 39.79 | 535.1 | 10.52 |
| **72** | 599.0 | 26.81 | 432.5 | 9.02 | 664.8 | 44.18 | 603.1 | 10.35 |

Table 8.5

| Skin permeation rate with SD* [μg/($cm^2$ h)] | | | | | | |
|---|---|---|---|---|---|---|
| Elapsed time [h] | Ex. 8a (n = 3) | | Ex. 8b (n = 3) | | Ex. 8c (n = 3) | |
| | Rate | SD | Rate | SD | Rate | SD |
| **0** | 0 | 0 | 0 | 0 | 0 | 0 |
| **4** | 6.57 | 1.805 | 12.06 | 4.624 | 40.82 | 10.809 |
| **8** | 12.56 | 1.747 | 13.08 | 1.461 | 45.93 | 1.929 |
| **24** | 8.48 | 0.921 | 6.50 | 0.194 | 18.28 | 2.477 |
| **32** | 6.59 | 0.399 | 4.06 | 0.346 | 8.47 | 0.541 |
| **48** | 4.62 | 0.214 | 2.59 | 0.244 | 4.46 | 0.094 |
| **72** | 3.17 | 0.211 | 1.92 | 0.116 | 2.32 | 0.210 |

Table 8.6

| Skin permeation rate with SD* [μg/(cm$^2$ h)] | | | | | | | | |
|---|---|---|---|---|---|---|---|---|
| Elapsed time [h] | Ex. 8d (n = 3) | | Ex. 8e (n = 3) | | Ex. 8f (n = 3) | | Ex. 8g (n = 3) | |
| | Rate | SD | Rate | SD | Rate | SD | Rate | SD |
| **0** | 0 | 0 | 0 | 0 | 0 | 0 | 0 | 0 |
| **4** | 18.49 | 5.150 | 12.58 | 2.805 | 21.68 | 2.967 | 31.27 | 1.241 |
| **8** | 19.82 | 1.423 | 13.04 | 1.120 | 31.36 | 2.366 | 27.84 | 0.347 |
| **24** | 12.19 | 0.419 | 8.06 | 0.092 | 13.28 | 0.823 | 11.04 | 0.139 |
| **32** | 7.98 | 0.215 | 6.22 | 0.302 | 8.05 | 0.419 | 6.62 | 0.144 |
| **48** | 5.42 | 0.087 | 4.49 | 0.265 | 5.43 | 0.269 | 4.32 | 0.114 |
| **72** | 4.17 | 0.092 | 3.31 | 0.155 | 3.70 | 0.247 | 2.83 | 0.020 |
| *: Standard deviation was calculated based on the n-method. | | | | | | | | |

**Utilization of cytisine**

The utilization of cytisine at 24 h was calculated based on the cumulative permeated amount at 24 h and the initial cytisine content. The results are shown in Table 8.5 and in Figure 8b.

Table 8.5

| Utilization of cytisine after 24 h [%] | | | | | | |
|---|---|---|---|---|---|---|
| Ex. 8a (n = 3) | Ex. 8b (n = 3) | Ex. 8c (n = 3) | Ex. 8d (n = 3) | Ex. 8e (n = 3) | Ex. 8f (n = 3) | Ex. 8g (n = 3) |
| **20.04** | **25.73** | **67.55** | **36.70** | **22.24** | **44.94** | **42.84** |

**EXAMPLES 9A-E**

**Coating composition**

The formulations of the cytisine-containing coating compositions of Examples 9a to 9e are summarized in Table 9.1 or 9.2 below. The formulations are based on weight percent, as also indicated in Tables 9.1 and 9.2.

Table 9.1

| Ingredient (Trade Name) | Ex. 9a | | Ex. 9b | | Ex. 9c | |
|---|---|---|---|---|---|---|
| | Amt [g] | Solids [%] | Amt [g] | Solids [%] | Amt [g] | Solids [%] |
| Cytisine base | 1.50 | 10.0 | 1.51 | 10.0 | 1.50 | 10.0 |
| Silicone adhesive in ethyl acetate. Solids content of 60 % by weight (Liveo™ BIO-PSA 7-4302) | 17.51 | 70.0 | 17.50 | 69.9 | 17.50 | 69.8 |
| Propylene glycol monocaprylate type II (Capryol® 90) | 1.50 | 10.0 | 1.50 | 10.0 | 1.51 | 10.1 |
| Basic aminoalkyl methacrylate copolymer (Eudragit® E100) | 1.50 | 10.0 | - | - | - | - |
| Neutral methacrylic acid copolymer (Eudragit® RL100) | - | - | 1.52 | 10.1 | - | - |
| Neutral methacrylic acid copolymer (Eudragit® RS100) | - | - | - | - | 1.51 | 10.1 |
| Ethyl acetate | 3.00 | - | 3.01 | - | 3.03 | - |
| Total | 25.01 | 100.0 | 25.04 | 100.0 | 25.05 | 100.0 |

(continued)

| Ingredient (Trade Name) | Ex. 9a | | Ex. 9b | | Ex. 9c | |
|---|---|---|---|---|---|---|
| | Amt [g] | Solids [%] | Amt [g] | Solids [%] | Amt [g] | Solids [%] |
| Area Weight [g/m$^2$] | 104.2 | | 101.4 | | 101.5 | |
| Cytisine content [mg/cm$^2$] | 1.042 | | 1.016 | | 1.015 | |

Table 9.2

| Ingredient (Trade Name) | Ex. 9d | | Ex. 9e | |
|---|---|---|---|---|
| | Amt [g] | Solids [%] | Amt [g] | Solids [%] |
| Cytisine base | 1.50 | 10.0 | 1.50 | 10.0 |
| Isobutylene adhesive in n-heptane. Solids content of 38% by weight (Duro-Tak™ 87-6908) | 26.77 | 69.9 | 22.94 | 60.0 |
| Propylene glycol monocaprylate type II (Capryol® 90) | 1.50 | 10.1 | 1.50 | 10.0 |
| Crospovidone | 1.50 | 10.0 | 3.00 | 20.0 |
| n-heptane | 4.43 | - | 6.76 | - |
| Total | 35.70 | 100.0 | 35.70 | 100.0 |
| Area Weight [g/m$^2$] | 97.0 | | 99.0 | |
| Cytisine content [mg/cm$^2$] | 0.971 | | 0.990 | |

**Preparation of the coating composition**

For Examples 9a to 9c, a beaker was loaded with cytisine base and propylene glycol monocaprylate type II. The solvent (ethyl acetate) was added, followed by the addition of the silicone adhesive. The mixture was stirred at approx. 300 rpm, followed by the addition of the basic aminoalkyl methacrylate copolymer or neutral methacrylic acid copolymer, as applicable. The mixture was then stirred at approx. 300 rpm until a homogenous mixture was obtained.

For Example 9d and 9e, a beaker was loaded with cytisine base and propylene glycol monocaprylate type II. The solvent (n-heptane) was added, followed by the addition of the isobutylene pressure sensitive adhesive. The mixture was stirred at approx. 800 rpm, followed by the addition of the crospovidone. The mixture was then stirred at approx. 500 rpm until a homogenous mixture was obtained.

**Coating of the coating composition**

The resulting cytisine-containing coating composition according to Examples 9a to 9c was coated on a polyethylene terephthalate film (one side fluoropolymer coated, 75 μm thickness, which may function as release liner) and dried for approx. 15 min at room temperature and approx. 25 min at 60 °C. The resulting cytisine-containing coating composition according to Examples 9d and 9e was coated on a polyethylene terephthalate film (one side fluoropolymer coated, 75 μm thickness, which may function as release liner) and dried for approx. 15 min at room temperature and approx. 15 min at 60 °C. The coating thickness gave an area weight of the matrix layer of 104.2 g/m$^2$ (Example 9a), 101.4 g/m$^2$ (Example 9b), 101.5 g/m$^2$ (Example 9c), 97.0 g/m$^2$ (Example 9d), and 99.0 g/m$^2$ (Example 9e), respectively. The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

The permeated amount and the corresponding skin permeation rates of TTS prepared according to Examples 9a

to 9e were determined by *in vitro* experiments in accordance with the OECD Guideline (adopted April 13, 2004) carried out with a 10.0 ml Franz diffusion cell. Split thickness human skin from cosmetic surgeries (female abdomen, date of birth 1988) was used. A dermatome was used to prepare skin to a thickness of 500 μm, with an intact epidermis for all TTS. Diecuts with an area of 1.161 $cm^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 9.2 and 9.3 as well as in Figure 9a.

Table 9.2

| Cumulative permeated amount with SD* [μg/$cm^2$] | | | | | | | | | | |
|---|---|---|---|---|---|---|---|---|---|---|
| Elapsed time [h] | Ex. 9a (n = 3) | | Ex. 9b (n = 3) | | Ex. 9c (n = 3) | | Ex. 9d (n = 3) | | Ex. 9e (n = 3) | |
| | Rate | SD | Rate | SD | Rate | SD | Rate | SD | Rate | SD |
| **0** | 0 | 0 | 0 | 0 | 0 | 0 | 0 | 0 | 0 | 0 |
| **4** | 125.1 | 71.93 | 95.9 | 10.50 | 85.1 | 9.23 | 372.3 | 138.21 | 480.9 | 47.21 |
| **8** | 225.9 | 62.32 | 193.7 | 21.80 | 170.6 | 6.61 | 547.1 | 145.44 | 700.4 | 27.35 |
| **24** | 368.0 | 36.46 | 336.4 | 41.19 | 289.8 | 3.18 | 732.9 | 81.09 | 906.6 | 7.70 |
| **32** | 405.4 | 32.87 | 381.4 | 44.44 | 320.9 | 7.25 | 767.8 | 62.31 | 937.3 | 7.06 |
| **48** | 454.5 | 29.54 | 438.4 | 50.19 | 362.9 | 9.36 | 796.1 | 45.49 | 953.2 | 9.09 |
| **72** | 506.2 | 29.67 | 498.5 | 51.90 | 406.0 | 10.21 | 817.3 | 37.34 | 958.9 | 10.09 |

Table 9.3

| Skin permeation rate with SD* [μg/($cm^2$ h)] | | | | | | | | | | |
|---|---|---|---|---|---|---|---|---|---|---|
| Elapsed time [h] | Ex. 9a (n = 3) | | Ex. 9b (n = 3) | | Ex. 9c (n = 3) | | Ex. 9d (n = 3) | | Ex. 9e (n = 3) | |
| | Rate | SD | Rate | SD | Rate | SD | Rate | SD | Rate | SD |
| **0** | 0 | 0 | 0 | 0 | 0 | 0 | 0 | 0 | 0 | 0 |
| **4** | 31.26 | 17.982 | 23.98 | 2.625 | 21.27 | 2.308 | 93.07 | 34.551 | 120.21 | 11.803 |
| **8** | 25.21 | 3.373 | 24.44 | 3.173 | 21.39 | 0.672 | 43.69 | 6.933 | 54.89 | 4.985 |
| **24** | 8.88 | 2.301 | 8.92 | 1.238 | 7.45 | 0.347 | 11.61 | 4.035 | 12.88 | 1.637 |
| **32** | 4.67 | 1.166 | 5.63 | 0.410 | 3.90 | 0.510 | 4.36 | 2.350 | 3.84 | 0.387 |
| **48** | 3.07 | 0.539 | 3.56 | 0.376 | 2.62 | 0.143 | 1.77 | 1.073 | 0.99 | 0.193 |
| **72** | 2.15 | 0.104 | 2.50 | 0.072 | 1.79 | 0.036 | 0.88 | 0.345 | 0.24 | 0.058 |
| *: Standard deviation was calculated based on the n-method. | | | | | | | | | | |

**Utilization of cytisine**

The utilization of cytisine at 24 h was calculated based on the cumulative permeated amount at 24 h and the initial cytisine content. The results are shown in Table 9.4 and in Figure 9b.

Table 9.4

| Utilization of cytisine after 24 h [%] | | | | |
|---|---|---|---|---|
| Ex. 9a (n = 3) | Ex. 9b (n = 3) | Ex. 9c (n = 3) | Ex. 9d (n = 3) | Ex. 9e (n = 3) |
| **35.30** | **33.10** | **28.54** | **75.45** | **91.56** |

**EXAMPLES 10A-E**

**Coating composition**

The formulations of the cytisine-containing coating compositions of Examples 10a to 10e are summarized in Table 10.1 or 10.2 below. The formulations are based on weight percent, as also indicated in Tables 10.1 and 10.2.

Table 10.1

| Ingredient (Trade Name) | Ex. 10a | | Ex. 10b | | Ex. 10c | |
|---|---|---|---|---|---|---|
| | Amt [g] | Solids [%] | Amt [g] | Solids [%] | Amt [g] | Solids [%] |
| Cytisine base | 0.74 | 5.0 | 0.75 | 5.0 | 0.75 | 5.0 |
| Isobutylene adhesive in n-heptane, Solids content of 40% by weight (Oppanol® B12 / N100, 80:20) | - | - | - | - | 25.31 | 67.6 |
| Silicone adhesive in ethyl acetate. Solids content of 60 % by weight (Liveo™ BIO-PSA 7-4202) | 8.75 | 35.0 | - | - | - | - |
| Silicone adhesive in ethyl acetate. Solids content of 60 % by weight (Liveo™ BIO-PSA 7-4302) | 8.75 | 35.0 | 17.50 | 69.9 | - | - |
| Dodecan-1-ol | 0.75 | 5.0 | 0.76 | 5.1 | 1.13 | 7.5 |
| Polyvinylpyrrolidone (Povidone K90F) | 2.98 | 20.0 | 3.01 | 20.0 | 2.99 | 19.9 |
| Ethyl alcohol | 11.93 | - | 12.03 | - | | - |
| n-heptane | - | - | - | - | 0.18 | |
| 2-propanol | - | - | - | - | 8.99 | - |
| Total | 33.90 | 100.0 | 34.05 | 100.0 | 39.34 | 100.0 |
| Area Weight [g/m$^2$] | 97.6 | | 95.3 | | 105.8 | |
| Cytisine content [mg/cm$^2$] | 0.484 | | 0.475 | | 0.528 | |

Table 10.2

| Ingredient (Trade Name) | Ex. 10d | | Ex. 10e | |
|---|---|---|---|---|
| | Amt [g] | Solids [%] | Amt [g] | Solids [%] |
| Cytisine base | 0.75 | 5.0 | 0.75 | 5.0 |
| Isobutylene adhesive in n-heptane, Solids content of 40 % by weight (Oppanol® B 12 / N100, 80:20) | 25.07 | 68.1 | 25.32 | 67.4 |
| Propylene glycol monocaprylate type II (Capryol® 90) | 1.13 | 7.3 | - | - |
| Dodecan-1-ol | - | - | 1.14 | 7.6 |
| Crospovidone | 3.00 | 19.6 | 3.01 | 20.0 |
| n-heptane | 9.28 | - | 8.27 | - |
| Total | 39.23 | 100.0 | 38.49 | 100.0 |
| Area Weight [g/m$^2$] | 96.7 | | 96.6 | |
| Cytisine content [mg/cm$^2$] | 0.473 | | 0.484 | |

**Preparation of the coating composition**

For Examples 10a to 10c, a beaker was loaded with the silicone adhesive or isobutylene pressure sensitive adhesive, as applicable. A presolution of cytisine base, dodecan-1-ol, polyvinylpyrrolidone and solvent (ethyl alcohol, 2-propanol, n-heptane, as applicable) was added. The mixture was stirred at approx. 200 rpm until a homogenous mixture was obtained.

For Examples 10d and 10e, a beaker was loaded with cytisine base. Propylene glycol monocaprylate type II or

dodecan-1-ol, as applicable, and the solvent (n-heptane) are added followed by the addition of isobutylene pressure sensitive adhesive and crospovidone. The mixture was then stirred from approx. 600 rpm until a homogenous mixture was obtained.

**Coating of the coating composition**

The resulting cytisine-containing coating composition according to Examples 10a and 10b was coated on a polyethylene terephthalate film (one side fluoropolymer coated, 75 μm thickness, which may function as release liner) and dried for approx. 15 min at room temperature and approx. 15 min at 60 °C. The resulting cytisine-containing coating composition according to Examples 10c to 10e was coated on a polyethylene terephthalate film (siliconized, 100 μm thickness, which may function as release liner) and dried for approx. 15 min at room temperature and approx. 15 min at 60 °C. The coating thickness gave an area weight of the matrix layer of 97.6 g/m$^2$ (Example 10a), 95.3 g/m$^2$ (Example 10b), 105.8 g/m$^2$ (Example 10c), 96.7 g/m$^2$ (Example 10d), and 96.6 g/m$^2$ (Example 10e), respectively. The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

The permeated amount and the corresponding skin permeation rates of TTS prepared according to Examples 10a to 10e were determined by *in vitro* experiments in accordance with the OECD Guideline (adopted April 13, 2004) carried out with a 10.0 ml Franz diffusion cell. Split thickness human skin from cosmetic surgeries (abdomen) was used. A dermatome was used to prepare skin to a thickness of 500 μm, with an intact epidermis for all TTS. Diecuts with an area of 1.171 cm$^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 10.3 and 10.4 as well as in Figure 10a.

Table 10.3

| Cumulative permeated amount with SD* [μg/cm$^2$] | | | | | | | | | | |
|---|---|---|---|---|---|---|---|---|---|---|
| **Elapsed time [h]** | **Ex. 10a (n = 3)** | | **Ex. 10b (n = 3)** | | **Ex. 10c (n = 3)** | | **Ex. 10d (n = 3)** | | **Ex. 10e (n = 3)** | |
| | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 |
| **4** | 29.6 | 15.52 | 62.1 | 43.14 | 71.6 | 28.75 | 11.8 | 8.28 | 15.4 | 4.93 |
| **8** | 112.7 | 29.79 | 174.3 | 81.52 | 161.6 | 46.53 | 73.1 | 24.71 | 120.6 | 11.68 |
| **24** | 316.4 | 19.82 | 343.9 | 56.61 | 370.5 | 32.49 | 328.6 | 10.92 | 405.7 | 4.00 |
| **32** | 357.1 | 13.66 | 370.8 | 41.96 | 410.8 | 23.71 | 375.7 | 6.48 | 428.9 | 2.16 |
| **48** | 389.0 | 7.32 | 389.6 | 26.70 | 439.9 | 14.38 | 405.4 | 5.49 | 435.6 | 1.44 |
| **72** | 406.5 | 2.83 | 399.1 | 17.35 | 453.9 | 9.10 | 417.0 | 5.56 | 437.1 | 1.33 |

Table 10.4

| Skin permeation rate with SD* [μg/(cm$^2$ h)] | | | | | | | | | | |
|---|---|---|---|---|---|---|---|---|---|---|
| **Elapsed time [h]** | **Ex. 10a (n = 3)** | | **Ex. 10b (n = 3)** | | **Ex. 10c (n = 3)** | | **Ex. 10d (n = 3)** | | **Ex. 10e (n = 3)** | |
| | **Rate** | **SD** | **Rate** | **SD** | **Rate** | **SD** | **Rate** | **SD** | **Rate** | **SD** |
| **0** | 0 | 0 | 0 | 0 | 0 | 0 | 0 | 0 | 0 | 0 |
| **4** | 7.40 | 3.880 | 15.53 | 10.784 | 17.90 | 7.188 | 2.94 | 2.070 | 3.84 | 1.234 |
| **8** | 20.77 | 3.761 | 28.05 | 10.168 | 22.50 | 4.478 | 15.35 | 4.155 | 26.30 | 1.745 |

(continued)

| Skin permeation rate with SD* [µg/(cm² h)] | | | | | | | | | | |
|---|---|---|---|---|---|---|---|---|---|---|
| **Elapsed time [h]** | **Ex. 10a (n = 3)** | | **Ex. 10b (n = 3)** | | **Ex. 10c (n = 3)** | | **Ex. 10d (n = 3)** | | **Ex. 10e (n = 3)** | |
| | **Rate** | **SD** | **Rate** | **SD** | **Rate** | **SD** | **Rate** | **SD** | **Rate** | **SD** |
| **24** | 12.73 | 0.655 | 10.60 | 1.745 | 13.06 | 0.962 | 15.97 | 0.871 | 17.82 | 0.595 |
| **32** | 5.08 | 0.775 | 3.36 | 1.888 | 5.04 | 1.111 | 5.89 | 0.633 | 2.90 | 0.244 |
| **48** | 1.99 | 0.402 | 1.18 | 0.986 | 1.82 | 0.593 | 1.85 | 0.211 | 0.41 | 0.049 |
| **72** | 0.73 | 0.20 | 0.40 | 0.427 | 0.58 | 0.224 | 0.48 | 0.04 | 0.07 | 0.008 |
| *: Standard deviation was calculated based on the n-method. | | | | | | | | | | |

**Utilization of cytisine**

The utilization of cytisine at 24 h was calculated based on the cumulative permeated amount at 24 h and the initial cytisine content. The results are shown in Table 10.5 and in Figure 10b.

Table 10.5

| Utilization of cytisine after 24 h [%] | | | | |
|---|---|---|---|---|
| **Ex. 10a (n = 3)** | **Ex. 10b (n = 3)** | **Ex. 10c (n = 3)** | **Ex. 10d (n = 3)** | **Ex. 10e (n = 3)** |
| **65.44** | **72.38** | **70.13** | **69.41** | **83.90** |

**EXAMPLES 11A+B**

**Coating composition**

The formulations of the cytisine-containing coating compositions of Examples 11a and 11b are summarized in Table 11.1 below. The formulations are based on weight percent, as also indicated in Table 11.1.

Table 11.1

| **Ingredient (Trade Name)** | **Ex. 11a** | | **Ex. 11b** | |
|---|---|---|---|---|
| | **Amt [g]** | **Solids [%]** | **Amt [g]** | **Solids [%]** |
| Cytisine base | 0.45 | 3.01 | 0.75 | 5.01 |
| Isobutylene adhesive in n-heptane, Solids content of 40 % by weight (Oppanol® B 12 / N100, 80:20) | 26.02 | 69.39 | 25.32 | 67.39 |
| Dodecan-1-ol | 1.14 | 7.58 | 1.14 | 7.58 |
| Crospovidone | 3.00 | 20.02 | 3.01 | 20.03 |
| n-heptane | 7.84 | - | 8.27 | - |
| Total | 38.45 | 100.00 | 38.49 | 100.00 |
| Area Weight [g/m²] | 96.6 | | 96.6 | |
| Cytisine content [mg/cm²] | 0.291 | | 0.484 | |

**Preparation of the coating composition**

For Examples 11a and 11b, a beaker was loaded with cytisine base and dodecan-1-ol. The solvent (n-heptane) was added, followed by the addition of the isobutylene pressure sensitive adhesive. The mixture was stirred at approx. 800 rpm, followed by the addition of the crospovidone. The mixture was then stirred at approx. 500 rpm until a homogenous mixture was obtained.

**Coating of the coating composition**

The resulting cytisine-containing coating composition according to Examples 11a and 11b was coated on a polyethylene terephthalate film (siliconized, 100 μm thickness, which may function as release liner) and dried for approx. 10 min at room temperature and approx. 15 min at 90 °C. The coating thickness gave an area weight of the matrix layer of 96.6 g/m$^2$ (Examples 11a and 11b). The dried film was laminated with a polyethylene terephthalate backing layer (23 μm thickness) to provide a cytisine-containing self-adhesive layer structure.

**Preparation of the TTS**

See Reference Example 1.

**Measurement of skin permeation rate**

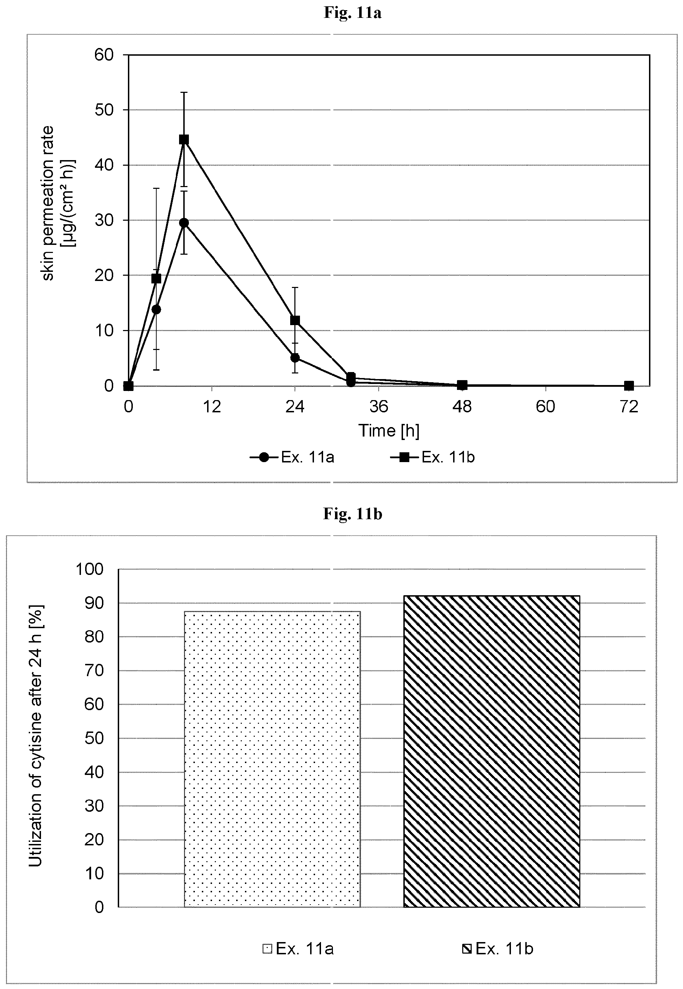

The permeated amount and the corresponding skin permeation rates of TTS prepared according to Examples 11a and 11b were determined by *in vitro* experiments in accordance with the OECD Guideline (adopted April 13, 2004) carried out with a 10.0 ml Franz diffusion cell. Split thickness human skin from cosmetic surgeries (abdomen) was used. A dermatome was used to prepare skin to a thickness of 500 μm, with an intact epidermis for all TTS. Diecuts with an area of 1.15 cm$^2$ were punched from the TTS. The cytisine permeated amount in the receptor medium of the Franz cell (phosphate buffer solution pH 7.4 with 0.1 % saline azide as antibacteriological agent) at a temperature of 32 ± 1°C was measured and the corresponding skin permeation rate calculated. The results are shown in Tables 11.2 and 11.3 as well as in Figure 11a.

Table 11.2

| Cumulative permeated amount with SD* [μg/cm$^2$] | | | | |
|---|---|---|---|---|
| **Elapsed time [h]** | **Ex. 11a (n = 3)** | | **Ex. 11b (n = 3)** | |
| | **Rate** | **SD** | **Rate** | **SD** |
| **0** | 0 | 0 | 0 | 0 |
| **4** | 55.3 | 28.98 | 77.5 | 65.83 |
| **8** | 173.5 | 50.90 | 256.2 | 97.55 |
| **24** | 254.3 | 8.30 | 445.4 | 2.64 |
| **32** | 259.6 | 3.73 | 457.1 | 6.85 |
| **48** | 260.8 | 2.43 | 459.3 | 8.53 |
| **72** | 261.1 | 2.19 | 459.8 | 8.82 |

Table 11.3

| Skin permeation rate with SD* [μg/(cm$^2$ h)] | | | | |
|---|---|---|---|---|
| **Elapsed time [h]** | **Ex. 9a (n = 3)** | | **Ex. 9b (n = 3)** | |
| | **Rate** | **SD** | **Rate** | **SD** |
| **0** | 0 | 0 | 0 | 0 |
| **4** | 13.8 | 7.2 | 19.38 | 16.458 |
| **8** | 29.5 | 5.7 | 44.68 | 8.524 |
| **24** | 5.1 | 2.7 | 11.82 | 5.993 |
| **32** | 0.7 | 0.1 | 1.47 | 0.992 |
| **48** | 0.1 | 0.1 | 0.14 | 0.107 |
| **72** | 0.0 | 0.0 | 0.02 | 0.013 |
| *: Standard deviation was calculated based on the n-method. | | | | |

**Utilization of cytisine**

The utilization of cytisine at 24 h was calculated based on the cumulative permeated amount at 24 h and the initial cytisine content. The results are shown in Table 11.4 and in Figure 11b.

Table 11.4

| **Utilization of cytisine after 24 h [%]** | |
|---|---|
| **Ex. 11a (n = 3)** | **Ex. 11b (n = 3)** |
| **87.45** | **92.10** |

**The** disclosure **relates in particular to the following further items:**

1. Transdermal therapeutic system for the transdermal administration of cytisine comprising a self-adhesive layer structure containing a therapeutically effective amount of cytisine, said self-adhesive layer structure comprising:

   A) a backing layer;
   B) a cytisine-containing layer comprising

      1. cytisine,
      2. a polymer selected from the group consisting of silicone-based polymers and polyisobutylenes, and
      3. a permeation enhancer comprising an organic compound having a linear backbone including 6 to 14 carbon atoms and provided with 1 hydroxy group.

2. Transdermal therapeutic system according to item 1,
wherein the permeation enhancer comprises at least one ester or alcohol having 11 or 12 carbon atoms and provided with 1 hydroxy group.

3. Transdermal therapeutic system according to item 1 or 2,
wherein the permeation enhancer is a mixture consisting of propylene glycol monocaprylate and propylene glycol dicaprylate or is dodecan-1-ol.

4. Transdermal therapeutic system according to item 3,
wherein the permeation enhancer is a mixture consisting of propylene glycol monocaprylate and propylene glycol dicaprylate, and the ratio of propylene glycol monocaprylate to propylene glycol dicaprylate is at least 75:25, preferably at least 85:15, and most preferably at least 90:10.

5. Transdermal therapeutic system according to any one of items 1 to 4,
wherein the permeation enhancer has a molecular weight of not less than 150 g/mol and not more than 210 g/mol, preferably of not less than 175 g/mol and not more than 205 g/mol, and most preferably of not less than 185 g/mol and not more than 202.5 g/mol.

6. Transdermal therapeutic system according to any one of items 1 to 5,
wherein the amount of the permeation enhancer ranges from 1 to 20 %, preferably from 2 to 15 %, more preferably from 4 to 12%, and most preferably from 5 to 11 % based on the total weight of the cytisine-containing layer.

7. Transdermal therapeutic system according to any one of items 1 to 6,
wherein the cytisine-containing layer does not comprise a permeation enhancer selected from the group consisting of propylene glycol, dipropylene glycol, glycerol triacetate, glycerol monocaprylate, diethyl sebacate, propylene glycol monolaurate, isopropyl palmitate and octyl dodecanol.

8. Transdermal therapeutic system according to any one of items 1 to 7,
wherein the cytisine-containing layer further comprises an additional polymer in an amount of from 0.1 to 30 %, preferably 1 to 25 %, more preferably 2 to 22 %, and most preferably from 2 to 12 % or from 18 to 22 % based on the total weight of the cytisine-containing layer.

9. Transdermal therapeutic system according to any one of items 1 to 8,

wherein the cytisine-containing layer further comprises an additional polymer selected from polymers which do not comprise hydroxyl groups, carboxylic acid groups, neutralized carboxylic acid groups and mixtures thereof.

10. Transdermal therapeutic system according to item 8 or 9,
wherein the additional polymer is selected from basic acrylate polymers or polyvinylpyrrolidones.

11. Transdermal therapeutic system according to any one of items 8 to 10,
wherein the additional polymer is an amine-functional acrylate or a polyvinylpyrrolidone.

12. Transdermal therapeutic system according to any one of items 1 to 11,
wherein the cytisine-containing layer comprises cytisine in an amount of from 2 to 25 %, preferably 2.5 to 18 %, more preferably 4 to 12 %, and most preferably of about 5% or about 10 % based on the total weight of the cytisine-containing layer.

13. Transdermal therapeutic system according to any one of items 1 to 12,
wherein the cytisine in the cytisine-containing layer is included in the form of the free base or as a pharmaceutically acceptable salt thereof.

14. Transdermal therapeutic system according to item 13,
wherein at least 90 mol%, preferably at least 95 mol%, more preferably at least 98 mol% and most preferably at least 99 mol% of the cytisine in the cytisine-containing layer is present in the form of the free base.

15. Transdermal therapeutic system according to any one of items 1 to 14,
wherein the transdermal therapeutic system contains at least 0.25 mg/cm$^2$, preferably at least 0.45 mg/cm$^2$, more preferably at least 0.70 mg/cm$^2$ and most preferably at least 0.95 mg/cm$^2$ cytisine.

16. Transdermal therapeutic system according to any one of items 1 to 15,
wherein the transdermal therapeutic system contains less than 2.0 mg/cm$^2$, less than 1.5 mg/cm$^2$, less than 1.2 mg/cm$^2$, or less than 1.0 mg/cm$^2$ cytisine.

17. Transdermal therapeutic system according to any one of items 1 to 16,
wherein the transdermal therapeutic system comprises cytisine in an amount of from 1 to 100 mg, preferably from 10 to 70 mg, more preferably from 15 to 50 mg.

18. Transdermal therapeutic system according to any one of items 1 to 17,
wherein the polymer is a pressure-sensitive adhesive or a mixture of pressure-sensitive adhesives.

19. Transdermal therapeutic system according to item 18,
wherein the pressure sensitive adhesive is obtainable by polycondensation of a silanol endblocked polydimethylsiloxane with a silicate resin, preferably with a resin-to-polymer ratio of from 50:50 to 70:30 and particularly preferably of 55:45, 60:40 or 65:35.

20. Transdermal therapeutic system according to any one of items 1 to 19,
wherein the polymer is at least one silicon-based polymer, and preferably the silicon-based polymer is a mixture of pressure sensitive adhesives obtainable by polycondensation of a silanol endblocked polydimethylsiloxane with a silicate resin with a resin-to-polymer ratio of 55:45 or of 60:40.

21. Transdermal therapeutic system according to any one of items 1 to 20, wherein the polymer is at least one silicone-based polymer, and the silicon-based polymer is preferably a mixture of pressure sensitive adhesives with

a solution viscosity at 25 °C and about 60 % solids content in heptane of 450 mPa s and/or a complex viscosity at 0.01 rad/s at 30 °C of $1\times10^8$ Poise, and
a solution viscosity at 25 °C and about 60 % solids content in heptane of 500 mPa s and/or a complex viscosity at 0.01 rad/s at 30 °C of $5\times10^6$ Poise.

22. Transdermal therapeutic system according to any one of items 1 to 21, wherein the polymer is at least one amine-compatible polysiloxane, and is preferably obtainable by polycondensation of a silanol endblocked polydimethylsiloxane with a silicate resin followed by at least partial trimethylsilylation of the residual silanol functionality.

23. Transdermal therapeutic system according to any one of items 1 to 18,
wherein the polymer is at least one polyisobutylene.

24. Transdermal therapeutic system according to any one of items 1 to 18 and 23,
wherein the polymer is a mixture of low-molecular weight polyisobutylene and high-molecular weight polyisobutylene with a low-molecular weight polyisobutylene to high-molecular weight polyisobutylene ratio in a range of from 100:1 to 1:100, preferably from 95:5 to 40:60, more preferably from 90:10 to 80:20.

25. Transdermal therapeutic system according to any one of items 1 to 24,
wherein the amount of the polymer ranges from 55 to 97 %, preferably from 60 to 75 % or from 68 to 82 % based on the total weight of the cytisine-containing layer.

26. Transdermal therapeutic system according to any one of items 1 to 25,
wherein the cytisine-containing layer does not comprise a hydroxyl group-containing acrylic polymer, carboxylic acid group-containing acrylic polymer, neutralized carboxylic acid group-containing polymer, or mixtures thereof.

27. Transdermal therapeutic system according to any one of items 1 to 26,
wherein the cytisine-containing layer further comprises at least one additive or excipient selected from solubilizers, fillers, gel-forming agents, substances for skincare, pH regulators, preservatives, tackifiers, softeners, and stabilizers, in particular from gel-forming agents, tackifiers, softeners, and stabilizers.

28. Transdermal therapeutic system according to any one of items 1 to 27,
wherein the area weight of the cytisine-containing layer ranges from 70 to 200 g/$m^2$, preferably from 80 to 160 g/$m^2$, and most preferably from 85 to 130 g/$m^2$.

29. Transdermal therapeutic system according to any one of items 1 to 28,
wherein the area of release ranges from 20 to 70 $cm^2$, preferably from 30 to 50 $cm^2$.

30. Transdermal therapeutic system according to any one of items 1 to 29,
wherein the transdermal therapeutic system provides a cumulative permeated amount of cytisine as measured in a Franz diffusion cell with dermatomed human skin of at least 150 $\mu$g/$cm^2$, at least 210 $\mu$g/$cm^2$, at least 250 $\mu$g/$cm^2$, at least 270 $\mu$g/$cm^2$, at least 280 $\mu$g/$cm^2$, or at least 285 $\mu$g/$cm^2$ after a time period of 24 hours.

31. Transdermal therapeutic system according to any one of items 1 to 30,

wherein the transdermal therapeutic system provides a cumulative permeated amount of cytisine as measured in a Franz diffusion cell with dermatomed human skin of 100 $\mu$g/$cm^2$ to 600 $\mu$g/$cm^2$,
preferably 200 $\mu$g/$cm^2$ to 550 $\mu$g/$cm^2$, more preferably 300 $\mu$g/cm2 to 500 $\mu$g/$cm^2$ after a time period of 24 hours.

32. Transdermal therapeutic system according to any one of items 1 to 31,
wherein the transdermal therapeutic system provides by transdermal delivery a mean release rate of 1 to 20 mg/day, preferably 2 to 12 mg/day, more preferably 3 to 10 mg/day over at least 24 hours of administration.

33. Transdermal therapeutic system according to any one of items 1 to 32 for use in a method of treating a human patient,

34. Transdermal therapeutic system for use according to item 33, wherein the method is a method of treating nicotine addiction.

35. Transdermal therapeutic system for use according to item 33 or 34, wherein the method is a method of reducing nicotine cravings.

36. Transdermal therapeutic system for use according to any one of claims 33 to 35, wherein the method is a method of smoking cessation.

37. Transdermal therapeutic system for use according to any one of items 33 to 36, wherein the transdermal therapeutic system is applied to the skin of the patient for a dosing interval of from 20 to 30 hours, preferably of about 24 hours.

38. A process for manufacturing a cytisine-containing layer for use in a transdermal therapeutic system according to any one of items 1 to 32 comprising the steps of:

1) combining at least the components

1. cytisine,
2. a polymer selected from the group consisting of silicone-based polymers and polyisobutylenes, and
3. a permeation enhancer selected from an organic compound having a linear backbone including 6 to 14 carbon atoms and provided with 1 hydroxy group

to obtain a coating composition;
2) coating the coating composition onto the backing layer or a release liner or any intermediate liner; and
3) drying the coated coating composition to form the cytisine-containing layer.

39. Transdermal therapeutic system for the transdermal administration of cytisine comprising a self-adhesive layer structure containing a therapeutically effective amount of cytisine, said self-adhesive layer structure comprising:

A) a backing layer;
B) a cytisine-containing layer comprising

1. cytisine in an amount of from 4 to 12 %,
2. at least one amine-compatible polysiloxane in an amount of from 68 to 82 %,
3. a copolymer based on dimethylaminoethyl methacrylate, butyl methacrylate and methyl methacrylate or a polyvinylpyrrolidone in an amount of from 2 to 22 %, and
4. a permeation enhancer comprising a mixture consisting of propylene glycol monocaprylate and propylene glycol dicaprylate or dodecan-1-ol in an amount of from 5 to 11 %,

based on the total weight of the cytisine-containing layer.

40. Transdermal therapeutic system for the transdermal administration of cytisine comprising a self-adhesive layer structure containing a therapeutically effective amount of cytisine, said self-adhesive layer structure comprising:

A) a backing layer;
B) a cytisine-containing layer comprising

1. cytisine in an amount of from 4 to 12 %,
2. at least one polyisobutylene in an amount of from 60 to 75 %,
3. a polyvinylpyrrolidone in an amount of from 18 to 22 %, and
4. a permeation enhancer comprising a mixture consisting of propylene glycol monocaprylate and propylene glycol dicaprylate or dodecan-1-ol in an amount of from 5 to 11 %,

based on the total weight of the cytisine-containing layer.

## Claims

**1.** Transdermal therapeutic system for the transdermal administration of cytisine comprising a self-adhesive layer structure containing a therapeutically effective amount of cytisine, said self-adhesive layer structure comprising:

A) a backing layer;
B) a cytisine-containing layer comprising

1. cytisine,
2. a polymer selected from the group consisting of silicone-based polymers and polyisobutylenes, and
3. a permeation enhancer comprising an organic compound having a linear backbone including 6 to 14 carbon atoms and provided with 1 hydroxy group.

**2.** Transdermal therapeutic system according to claim 1,

wherein the permeation enhancer comprises at least one ester or alcohol having 11 or 12 carbon atoms and provided with 1 hydroxy group.

**3.** Transdermal therapeutic system according to claim 1 or 2,

wherein the permeation enhancer is a mixture consisting of propylene glycol monocaprylate and propylene glycol dicaprylate or is dodecan-1-ol, preferably a mixture consisting of propylene glycol monocaprylate and propylene glycol dicaprylate, wherein the ratio of propylene glycol monocaprylate to propylene glycol dicaprylate is at least 75:25, preferably at least 85:15, and most preferably at least 90:10, and/or
wherein the permeation enhancer has a molecular weight of not less than 150 g/mol and not more than 210 g/mol, preferably of not less than 175 g/mol and not more than 205 g/mol, and most preferably of not less than 185 g/mol and not more than 202.5 g/mol.

**4.** Transdermal therapeutic system according to any one of claims 1 to 3,
wherein the amount of the permeation enhancer ranges from 1 to 20 %, preferably from 2 to 15 %, more preferably from 4 to 12%, and most preferably from 5 to 11 % based on the total weight of the cytisine-containing layer.

**5.** Transdermal therapeutic system according to any one of claims 1 to 4,

wherein the cytisine-containing layer further comprises an additional polymer selected from polymers which do not comprise hydroxyl groups, carboxylic acid groups, neutralized carboxylic acid groups and mixtures thereof, wherein preferably the additional polymer is selected from basic acrylate polymers or polyvinylpyrrolidones, wherein more preferably the additional polymer is an amine-functional acrylate or a polyvinylpyrrolidone.

**6.** Transdermal therapeutic system according to any one of claims 1 to 5,
wherein the cytisine-containing layer further comprises an additional polymer in an amount of from 0.1 to 30 %, preferably 1 to 25 %, more preferably 2 to 22 %, and most preferably from 2 to 12 % or from 18 to 22 % based on the total weight of the cytisine-containing layer.

**7.** Transdermal therapeutic system according to any one of claims 1 to 6,

wherein the cytisine-containing layer comprises cytisine in an amount of from 2 to 25 %, preferably 2.5 to 18 %, more preferably 4 to 12 %, and most preferably of about 5% or about 10 % based on the total weight of the cytisine-containing layer, and/or
wherein the cytisine in the cytisine-containing layer is included in the form of the free base or as a pharmaceutically acceptable salt thereof, wherein preferably at least 90 mol%, preferably at least 95 mol%, more preferably at least 98 mol% and most preferably at least 99 mol% of the cytisine in the cytisine-containing layer is present in the form of the free base.

**8.** Transdermal therapeutic system according to any one of claims 1 to 7,

wherein the transdermal therapeutic system contains at least 0.25 $mg/cm^2$, preferably at least 0.45 $mg/cm^2$, more preferably at least 0.70 $mg/cm^2$ and most preferably at least 0.95 $mg/cm^2$ cytisine, and/or
wherein the transdermal therapeutic system contains less than 2.0 $mg/cm^2$, less than 1.5 $mg/cm^2$, less than 1.2 $mg/cm^2$, or less than 1.0 $mg/cm^2$ cytisine, and/or
wherein the transdermal therapeutic system comprises cytisine in an amount of from 1 to 100 mg, preferably from 10 to 70 mg, more preferably from 15 to 50 mg.

**9.** Transdermal therapeutic system according to any one of claims 1 to 8,

wherein the polymer is at least one amine-compatible polysiloxane, and is preferably obtainable by polycondensation of a silanol endblocked polydimethylsiloxane with a silicate resin followed by at least partial trimethylsilylation of the residual silanol functionality. or
wherein the polymer is a mixture of low-molecular weight polyisobutylene and high-molecular weight polyisobutylene with a low-molecular weight polyisobutylene to high-molecular weight polyisobutylene ratio in a range of from 100:1 to 1:100, preferably from 95:5 to 40:60, more preferably from 90:10 to 80:20.

**10.** Transdermal therapeutic system according to any one of claims 1 to 9,

wherein the amount of the polymer ranges from 55 to 97 %, preferably from 60 to 75 % or from 68 to 82 % based on the total weight of the cytisine-containing layer.

**11.** Transdermal therapeutic system according to any one of claims 1 to 10, wherein the cytisine-containing layer further comprises at least one additive or excipient selected from solubilizers, fillers, gel-forming agents, substances for skincare, pH regulators, preservatives, tackifiers, softeners, and stabilizers, in particular from gel-forming agents, tackifiers, softeners, and stabilizers.

**12.** Transdermal therapeutic system according to any one of claims 1 to 11,

wherein the area weight of the cytisine-containing layer ranges from 70 to 200 $g/m^2$, preferably from 80 to 160 $g/m^2$, and most preferably from 85 to 130 $g/m^2$ and/or
wherein the area of release ranges from 20 to 70 $cm^2$, preferably from 30 to 50 $cm^2$.

**13.** Transdermal therapeutic system according to any one of claims 1 to 12,

wherein the transdermal therapeutic system provides a cumulative permeated amount of cytisine as measured in a Franz diffusion cell with dermatomed human skin of at least 150 $\mu g/cm^2$, at least 210 $\mu g/cm^2$, at least 250 $\mu g/cm^2$, at least 270 $\mu g/cm^2$, at least 280 $\mu g/cm^2$, or at least 285 $\mu g/cm^2$ after a time period of 24 hours, and/or
wherein the transdermal therapeutic system provides a cumulative permeated amount of cytisine as measured in a Franz diffusion cell with dermatomed human skin of 100 $\mu g/cm^2$ to 600 $\mu g/cm^2$, preferably 200 $\mu g/cm^2$ to 550 $\mu g/cm^2$, more preferably 300 μg/cm2 to 500 $\mu g/cm^2$ after a time period of 24 hours, and/or
wherein the transdermal therapeutic system provides by transdermal delivery a mean release rate of 1 to 20 mg/day, preferably 2 to 12 mg/day, more preferably 3 to 10 mg/day over at least 24 hours of administration.

**14.** Transdermal therapeutic system according to any one of claims 1 to 13 for use in a method of treating a human patient, preferably in a method of treating nicotine addiction, more preferably in a method of reducing nicotine cravings, and most preferably a method of smoking cessation, wherein preferably the transdermal therapeutic system is applied to the skin of the patient for a dosing interval of from 20 to 30 hours, preferably of about 24 hours.

**15.** A process for manufacturing a cytisine-containing layer for use in a transdermal therapeutic system according to any one of claims 1 to 13 comprising the steps of:

1) combining at least the components

1. cytisine,
2. a polymer selected from the group consisting of silicone-based polymers and polyisobutylenes, and
3. a permeation enhancer selected from an organic compound having a linear backbone including 6 to 14 carbon atoms and provided with 1 hydroxy group

to obtain a coating composition;
2) coating the coating composition onto the backing layer or a release liner or any intermediate liner; and
3) drying the coated coating composition to form the cytisine-containing layer.

## Patentansprüche

**1.** Transdermales therapeutisches System für die transdermale Verabreichung von Cytisin, umfassend eine selbstklebende Schichtstruktur enthaltend eine therapeutisch wirksame Menge an Cytisin, wobei besagte selbstklebende Schichtstruktur umfasst:

A) eine Rückschicht;
B) eine Cytisin-enthaltende Schicht umfassend

1. Cytisin,
2. ein Polymer, ausgewählt aus der Gruppe bestehend aus Silikonbasierten Polymeren und Polyisobutylenen, und
3. einen Permeationsverstärker umfassend eine organische Verbindung mit einem linearen Rückgrat mit 6

bis 14 Kohlenstoffatomen und 1 Hydroxygruppe.

**2.** Transdermales therapeutisches System gemäß Anspruch 1,
wobei der Permeationsverstärker mindestens einen Ester oder Alkohol mit 11 oder 12 Kohlenstoffatomen und 1 Hydroxygruppe umfasst.

**3.** Transdermales therapeutisches System gemäß Anspruch 1 oder 2,

wobei der Permeationsverstärker eine Mischung bestehend aus Propylenglykolmonocaprylat und Propylenglykoldicaprylat ist oder Dodecan-1-ol ist, vorzugsweise eine Mischung bestehend aus Propylenglykolmonocaprylat und Propylenglykoldicaprylat, wobei das Verhältnis von Propylenglykolmonocaprylat zu Propylenglykoldicaprylat mindestens 75:25, vorzugsweise mindestens 85:15 und am meisten bevorzugt mindestens 90:10 ist, und/oder
wobei der Permeationsverstärker ein Molekulargewicht von nicht weniger als 150 g/mol und nicht mehr als 210 g/mol, vorzugsweise von nicht weniger als 175 g/mol und nicht mehr als 205 g/mol und am meisten bevorzugt von nicht weniger als 185 g/mol und nicht mehr als 202,5 g/mol aufweist.

**4.** Transdermales therapeutisches System gemäß einem der Ansprüche 1 bis 3, wobei die Menge des Permeationsverstärkers im Bereich von 1 bis 20 %, vorzugsweise von 2 bis 15 %, mehr bevorzugt von 4 bis 12 % und am meisten bevorzugt von 5 bis 11 %, bezogen auf das Gesamtgewicht der Cytisin-enthaltenden Schicht, liegt.

**5.** Transdermales therapeutisches System gemäß einem der Ansprüche 1 bis 4,

wobei die Cytisin-enthaltende Schicht ferner ein zusätzliches Polymer, ausgewählt aus Polymeren, die keine Hydroxylgruppen, Carbonsäure-Gruppen, neutralisierten Carbonsäure-Gruppen und Mischungen davon umfassen, umfasst,
wobei vorzugsweise das zusätzliche Polymer aus basischen Acrylatpolymeren oder Polyvinylpyrrolidonen ausgewählt ist,
wobei mehr bevorzugt das zusätzliche Polymer ein aminofunktionelles Acrylat oder ein Polyvinylpyrrolidon ist.

**6.** Transdermales therapeutisches System gemäß einem der Ansprüche 1 bis 5, wobei die Cytisin-enthaltende Schicht ferner ein zusätzliches Polymer in einer Menge von 0,1 bis 30 %, vorzugsweise 1 bis 25 %, mehr bevorzugt 2 bis 22 % und am meisten bevorzugt von 2 bis 12 % oder von 18 bis 22 %, bezogen auf das Gesamtgewicht der Cytisin-enthaltenden Schicht, umfasst.

**7.** Transdermales therapeutisches System gemäß einem der Ansprüche 1 bis 6,

wobei die Cytisin-enthaltende Schicht Cytisin in einer Menge von 2 bis 25 %, vorzugsweise 2,5 bis 18 %, mehr bevorzugt 4 bis 12 % und am meisten bevorzugt von etwa 5 % oder etwa 10 %, bezogen auf das Gesamtgewicht der Cytisin-enthaltenden Schicht, umfasst, und/oder
wobei das Cytisin in der Cytisin-enthaltenden Schicht in Form der freien Base oder als ein pharmazeutisch verträgliches Salz davon enthalten ist, wobei vorzugsweise mindestens 90 Mol-%, vorzugsweise mindestens 95 Mol-%, mehr bevorzugt mindestens 98 Mol-% und am meisten bevorzugt mindestens 99 Mol-% des Cytisins in der Cytisin-enthaltenden Schicht in Form der freien Base vorliegen.

**8.** Transdermales therapeutisches System gemäß einem der Ansprüche 1 bis 7,

wobei das transdermale therapeutische System mindestens 0,25 mg/cm$^2$, vorzugsweise mindestens 0,45 mg/cm$^2$, mehr bevorzugt mindestens 0,70 mg/cm$^2$ und am meisten bevorzugt mindestens 0,95 mg/cm$^2$ Cytisin enthält, und/oder
wobei das transdermale therapeutische System weniger als 2,0 mg/cm$^2$, weniger als 1,5 mg/cm$^2$, weniger als 1,2 mg/cm$^2$ oder weniger als 1,0 mg/cm$^2$ Cytisin enthält, und/oder
wobei das transdermale therapeutische System Cytisin in einer Menge von 1 bis 100 mg, vorzugsweise von 10 bis 70 mg, mehr bevorzugt von 15 bis 50 mg umfasst.

**9.** Transdermales therapeutisches System gemäß einem der Ansprüche 1 bis 8,

wobei das Polymer mindestens ein Amin-verträgliches Polysiloxan ist und bevorzugt durch Polykondensation

eines Silanol-endblockierten Polydimethylsiloxans mit einem Silikatharz, gefolgt von mindestens teilweiser Trimethylsilylierung der verbleibenden Silanolfunktionalität, erhältlich ist, oder
wobei das Polymer eine Mischung aus niedermolekularem Polyisobutylen und hochmolekularem Polyisobutylen ist mit einem Verhältnis von niedermolekularem Polyisobutylen zu hochmolekularem Polyisobutylen im Bereich von 100:1 bis 1:100, vorzugsweise von 95:5 bis 40:60, mehr bevorzugt von 90:10 bis 80:20.

**10.** Transdermales therapeutisches System gemäß einem der Ansprüche 1 bis 9, wobei die Menge des Polymers im Bereich von 55 bis 97 %, vorzugsweise von 60 bis 75 % oder von 68 bis 82 %, bezogen auf das Gesamtgewicht der Cytisin-enthaltenden Schicht, liegt.

**11.** Transdermales therapeutisches System gemäß einem der Ansprüche 1 bis 10, wobei die Cytisin-enthaltende Schicht ferner mindestens einen Zusatzstoff oder Hilfsstoff, ausgewählt aus Lösungsvermittlern, Füllstoffen, Gelbildnern, Stoffen für die Hautpflege, pH-Regulatoren, Konservierungsstoffen, Klebkraftverstärkern, Weichmachern und Stabilisatoren, insbesondere aus Gelbildnern, Klebkraftverstärkern, Weichmachern und Stabilisatoren, umfasst.

**12.** Transdermales therapeutisches System gemäß einem der Ansprüche 1 bis 11,

wobei das Flächengewicht der Cytisin-enthaltenden Schicht im Bereich von 70 bis 200 g/m$^2$, vorzugsweise von 80 bis 160 g/m$^2$ und am meisten bevorzugt von 85 bis 130 g/m$^2$ liegt und/oder
wobei die Freisetzungsfläche im Bereich von 20 bis 70 cm$^2$, vorzugsweise von 30 bis 50 cm$^2$ liegt.

**13.** Transdermales therapeutisches System gemäß einem der Ansprüche 1 bis 12,

wobei das transdermale therapeutische System eine kumulierte permeierte Menge an Cytisin, gemessen in einer Franz-Diffusionszelle mit dermatomisierter Humanhaut, von mindestens 150 $\mu$g/cm$^2$, mindestens 210 $\mu$g/cm$^2$, mindestens 250 $\mu$g/cm$^2$, mindestens 270 $\mu$g/cm$^2$, mindestens 280 $\mu$g/cm$^2$ oder mindestens 285 $\mu$g/cm$^2$ nach einem Zeitraum von 24 Stunden bereitstellt, und/oder
wobei das transdermale therapeutische System eine kumulierte permeierte Mengen an Cytisin, gemessen in einer Franz-Diffusionszelle mit dermatomisierter Humanhaut von 100 $\mu$g/cm$^2$ bis 600 $\mu$g/cm$^2$, vorzugsweise 200 $\mu$g/cm$^2$ bis 550 $\mu$g/cm$^2$, mehr bevorzugt 300 $\mu$g/cm$^2$ bis 500 $\mu$g/cm$^2$ nach einem Zeitraum von 24 Stunden bereitstellt, und/oder
wobei das transdermale therapeutische System durch transdermale Verabreichung eine mittlere Freisetzungsrate von 1 bis 20 mg/Tag, vorzugsweise 2 bis 12 mg/Tag, mehr bevorzugt 3 bis 10 mg/Tag über mindestens 24 Stunden der Verabreichung bereitstellt.

**14.** Transdermales therapeutisches System gemäß einem der Ansprüche 1 bis 13 zur Verwendung in einem Verfahren zur Behandlung eines menschlichen Patienten, vorzugsweise in einem Verfahren zur Behandlung von Nikotinsucht, mehr bevorzugt in einem Verfahren zur Verringerung des Nikotinverlangens und am meisten bevorzugt in einem Verfahren zur Raucherentwöhnung,
wobei vorzugsweise das transdermale therapeutische System für ein Dosierungsintervall von 20 bis 30 Stunden, vorzugsweise von etwa 24 Stunden, auf die Haut des Patienten aufgebracht wird.

**15.** Verfahren zur Herstellung einer Cytisin-enthaltenden Schicht zur Verwendung in einem transdermalen therapeutischen System gemäß einem der Ansprüche 1 bis 13, umfassend die folgenden Schritte:

1) Kombinieren mindestens der Komponenten

1. Cytisin,
2. ein Polymer, ausgewählt aus der Gruppe bestehend aus Silikonbasierten Polymeren und Polyisobutylenen, und
3. einen Permeationsverstärker umfassend eine organische Verbindung mit einem linearen Rückgrat mit 6 bis 14 Kohlenstoffatomen und 1 Hydroxygruppe,

um eine Beschichtungszusammensetzung zu erhalten;
2) Auftragen der Beschichtungszusammensetzung auf die Rückschicht oder eine Trennschicht oder eine beliebige Zwischenschicht; und
3) Trocknen der aufgetragenen Beschichtungszusammensetzung, um die Cytisin-enthaltende Schicht zu bilden.

**Revendications**

1. Système thérapeutique transdermique pour l'administration transdermique de cytisine comprenant une structure de couche auto-adhésive contenant une quantité thérapeutiquement efficace de cytisine, ladite structure de couche auto-adhésive comprenant :

   A) une couche de support ;
   B) une couche contenant de la cytisine comprenant

      1. cytisine,
      2. un polymère choisi dans le groupe constitué par les polymères à base de silicone et des polyisobutylènes, et
      3. un promoteur de pénétration comprenant un composé organique ayant un squelette linéaire comprenant 6 à 14 atomes de carbone et muni de 1 groupe hydroxy.

2. Système thérapeutique transdermique selon la revendication 1, dans lequel le promoteur de perméation comprend au moins un ester ou un alcool ayant 11 ou 12 atomes de carbone et muni de 1 groupe hydroxy.

3. Système thérapeutique transdermique selon la revendication 1 ou 2,

   dans lequel le promoteur de perméation est un mélange constitué de monocaprylate de propylène glycol et de dicaprylate de propylène glycol ou est du dodécan-1-ol, de préférence un mélange constitué de monocaprylate de propylène glycol et de dicaprylate de propylène glycol, dans lequel le rapport du monocaprylate de propylène glycol au dicaprylate de propylène glycol est d'au moins 75:25, de préférence d'au moins 85:15, et le plus préférablement d'au moins 90:10, et/ou
   dans lequel le promoteur de perméation a un poids moléculaire d'au moins 150 g/mol et pas plus de 210 g/mol, de préférence d'au moins 175 g/mol et d'au plus 205 g/mol, et le plus préférablement d'au moins 185 g/mol et d'au plus 202,5 g/mol.

4. Système thérapeutique transdermique selon l'une quelconque des revendications 1 à 3, dans lequel la quantité du promoteur de perméation varie de 1 à 20 %, de préférence de 2 à 15 %, plus préférablement de 4 à 12 %, et le plus préférablement de 5 à 11 % sur la base du poids total de la couche contenant de la cytisine.

5. Système thérapeutique transdermique selon l'une quelconque des revendications 1 à 4,

   dans lequel la couche contenant de la cytisine comprend en outre un polymère supplémentaire choisi parmi des polymères qui ne comprennent pas de groupes hydroxyle, de groupes acides carboxylique, de groupes acides carboxylique neutralisés et de mélanges de ceux-ci,
   dans lequel de préférence le polymère supplémentaire est choisi parmi les polymères d'acrylate basiques ou les polyvinylpyrrolidones,
   dans lequel plus préférablement le polymère supplémentaire est un acrylate à fonction amine ou une polyvinylpyrrolidone.

6. Système thérapeutique transdermique selon l'une quelconque des revendications 1 à 5, dans lequel la couche contenant de la cytisine comprend en outre un polymère supplémentaire en une quantité de 0,1 à 30 %, de préférence 1 à 25 %, plus préférablement 2 à 22 %, et le plus préférablement de 2 à 12 % ou de 18 à 22 % sur la base du poids total de la couche contenant de la cytisine.

7. Système thérapeutique transdermique selon l'une quelconque des revendications 1 à 6,

   dans lequel la couche contenant de la cytisine comprend de la cytisine en une quantité de 2 à 25%, de préférence de 2,5 à 18 %, plus préférablement de 4 à 12 %, et le plus préférablement d'environ 5 % ou d'environ 10 % sur la base du poids total de la couche contenant de la cytisine, et/ou
   dans lequel la cytisine dans la couche contenant de la cytisine est incluse sous la forme de la base libre ou sous la forme d'un sel pharmaceutiquement acceptable de celle-ci, dans lequel de préférence au moins 90 % en moles, de préférence au moins 95 % en moles, plus préférablement au moins 98 % en moles et le plus préférablement au moins 99 % en moles de la cytisine dans la couche contenant de la cytisine est présente sous la forme de la base

libre.

**8.** Système thérapeutique transdermique selon l'une quelconque des revendications 1 à 7,

dans lequel le système thérapeutique transdermique contient au moins 0,25 mg/$cm^2$, de préférence au moins 0,45 mg/$cm^2$, plus préférablement au moins 0,70 mg/$cm^2$ et le plus préférablement au moins 0,95 mg/$cm^2$ de cytisine, et/ou
dans lequel le système thérapeutique transdermique contient moins de 2,0 mg/$cm^2$, moins de 1,5 mg/$cm^2$, moins de 1,2 mg/$cm^2$ ou moins de 1,0 mg/cm2 de cytisine, et/ou
dans lequel le système thérapeutique transdermique comprend de la cytisine en une quantité de 1 à 100 mg, de préférence de 10 à 70 mg, plus préférablement de 15 à 50 mg.

**9.** Système thérapeutique transdermique selon l'une quelconque des revendications 1 à 8,

dans lequel le polymère est au moins un polysiloxane compatible avec les amines, et peut de préférence être obtenu par polycondensation d'un polydiméthylsiloxane bloqué en bout de silanol avec une résine de silicate suivie d'une triméthylsilylation au moins partielle de la fonctionnalité silanol résiduelle, ou
dans lequel le polymère est un mélange de polyisobutylène de faible poids moléculaire et de polyisobutylène de poids moléculaire élevé avec un rapport de polyisobutylène de faible poids moléculaire à polyisobutylène de poids moléculaire élevé dans une plage de 100:1 à 1:100, de préférence de 95:5 à 40:60, plus préférablement de 90:10 à 80:20.

**10.** Système thérapeutique transdermique selon l'une quelconque des revendications 1 à 9,
dans lequel la quantité du polymère varie de 55 à 97 %, de préférence de 60 à 75 % ou de 68 à 82 % sur la base du poids total de la couche contenant de la cytisine.

**11.** Système thérapeutique transdermique selon l'une quelconque des revendications 1 à 10,
dans lequel la couche contenant de la cytisine comprend en outre au moins un additif ou excipient choisi parmi les solubilisants, les charges, les agents gélifiants, les substances pour les soins de la peau, les régulateurs de pH, les conservateurs, les agents d'adhésivité, les adoucissants et les stabilisants, en particulier parmi les agents gélifiants, les agents d'adhésivité, les adoucissants et les stabilisants.

**12.** Système thérapeutique transdermique selon l'une quelconque des revendications 1 à 11,

dans lequel la masse surfacique de la couche contenant de la cytisine varie de 70 à 200 g/$m^2$, de préférence de 80 à 160 g/$m^2$, et le plus préférablement de 85 à 130 g/$m^2$, et/ou
dans lequel la zone de libération varie de 20 à 70 $cm^2$, de préférence de 30 à 50 $cm^2$.

**13.** Système thérapeutique transdermique selon l'une quelconque des revendications 1 à 12,

dans lequel le système thérapeutique transdermique fournit une quantité cumulée absorbée de cytisine, telle que mesurée dans une cellule de diffusion de Franz à peau humaine dermatomée, d'au moins 150 $\mu$g/$cm^2$, d'au moins 210 $\mu$g/$cm^2$, d'au moins 250 $\mu$g/$cm^2$, d'au moins 270 $\mu$g/$cm^2$, d'au moins 280 $\mu$g/$cm^2$ ou d'au moins 285 $\mu$g/$cm^2$ après une période de 24 heures, et/ou
dans lequel le système thérapeutique transdermique fournit une quantité cumulée absorbée de cytisine, telle que mesurée dans une cellule de diffusion de Franz à peau humaine dermatomée, de 100 $\mu$g/$cm^2$ à 600 $\mu$g/$cm^2$, de préférence de 200 $\mu$g/$cm^2$ à 550 $\mu$g/$cm^2$, plus préférablement de 300 $\mu$g/$cm^2$ à 500 $\mu$g/$cm^2$ après une période de 24 heures, et/ou
dans lequel le système thérapeutique transdermique fournit par administration transdermique une vitesse de libération moyenne de 1 à 20 mg/jour, de préférence de 2 à 12 mg/jour, plus préférablement de 3 à 10 mg/jour sur au moins 24 heures d'administration.

**14.** Système thérapeutique transdermique selon l'une quelconque des revendications 1 à 13 pour utilisation dans une méthode de traitement d'un patient humain, de préférence dans une méthode de traitement de la dépendance à la nicotine, plus préférablement dans un procédé de réduction des fringales de nicotine, et le plus préférablement un procédé de sevrage tabagique,
dans lequel de préférence le système thérapeutique transdermique est appliqué sur la peau du patient pendant un intervalle de dosage de 20 à 30 heures, de préférence d'environ 24 heures.

**15.** Un procédé de fabrication d'une couche contenant de la cytisine destinée à être utilisée dans un système thérapeutique transdermique selon l'une quelconque des revendications 1 à 13, comprenant les étapes consistant à :

1) combiner au moins les composants

1. cytisine,
2. un polymère choisi dans le groupe constitué par les polymères à base de silicone et les polyisobutylènes, et
3. un promoteur de perméation choisi parmi un composé organique ayant un squelette linéaire comprenant 6 à 14 atomes de carbone et muni de 1 groupe

pour obtenir une composition de revêtement ;
2) revêtir la composition de revêtement sur la couche de support ou une doublure de libération ou toute doublure intermédiaire; et
3) sécher la composition de revêtement enduite pour former la couche contenant de la cytisine.

**Fig. 1a**

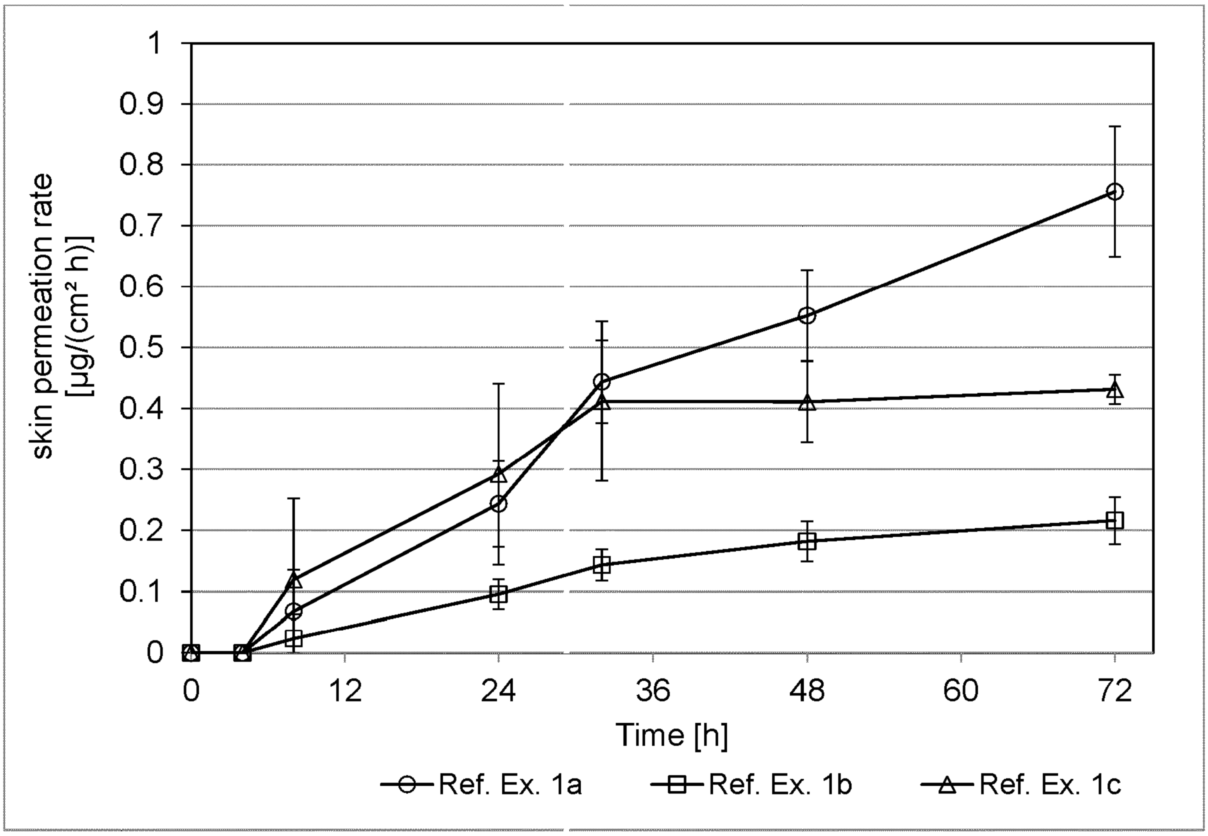
skin permeation rate [µg/(cm² h)]
1
0.9
0.8
0.7
0.6
0.5
0.4
0.3
0.2
0.1
0
0
12
24
36
48
60
72
Time [h]
Ref. Ex. 1a
Ref. Ex. 1b
Ref. Ex. 1c

**Fig. 1b**

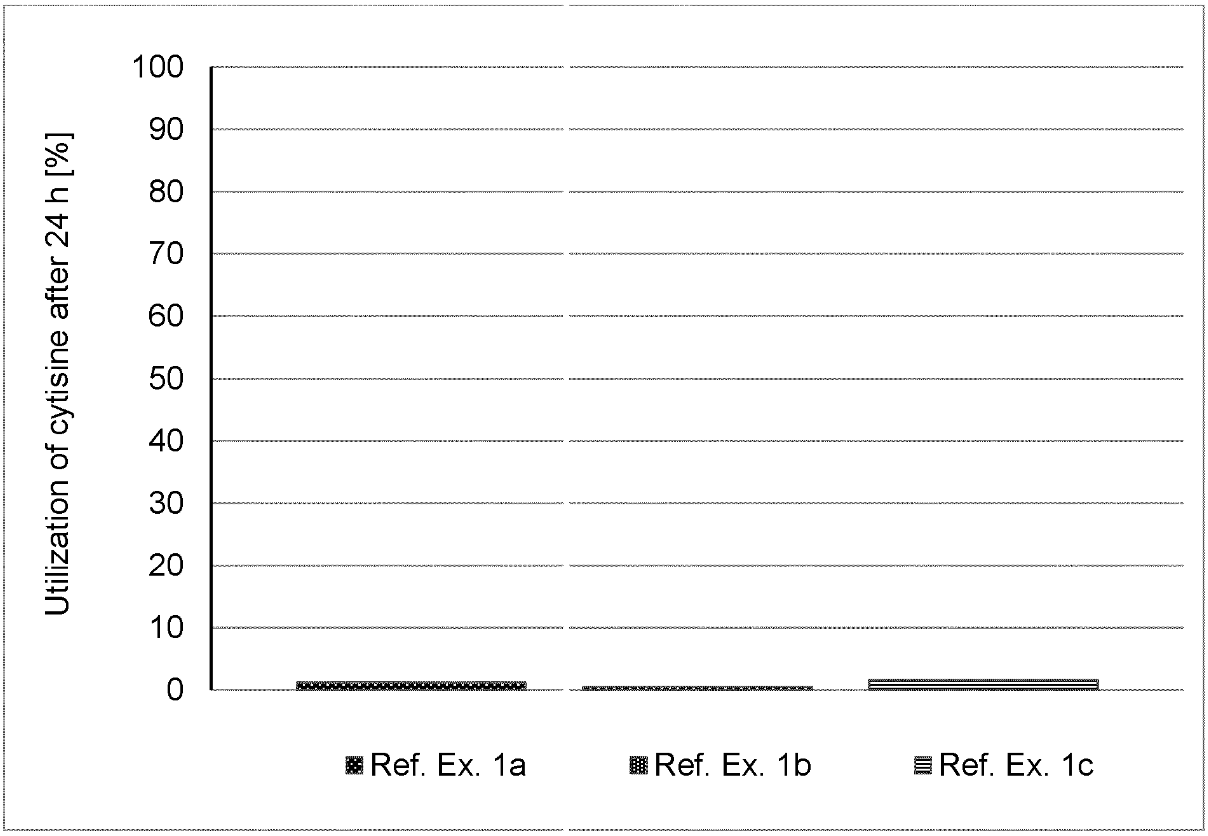
Utilization of cytisine after 24 h [%]
100
90
80
70
60
50
40
30
20
10
0
Ref. Ex. 1a
Ref. Ex. 1b
Ref. Ex. 1c

**Fig. 2a**

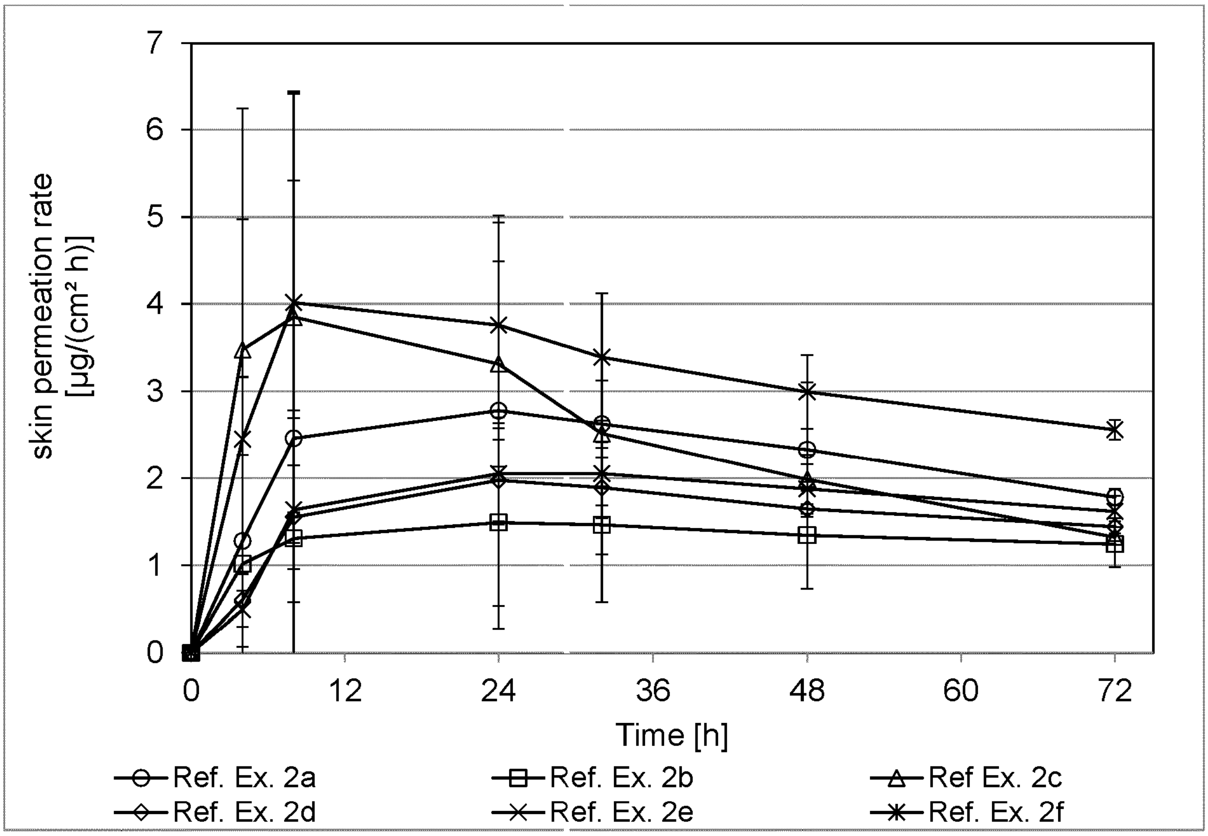
skin permeation rate [µg/(cm² h)]
Time [h]
0
1
2
3
4
5
6
7
0
12
24
36
48
60
72
Ref. Ex. 2a
Ref. Ex. 2b
Ref Ex. 2c
Ref. Ex. 2d
Ref. Ex. 2e
Ref. Ex. 2f

**Fig. 2b**

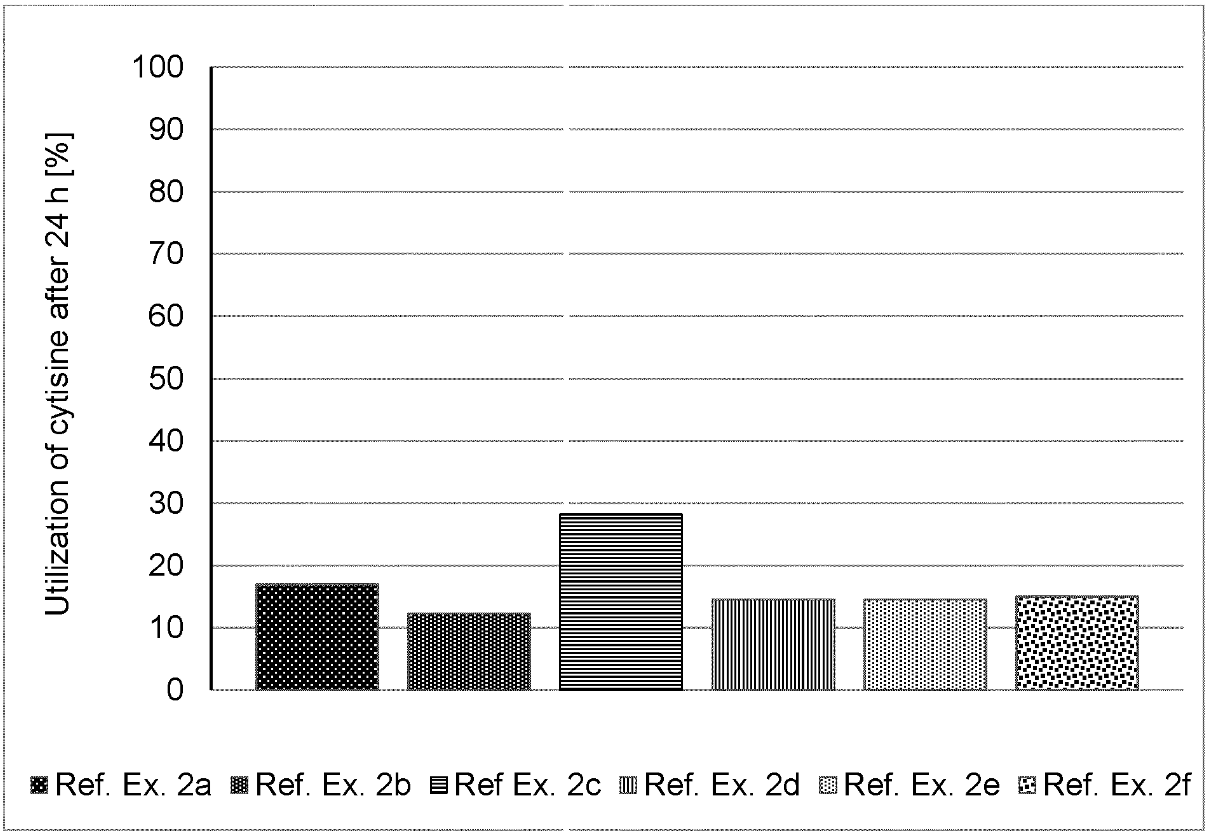
Utilization of cytisine after 24 h [%]
0
10
20
30
40
50
60
70
80
90
100
Ref. Ex. 2a
Ref. Ex. 2b
Ref Ex. 2c
Ref. Ex. 2d
Ref. Ex. 2e
Ref. Ex. 2f

Fig. 3a

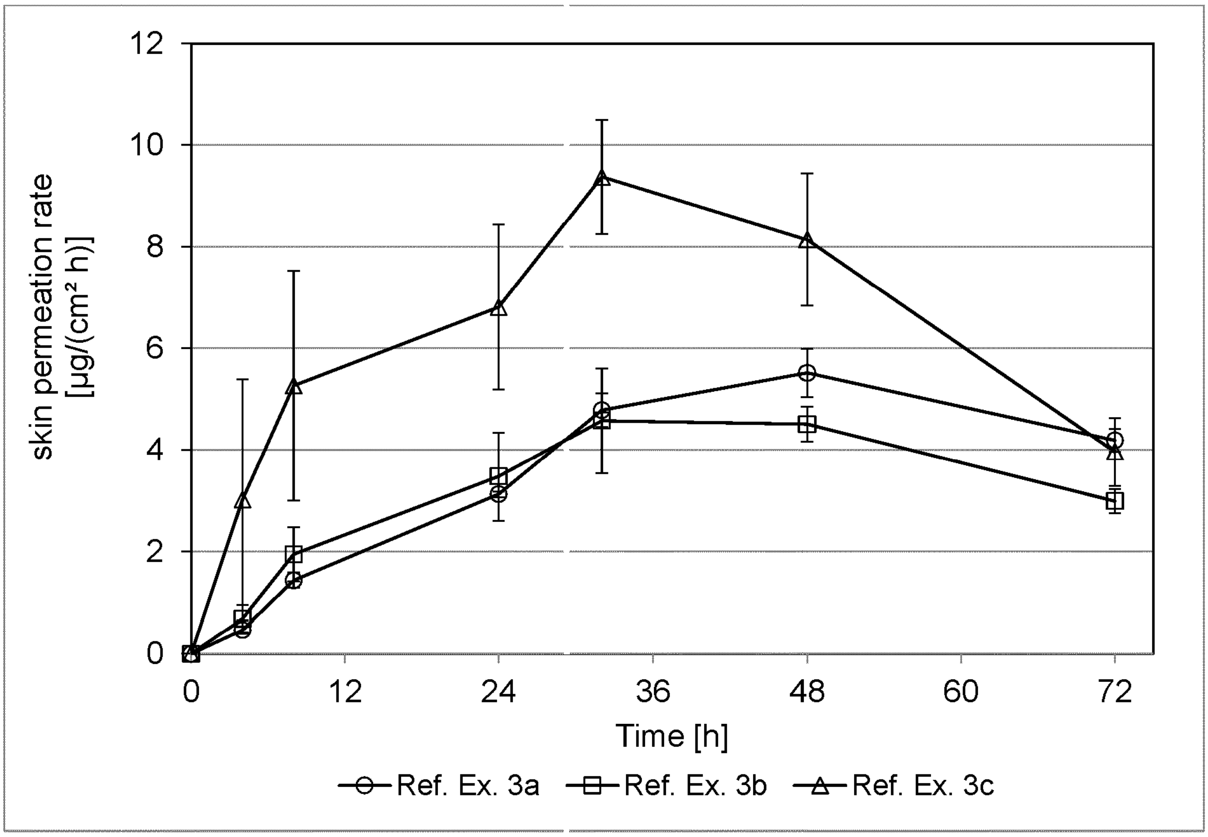
skin permeation rate
[µg/(cm² h)]
12
10
8
6
4
2
0
0
12
24
36
48
60
72
Time [h]
Ref. Ex. 3a
Ref. Ex. 3b
Ref. Ex. 3c

Fig. 3b

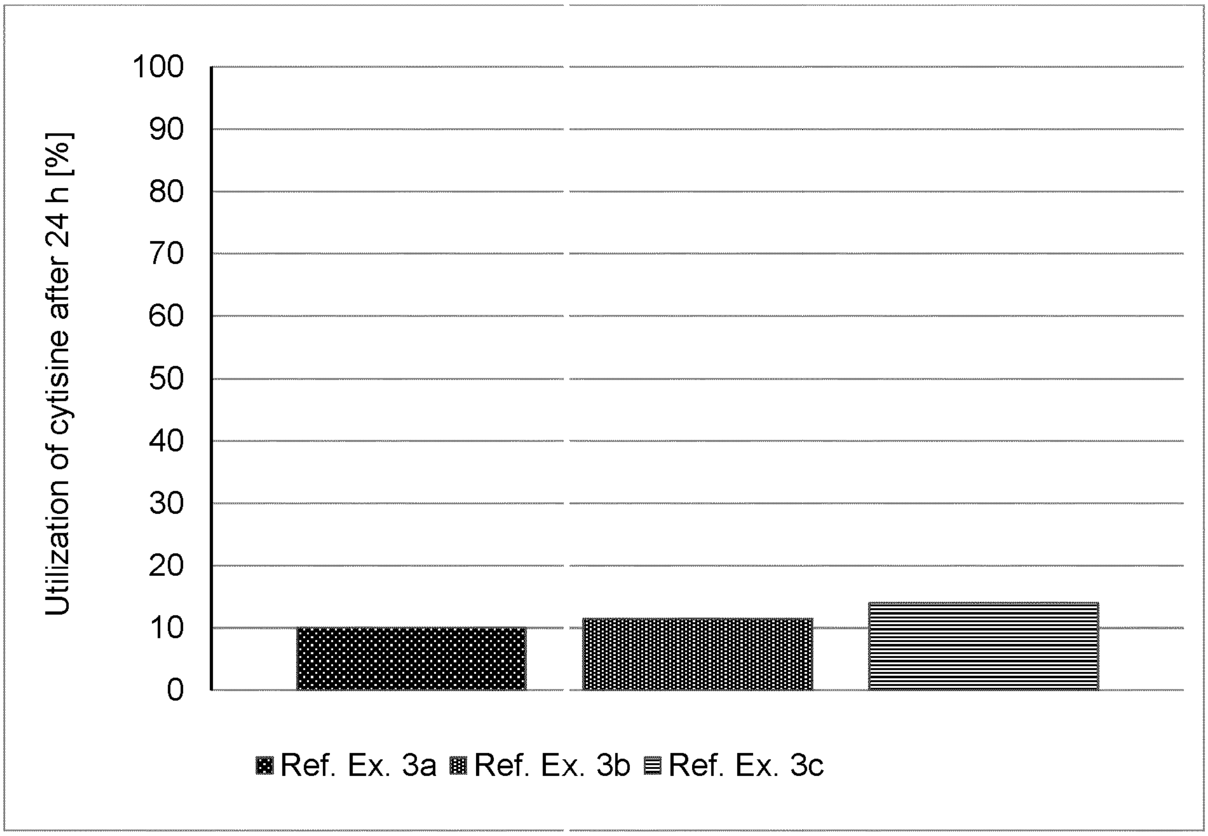
Utilization of cytisine after 24 h [%]
100
90
80
70
60
50
40
30
20
10
0
Ref. Ex. 3a
Ref. Ex. 3b
Ref. Ex. 3c

**Fig. 4a**

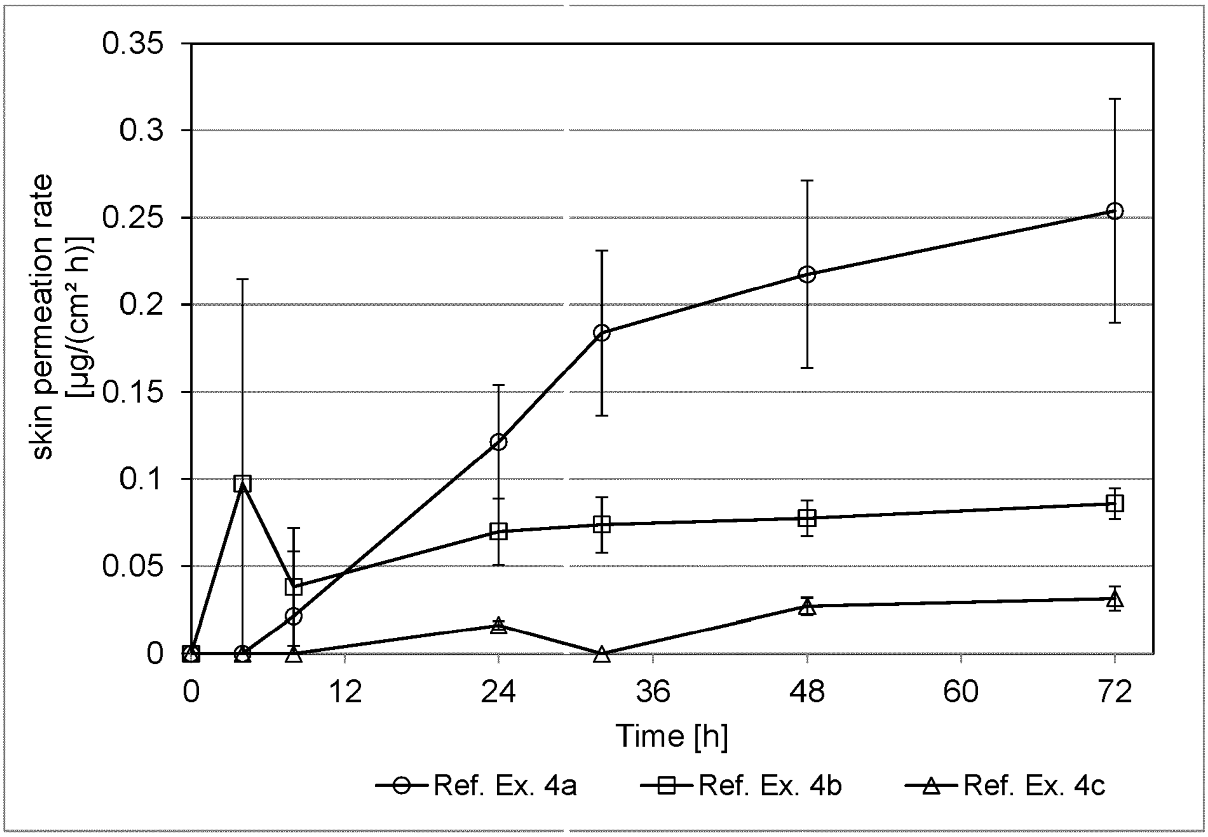
0.35
0.3
0.25
0.2
0.15
0.1
0.05
0
skin permeation rate
[µg/(cm² h)]
0
12
24
36
48
60
72
Time [h]
Ref. Ex. 4a
Ref. Ex. 4b
Ref. Ex. 4c

**Fig. 4b**

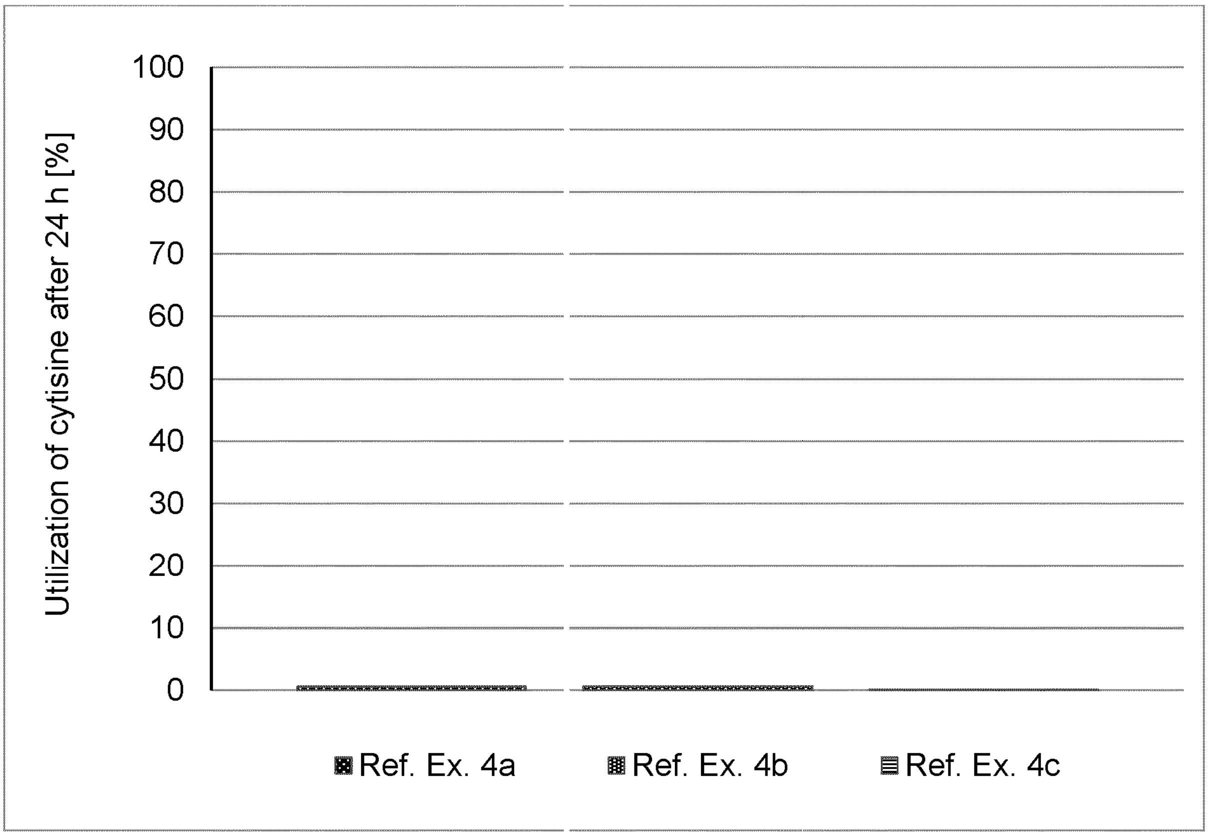
100
90
80
70
60
50
40
30
20
10
0
Utilization of cytisine after 24 h [%]
Ref. Ex. 4a
Ref. Ex. 4b
Ref. Ex. 4c

**Fig. 5a**

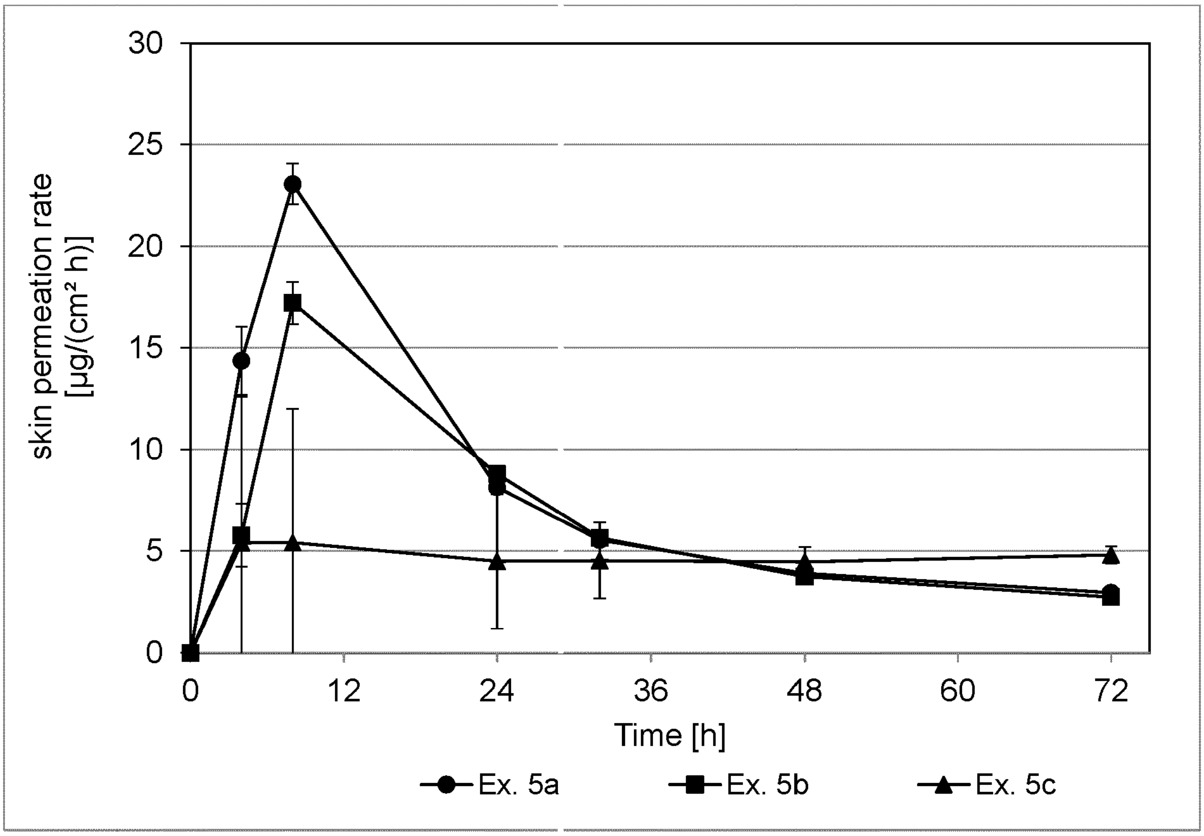
30
25
20
15
10
5
0
skin permeation rate
[µg/(cm² h)]
0
12
24
36
48
60
72
Time [h]
Ex. 5a
Ex. 5b
Ex. 5c

**Fig. 5b**

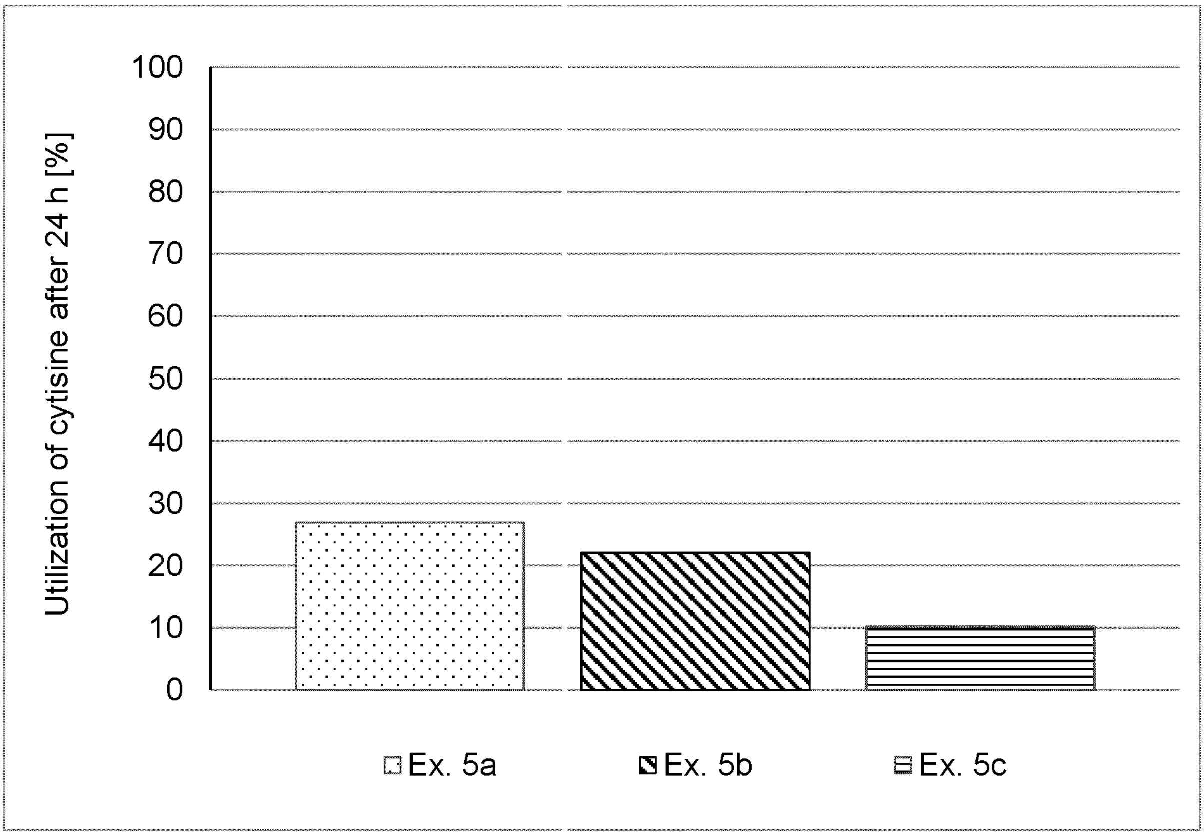
100
90
80
70
60
50
40
30
20
10
0
Utilization of cytisine after 24 h [%]
Ex. 5a
Ex. 5b
Ex. 5c

Fig. 6a

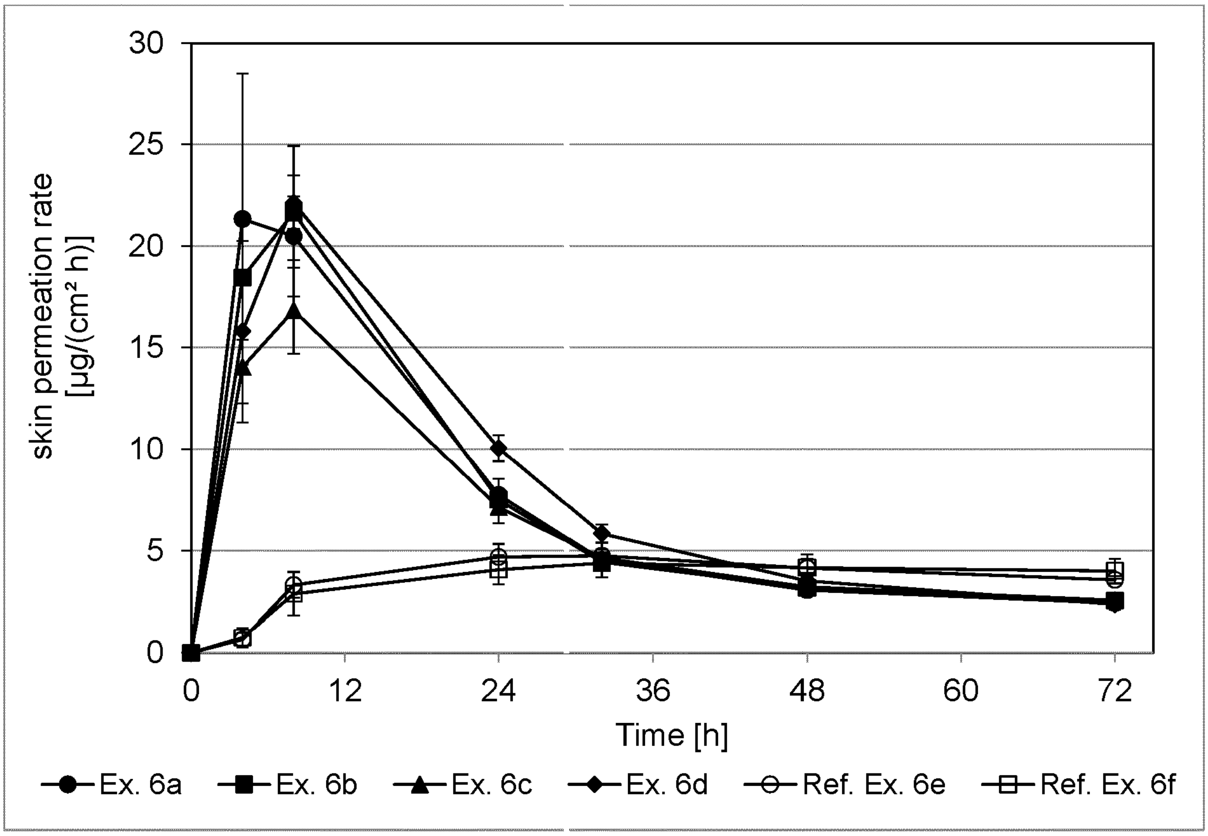
30
25
20
15
10
5
0
skin permeation rate
[µg/(cm² h)]
0
12
24
36
48
60
72
Time [h]
Ex. 6a
Ex. 6b
Ex. 6c
Ex. 6d
Ref. Ex. 6e
Ref. Ex. 6f

Fig. 6b

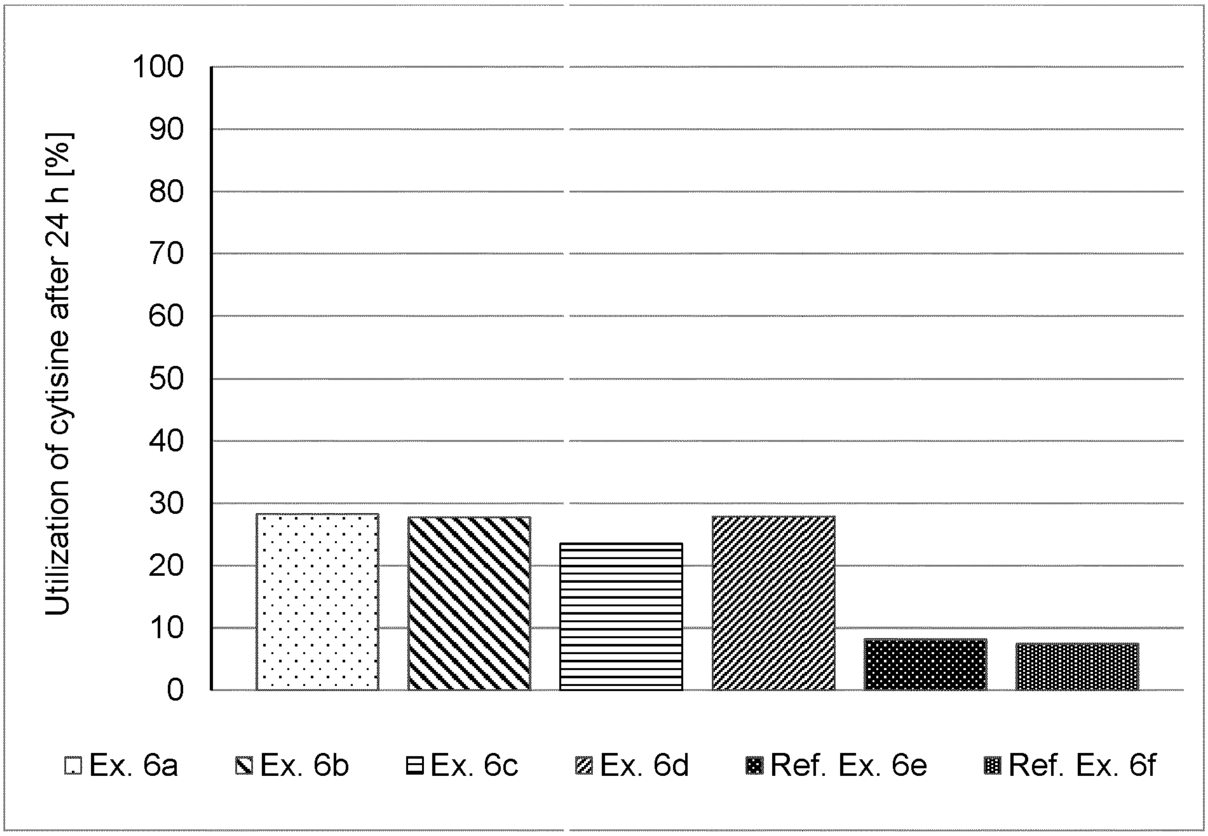
100
90
80
70
60
50
40
30
20
10
0
Utilization of cytisine after 24 h [%]
Ex. 6a
Ex. 6b
Ex. 6c
Ex. 6d
Ref. Ex. 6e
Ref. Ex. 6f

**Fig. 7a**

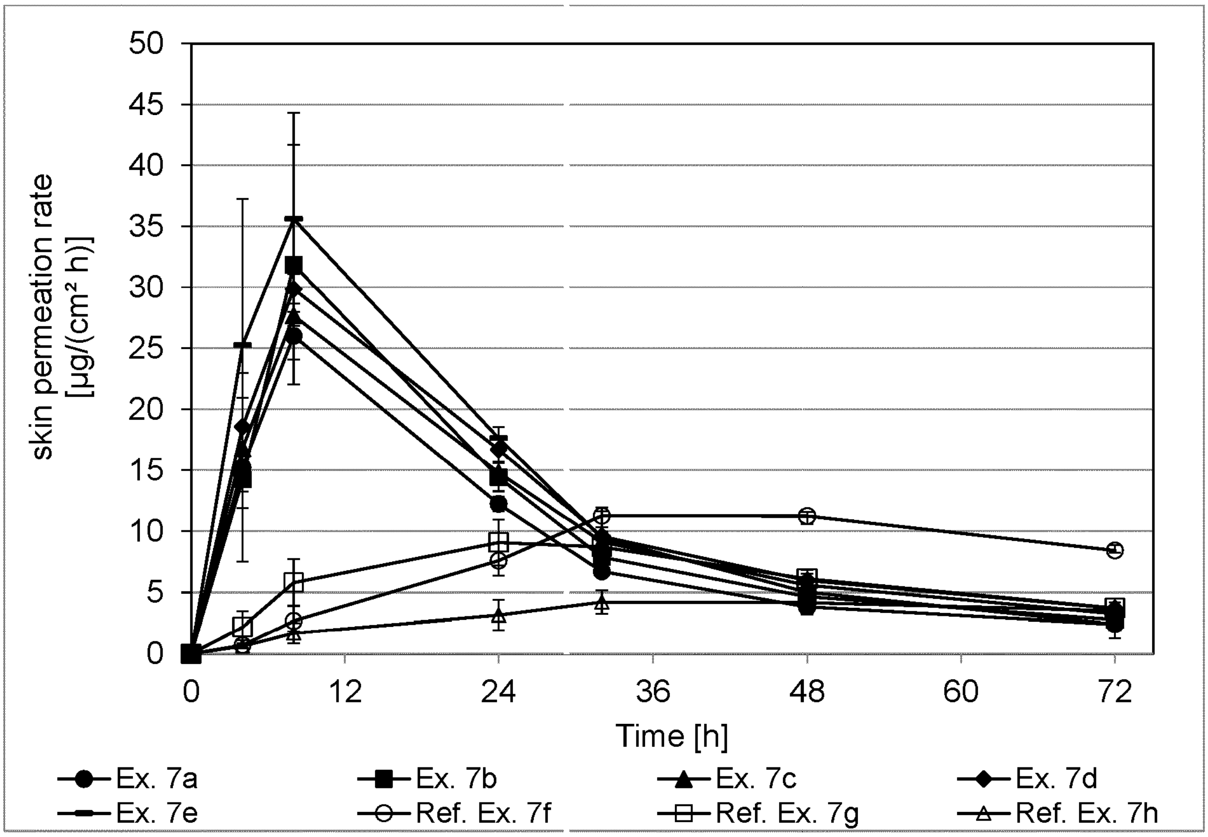
skin permeation rate
[µg/(cm² h)]
50
45
40
35
30
25
20
15
10
5
0
0
12
24
36
48
60
72
Time [h]
Ex. 7a
Ex. 7b
Ex. 7c
Ex. 7d
Ex. 7e
Ref. Ex. 7f
Ref. Ex. 7g
Ref. Ex. 7h

**Fig. 7b**

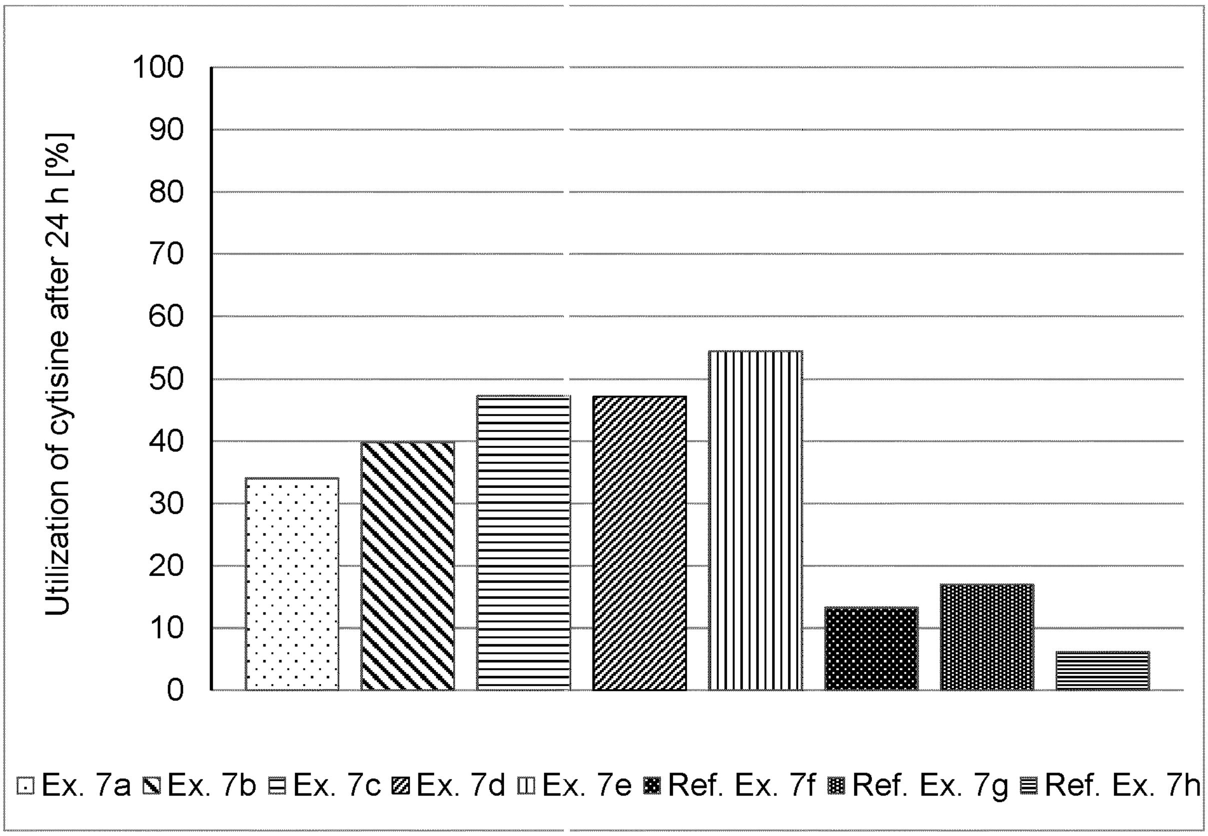
Utilization of cytisine after 24 h [%]
100
90
80
70
60
50
40
30
20
10
0
Ex. 7a
Ex. 7b
Ex. 7c
Ex. 7d
Ex. 7e
Ref. Ex. 7f
Ref. Ex. 7g
Ref. Ex. 7h

**Fig. 8a**

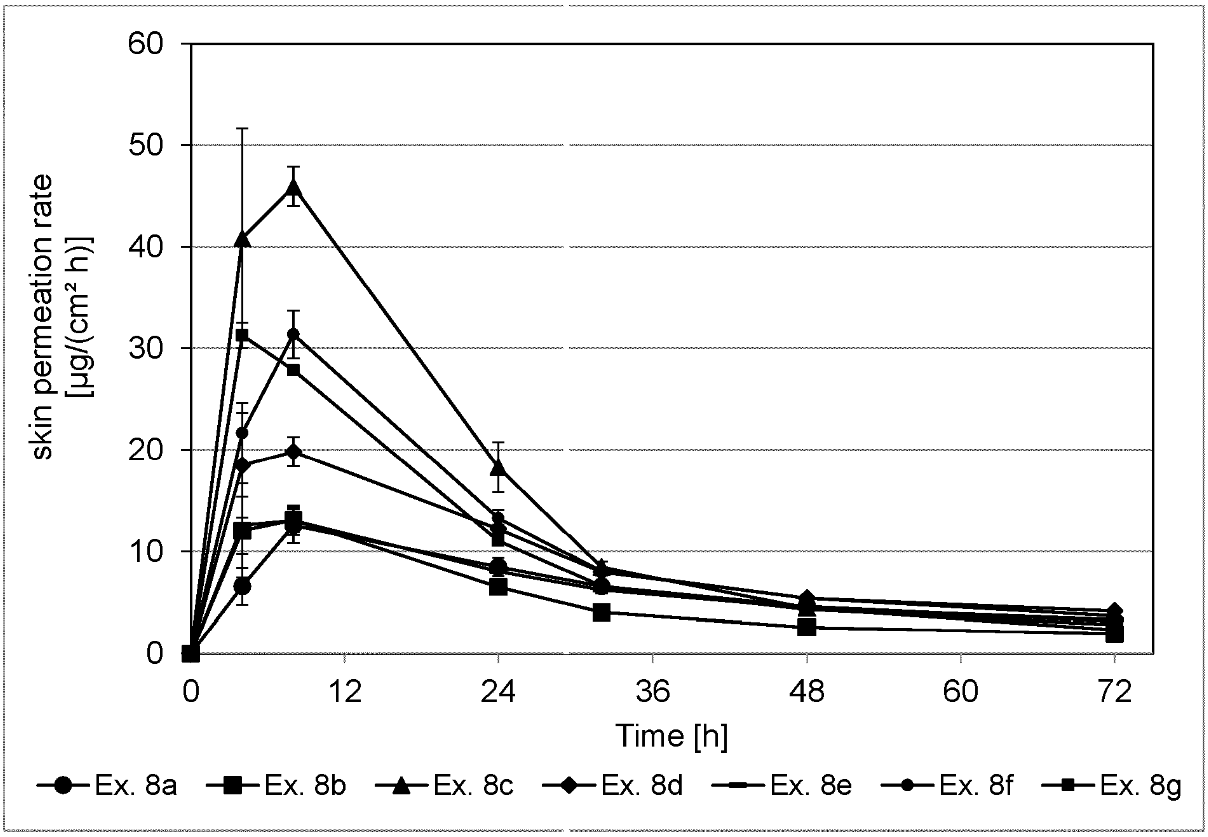
skin permeation rate [µg/(cm² h)]
0
10
20
30
40
50
60
0
12
24
36
48
60
72
Time [h]
Ex. 8a
Ex. 8b
Ex. 8c
Ex. 8d
Ex. 8e
Ex. 8f
Ex. 8g

**Fig. 8b**

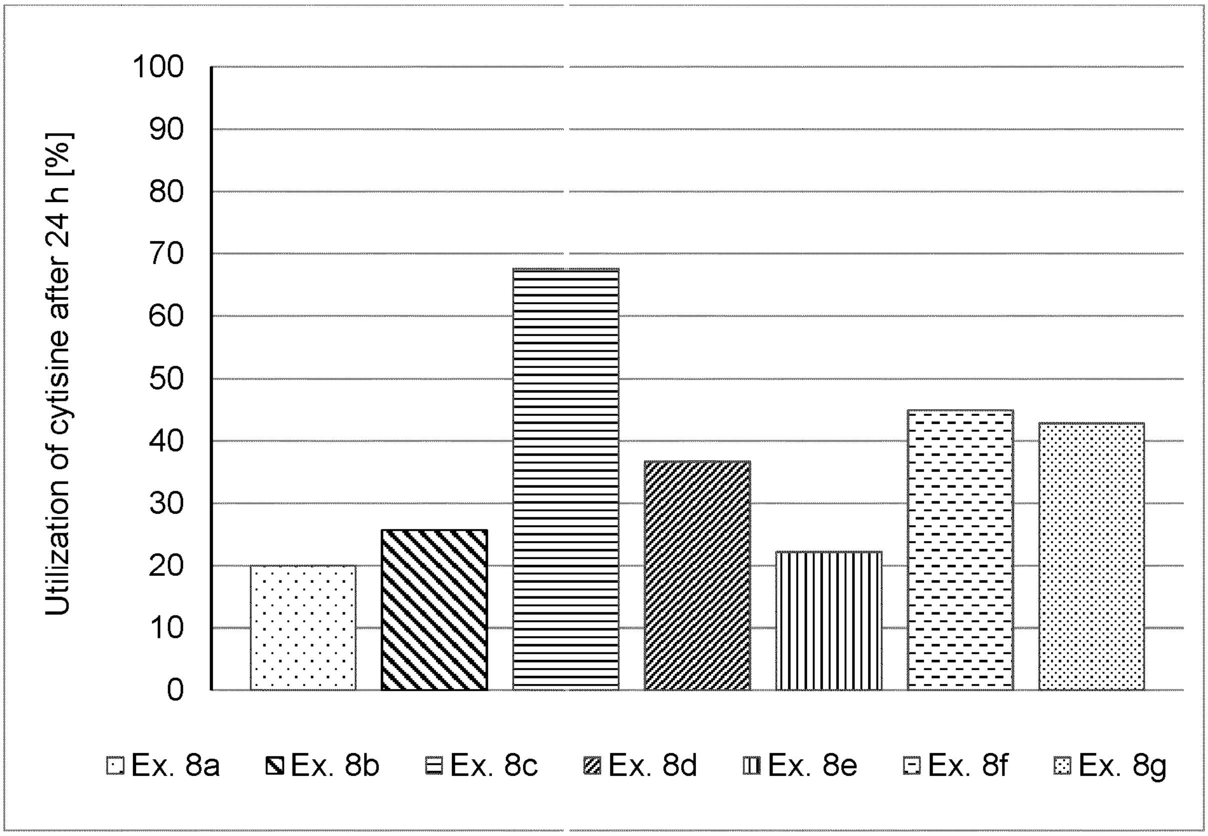
Utilization of cytisine after 24 h [%]
0
10
20
30
40
50
60
70
80
90
100
Ex. 8a
Ex. 8b
Ex. 8c
Ex. 8d
Ex. 8e
Ex. 8f
Ex. 8g

**Fig. 9a**

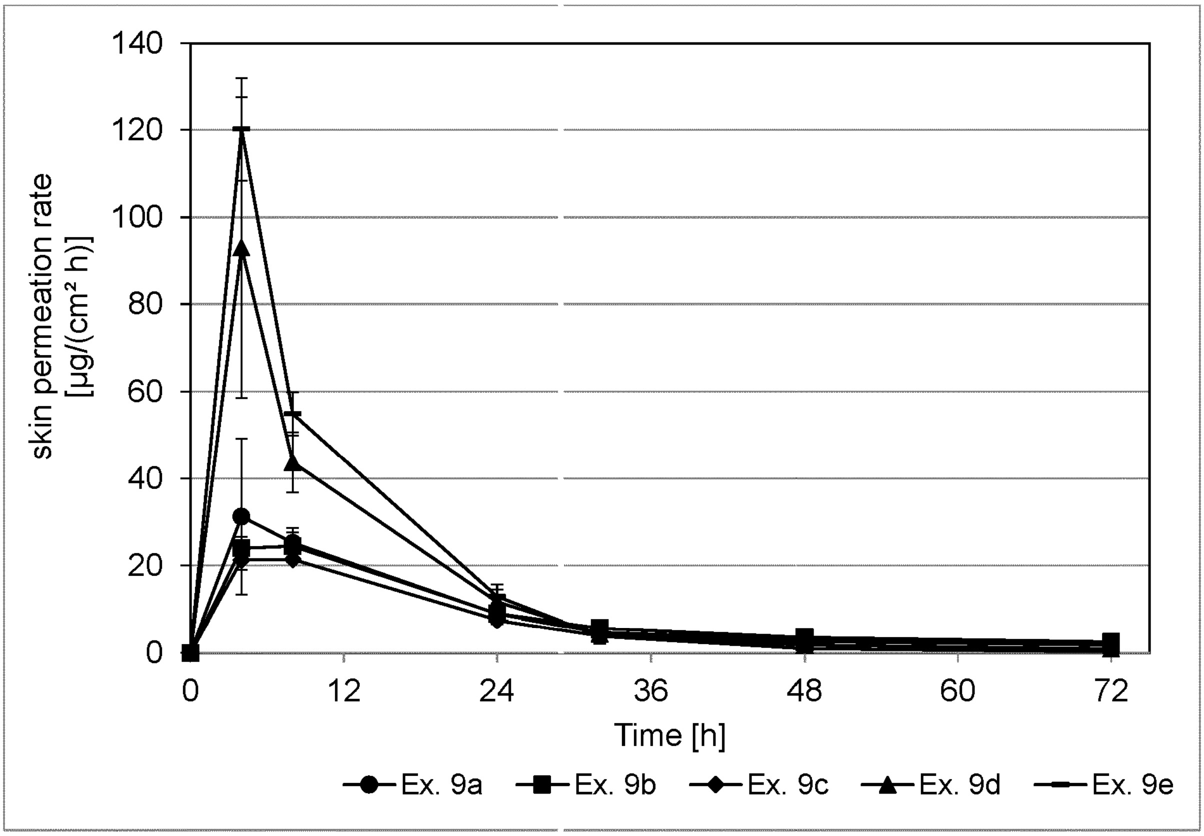
skin permeation rate [µg/(cm² h)]
140
120
100
80
60
40
20
0
0
12
24
36
48
60
72
Time [h]
Ex. 9a
Ex. 9b
Ex. 9c
Ex. 9d
Ex. 9e

**Fig. 9b**

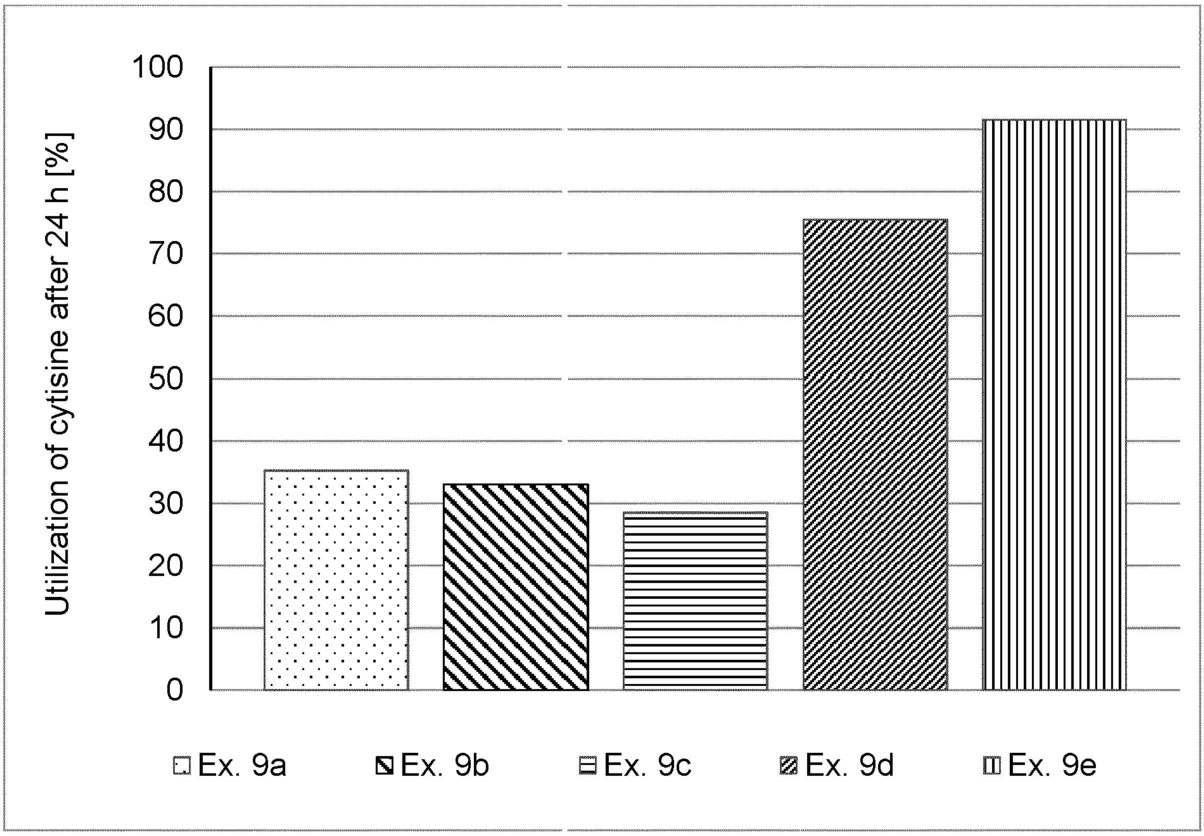
Utilization of cytisine after 24 h [%]
100
90
80
70
60
50
40
30
20
10
0
Ex. 9a
Ex. 9b
Ex. 9c
Ex. 9d
Ex. 9e

**Fig. 10a**

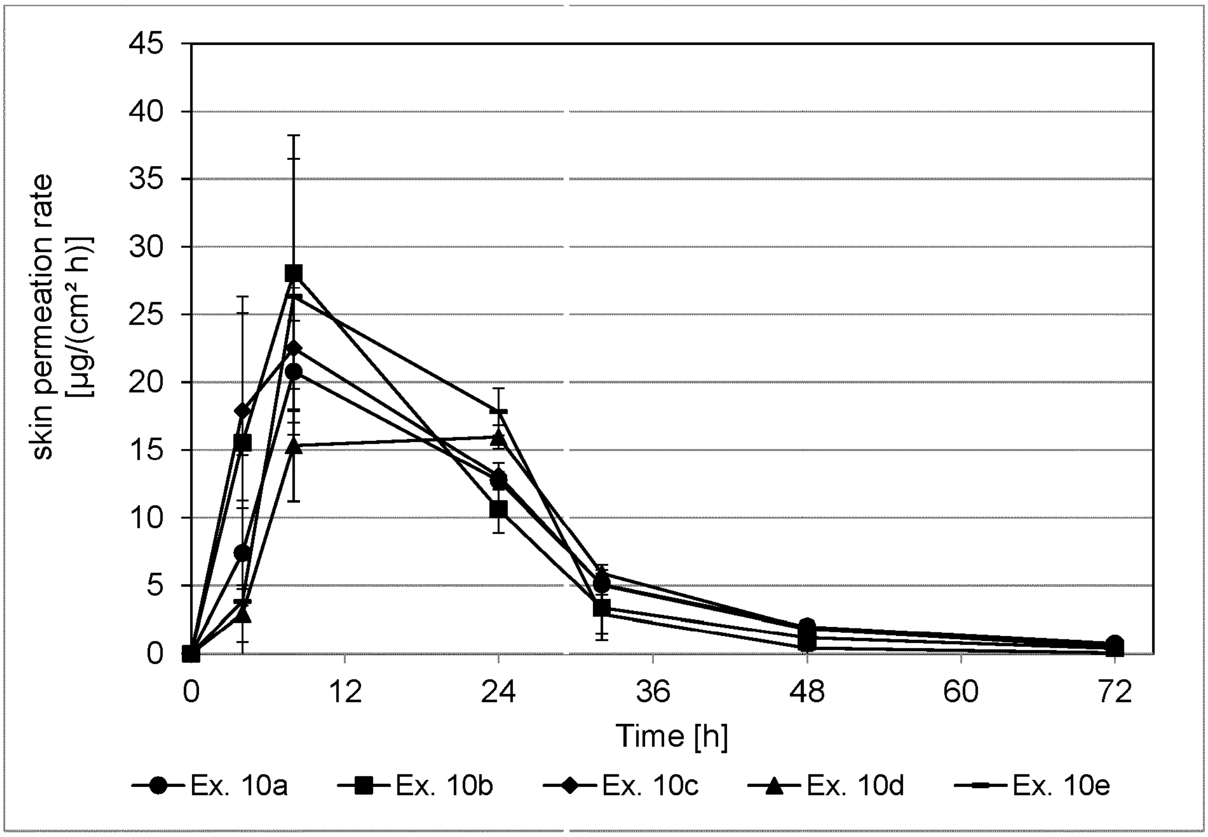


**Fig. 10b**

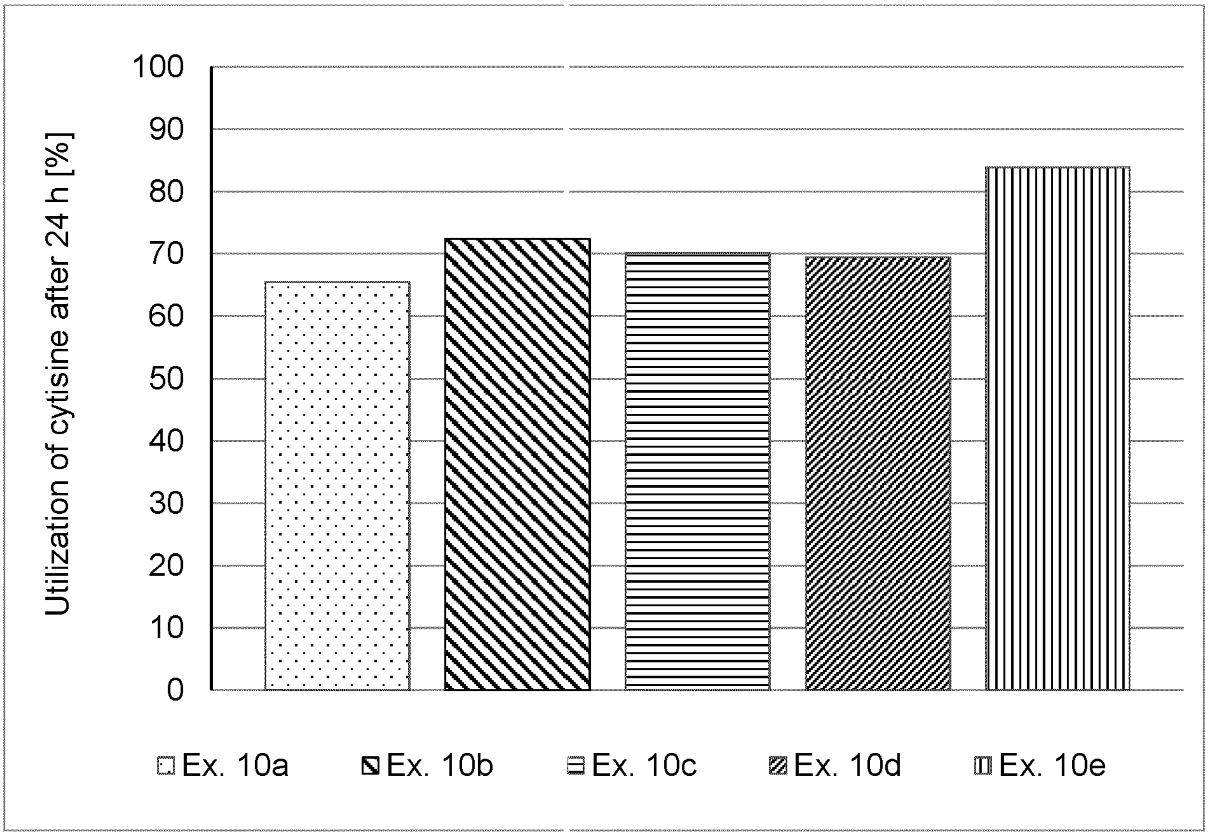

Fig. 11a

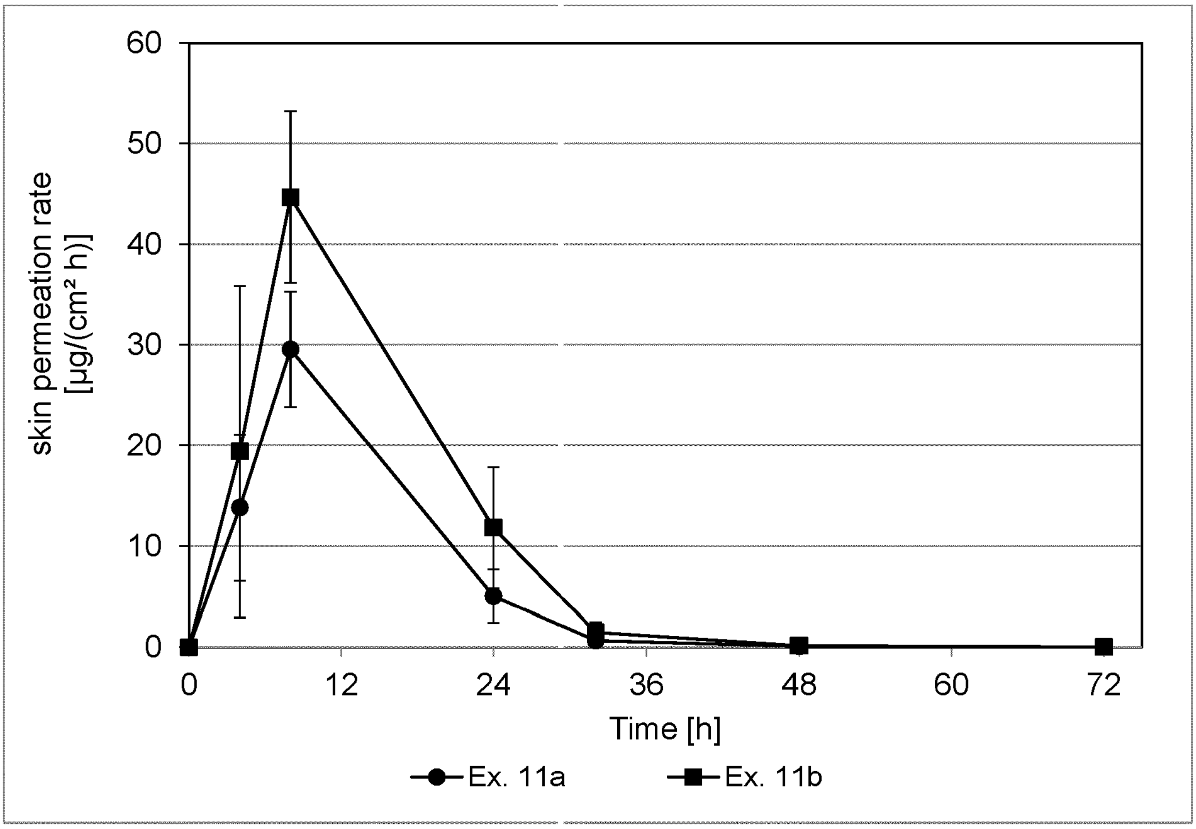


Fig. 11b